(19) Europäisches Patentamt
European Patent Office
Office européen des brevets

(11) Veröffentlichungsnummer: **0 027 214**
**B1**

(12) **EUROPÄISCHE PATENTSCHRIFT**

(45) Veröffentlichungstag der Patentschrift: **16.05.84**

(21) Anmeldenummer: **80105970.0**

(22) Anmeldetag: **02.10.80**

(51) Int. Cl.³: **C 07 D 487/04,** C 07 D 487/14, C 07 D 495/14, C 07 D 495/22, A 61 K 31/55, C 07 D 495/04, C 07 D 243/14 // C07C79/46, C07C101/54, C07D265/26, C07D333/38 ,(C07D487/04, 243/00, 235/00),(C07D487/14, 243/00, 235/00, 209/00),(C07D495/14, 333/00, 243/00, 235/00),(C07D495/22, 333/00, 243/00, 235/00, 209/00)

(54) **Imidazodiazepin-Derivate, Verfahren und Zwischenprodukte zu ihrer Herstellung, diese enthaltende Arzneimittel und deren therapeutische Verwendung.**

(30) Priorität: **04.10.79 CH 8971/79**
**04.10.79 CH 8972/79**
**30.11.79 CH 10664/79**
**30.11.79 CH 10665/79**
**25.07.80 CH 5716/80**

(43) Veröffentlichungstag der Anmeldung: **22.04.81 Patentblatt 81/16**

(45) Bekanntmachung des Hinweises auf die Patenterteilung: **16.05.84 Patentblatt 84/20**

(84) Benannte Vertragsstaaten: **AT BE CH DE FR IT LI LU NL SE**

(56) Entgegenhaltungen:
**DE - A - 2 813 549**
**US - A - 3 933 794**
**US - A - 4 002 610**

**CHEMICAL ABSTRACTS, Band 82, Nr. 19, 12. Mai 1975, Zusammenfassung Nr. 125421n Columbus, Ohio, U.S.A.**

(73) Patentinhaber: **F. HOFFMANN-LA ROCHE & CO. Aktiengesellschaft, CH-4002 Basel (CH)**

(72) Erfinder: **Haefely, Willy, Dr., Unterer Rebbergweg 125, CH-4153 Reinach (CH)**
Erfinder: **Hunkeler, Walter, Dr., Im Stigler 32, CH-4465 Magden (CH)**
Erfinder: **Kyburz, Emilio, Dr., Unterer Rebbergweg 127, CH-4153 Reinach (CH)**
Erfinder: **Möhler, Hanns, Dr., Unterer Baselblick 31, D-7851 Inzlingen (DE)**
Erfinder: **Pieri, Lorenzo, Dr., Burgstrasse 58, CH-4125 Riehen (CH)**
Erfinder: **Polc, Petar, Dr., Holeeholzweg 59, CH-4102 Binningen (CH)**
Erfinder: **Gerecke, Max, Dr., Bruderholzstrasse 47, CH-4153 Reinach (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Imidazodiazepin-Derivate, Verfahren und Zwischenprodukte zu ihrer Herstellung, diese enthaltende Arzneimittel und deren therapeutische Verwendung

Die vorliegende Erfindung betrifft Imidazodiazepin-Derivate. Im speziellen betrifft sie Imidazo[1,5-a][1,4]-diazepin-Derivate der allgemeinen Formel

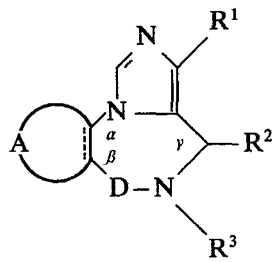

(I)

worin A zusammen mit den beiden mit $\alpha$ und $\beta$ bezeichneten Kohlenstoffatomen eine der Gruppen

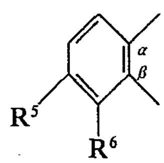 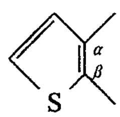 und 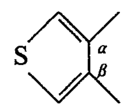

(a) (b) (c)

und die gestrichelte Linie die in Fällen (a) und (b) vorliegende Doppelbindung bedeuten, D $>C=O$ oder $>C=S$, $R^1$ Cyano, $(C_{2-7})$-Alkanoyl oder einen Rest der Formel $-COOR^4$, $R^4$ Methyl, Äthyl, Isopropyl oder 2-Hydroxyäthyl, $R^5$ Wasserstoff, Trifluormethyl oder Halogen und $R^6$ Wasserstoff, Trifluormethyl, Halogen oder $(C_{1-7})$-Alkyl bedeuten und entweder $R^2$ Wasserstoff und $R^3$ Wasserstoff oder $(C_{1-7})$-Alkyl bedeuten oder $R^2$ und $R^3$ zusammen Trimethylen oder Propenylen bedeuten und das mit $\gamma$ bezeichnete Kohlenstoffatom die S- oder R,S-Konfiguration aufweist,

und pharmazeutisch annehmbare Säureadditionssalze davon.

Diese Verbindungen sind neu und zeichnen sich durch wertvolle pharmakodynamische Eigenschaften aus.

Gegenstand der vorliegenden Erfindung sind Verbindungen der allgemeinen Formel I und pharmazeutisch annehmbare Säureadditionssalze davon als solche und als pharmazeutische Wirkstoffe, die Herstellung dieser Verbindungen und Zwischenprodukte für die Herstellung dieser Verbindungen, ferner Arzneimittel, enthaltend eine Verbindung der allgemeinen Formel I oder ein pharmazeutisch annehmbares Säureadditionssalz davon.

Der Ausdruck »$(C_1-C_7)$-Alkyl« bezeichnet gesättigte Kohlenwasserstoffreste, welche geradkettig oder verzweigt sein können, vorzugsweise mit höchstens 4 Kohlenstoffatomen, wie Methyl, Äthyl, n-Propyl, Isopropyl, n-Butyl, s-Butyl, t-Butyl und dergleichen. Der Ausdruck »Alkanoyl« umfaßt Reste wie Acetyl, Propionyl, Butanoyl, s-Butanoyl und dergleichen. Der Ausdruck »Halogen« bedeutet die vier Formen Fluor, Chlor, Brom und Jod.

$R^1$ bedeutet vorzugsweise Cyano oder einen Rest der Formel $-COOR^4$, wobei $R^4$ vorzugsweise Methyl, Äthyl oder Isopropyl bedeutet.

Wenn $R^2$ Wasserstoff bedeutet, so ist die bevorzugte Bedeutungsmöglichkeit von $R^3$ Methyl. Wenn $R^2$ und $R^3$ zusammen Trimethylen bedeutet, so weist das in Formel I mit $\gamma$ bezeichnete Kohlenstoffatom vorzugsweise die S-Konfiguration auf.

Das Symbol A bedeutet vorzugsweise die eingangs dargestellte Gruppe (a), wobei die gestrichelte Linie die in diesem Fall vorliegende Doppelbindung bedeutet; dabei bedeuten $R^5$ vorzugsweise Wasserstoff oder Fluor und $R^6$ vorzugsweise Wasserstoff, Fluor, Chlor oder Methyl, wobei vorzugsweise mindestens eines von $R^5$ und $R^6$ Wasserstoff bedeutet.

Eine ganz besonders bevorzugte Verbindung im Rahmen der vorliegenden Erfindung ist Äthyl-8-fluor-5,6-dihydro-5-methyl-6-oxo-4H-imidazo[1,5-a][1,4]benzodiazepin-3-carboxylat.

Im Rahmen der vorliegenden Erfindung besonders bevorzugte von der allgemeinen Formel I umfaßte Verbindungen sind:

Äthyl-7-fluor-5,6-dihydro-5-methyl-6-oxo-4H-imidazo[1,5-a][1,4]benzodiazepin-3-carboxylat,
Äthyl-7-chlor-5,6-dihydro-5-methyl-6-oxo-4H-imidazo[1,5-a][1,4]benzodiazepin-3-carboxylat,
Äthyl-(S)-8-chlor-11,12,13,13a-tetrahydro-9-oxo-9H-imidazo[1,5-a]pyrrolo[2,1-c]-[1,4]benzodiazepin-1-carboxylat,

Äthyl-(R,S)-11,13a-dihydro-9-oxo-9H-imidazo[1,5-a]-pyrrolo[2,1-c][1,4]benzodiazepin-1-carboxylat und
Äthyl-(R,S)-8-fluor-11,13a-dihydro-9-oxo-9H-imidazo[1,5-a]pyrrolo[2,1-c][1,4]benzodiazepin-1-carboxylat.

Weitere bevorzugte Verbindungen der allgemeinen Formel I sind:

Äthyl-5,6-dihydro-5-methyl-6-oxo-4H-imidazo[1,5-a][1,4]benzodiazepin-3-carboxylat,
Methyl-5,6-dihydro-5-methyl-6-oxo-4H-imidazo[1,5-a][1,4]benzodiazepin-3-carboxylat,
Isopropyl-5,6-dihydro-5-methyl-6-oxo-4H-imidazo[1,5-a][1,4]benzodiazepin-3-carboxylat,
Methyl-8-fluor-5,6-dihydro-5-methyl-6-oxo-4H-imidazo[1,5-a][1,4]benzodiazepin-3-carboxylat,
Isopropyl-8-fluor-5,6-dihydro-5-methyl-6-oxo-4H-imidazo[1,5-a][1,4]benzodiazepin-3-carboxylat,
Äthyl-5,6-dihydro-5,7-dimethyl-6-oxo-4H-imidazo[1,5-a][1,4]benzodiazepin-3-carboxylat,
Äthyl-(S)-11,12,13,13a-tetrahydro-9-oxo-9H-imidazo[1,5-a]pyrrolo[2,1-c][1,4]benzodiazepin-1-carboxylat,
Methyl-(S)-11,12,13,13a-tetrahydro-9-oxo-9H-imidazo[1,5-a]pyrrolo[2,1-c][1,4]benzodiazepin-1-carboxylat,
Isopropyl-(S)-11,12,13,13a-tetrahydro-9-oxo-9H-imidazo[1,5-a]pyrrolo[2,1-c][1,4]benzodiazepin-1-carboxylat,
Äthyl-(S)-7-fluor-11,12,13,13a-tetrahydro-9-oxo-9H-imidazo[1,5-a]pyrrolo[2,1-c]-[1,4]benzodiazepin-1-carboxylat,
Äthyl-(S)-11,12,13,13a-tetrahydro-8-methyl-9-oxo-9H-imidazo[1,5-a]pyrrolo[2,1-c]-[1,4]benzodiazepin-1-carboxylat,
Äthyl-5,6-dihydro-5-methyl-6-thioxo-4H-imidazo[1,5-a][1,4]benzodiazepin-3-carboxylat,
Äthyl-8-fluor-5,6-dihydro-5-methyl-6-thioxo-4H-imidazo[1,5-a][1,4]benzodiazepin-3-carboxylat,
8-Fluor-5,6-dihydro-5-methyl-6-oxo-4H-imidazo[1,5-a][1,4]benzodiazepin-3-carbonitril,
(S)-11,12,13,13a-Tetrahydro-9-oxo-9H-imidazo[1,5-a]-pyrrolo[2,1-c][1,4]benzodiazepin-1-carbonitril,
5,6-Dihydro-5-methyl-6-oxo-4H-imidazo[1,5-a][1,4]benzodiazepin-3-carbonitril,
Methyl-(S)-10,11,12,12a-tetrahydro-8-oxo-8H-imidazo[5,1-c]pyrrolo[1,2-a]thieno-[3,2-e][1,4]diazepin-1-carboxylat,
Äthyl-(S)-10,11,12,12a-tetrahydro-8-oxo-8H-imidazo[5,1-c]pyrrolo[1,2-a]thieno-[3,2-e][1,4]diazepin-1-carboxylat,
Äthyl-5,6-dihydro-5-methyl-6-oxo-4H-imidazo[1,5-a]thieno[2,3-f][1,4]diazepin-3-carboxylat und
Methyl-5,6-dihydro-5-methyl-6-oxo-4H-imidazo[1,5-a]thieno[2,3-f][1,4]diazepin-3-carboxylat.

Ferner sind auch die folgenden Verbindungen repräsentative Vertreter der von der Formel I umfaßten Stoffklasse:

4,5-Dihydro-5-methyl-3-propionyl-6H-imidazo[1,5-a][1,4]benzodiazepin-6-on,
Äthyl-5,6-dihydro-6-oxo-4H-imidazo[1,5-a][1,4]benzodiazepin-3-carboxylat,
Äthyl-8-fluor-5,6-dihydro-6-oxo-4H-imidazo[1,5-a][1,4]benzodiazepin-3-carboxylat,
Äthyl-8-chlor-5,6-dihydro-5-methyl-6-oxo-4H-imidazo[1,5-a][1,4]benzodiazepin-3-carboxylat,
Äthyl-8-brom-5,6-dihydro-5-methyl-6-oxo-4H-imidazo[1,5-a][1,4]benzodiazepin-3-carboxylat,
(S)-(+)-Äthyl-7-chlor-11,12,13,13a-tetrahydro-9-oxo-9H-imidazo[1,5-a]pyrrolo[2,1-c]-[1,4]benzodiazepin-1-carboxylat,
Äthyl-5,6-dihydro-5-methyl-6-oxo-4H-imidazo[1,5-a]thieno[3,4-f][1,4]diazepin-3-carboxylat,
(2-Hydroxyäthyl)-5,6-dihydro-5-methyl-6-oxo-4H-imidazo[1,5-a][1,4]benzodiazepin-3-carboxylat,
(2-Hydroxyläthyl)-8-fluor-5,6-dihydro-5-methyl-6-oxo-4H-imidazo[1,5-a][1,4]benzodiazepin-3-carboxylat,
(2-Hydroxyäthyl)-(S)-10,11,12,12a-tetrahydro-8-oxo-8H-imidazo[5,1-c]pyrrolo[1,2-a]-thieno[3,2-e][1,4]diazepin-1-carboxylat,
Äthyl-(R,S)-13,13a-dihydro-9-oxo-9H-imidazo[1,5-a]pyrrolo[2,1-c][1,4]benzodiazepin-1-carboxylat,

Äthyl-5,6-dihydro-5-methyl-8-trifluormethyl-6-oxo-4H-imidazo[1,5-a][1,4]benzodiazepin-3-carboxylat,
Äthyl-5,6-dihydro-5-methyl-7-trifluormethyl-6-oxo-4H-imidazo[1,5-a][1,4]benzodiazepin-3-carboxylat,
Äthyl-7-chlor-5,6-dihydro-8-fluor-5-methyl-6-oxo-4H-imidazo[1,5-a][1,4]benzodiazepin-3-carboxylat und
Äthyl-(S)-8-chlor-7-fluor-9-oxo-9H-11,12,13,13a-tetrahydro-imidazo[1,5-a]pyrrolo-[2,1-c][1,4]benzodiazepin-1-carboxylat.

Die Imidazo[1,5-a][1,4]diazepine der allgemeinen Formel I und ihre pharmazeutisch annehmbaren Säureadditionssalze können erfindungsgemäß hergestellt werden, indem man

a) eine Verbindung der allgemeinen Formel

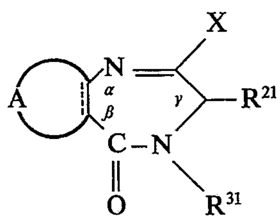

(II)

worin A und die gestrichelte Linie obige Bedeutung besitzen und X eine Abgangsgruppe bedeutet, und entweder $R^{21}$ Wasserstoff und $R^{31}$ niederes Alkyl bedeuten, oder $R^{21}$ und $R^{31}$ zusammen Trimethylen oder Propenylen bedeuten und das mit $\gamma$ bezeichnete Kohlenstoffatom die S- oder R,S-Konfiguration aufweist,

in Gegenwart einer Base mit einem Isocyanessigester der allgemeinen Formel

$$CN—CH_2—COOR^{41} \qquad \text{(III)}$$

worin $R^{41}$ Methyl, Äthyl oder Isopropyl bedeutet,

umsetzt, oder

b) eine Verbindung der allgemeinen Formel

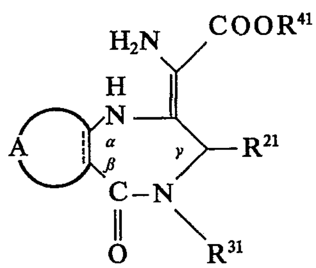

(IV)

worin A die gestrichelte Linie, $R^{21}$, $R^{31}$ und $R^{41}$ obige Bedeutung besitzen,
mit einem Formylierungsmittel behandelt, oder

c) eine Verbindung der allgemeinen Formel

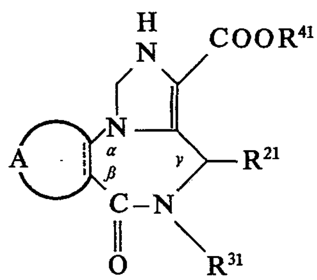

(V)

oder

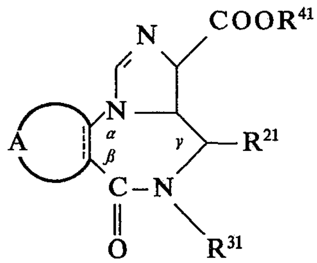

(VI)

worin A die gestrichelte Linie, $R^{21}$, $R^{31}$ und $R^{41}$ obige Bedeutung besitzen, dehydriert, oder

d) in einer Verbindung der allgemeinen Formel

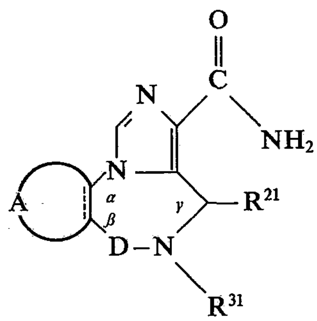

(VII)

worin A die gestrichelte Linie, D, $R^{21}$ und $R^{31}$ obige Bedeutung besitzen, die Carboxamido-Gruppe in die Nitril-Gruppe umwandelt, oder

e) eine Verbindung der allgemeinen Formel

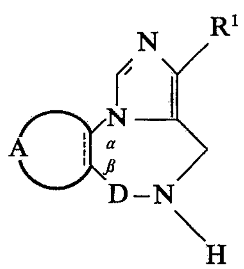

(Ia)

worin A die gestrichelte Linie, D und $R^1$ obige Bedeutung besitzen, an der sekundären Aminogruppe entsprechend substituiert, oder

f) in einer Verbindung der allgemeinen Formel

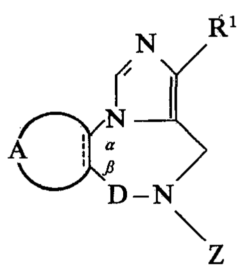

(VIII)

worin A, die gestrichelte Linie, D und $R^1$ obige Bedeutung besitzen, und Z eine Schutzgruppe bedeutet,
die Schutzgruppe abspaltet, oder

g) in einer Verbindung der allgemeinen Formel

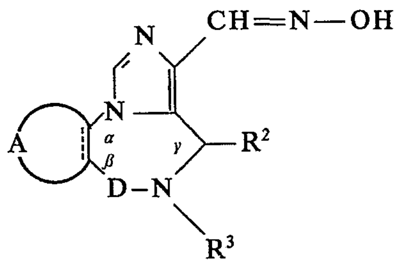

(IX)

worin A, die gestrichelte Linie, D, $R^2$ und $R^3$ obige Bedeutung besitzen,

die Oxim-Gruppe in die Nitril-Gruppe überführt, oder

h) in einer Verbindung der allgemeinen Formel

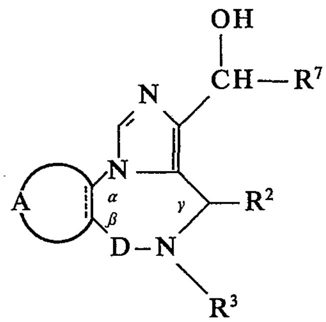

(X)

worin A, die gestrichelte Linie, D, $R^2$ und $R^3$ obige Bedeutung besitzen, und $R^7$ niederes Alkyl bedeutet,

die Hydroxyl-Gruppe in die Keto-Gruppe überführt, oder

i) eine Verbindung der allgemeinen Formel

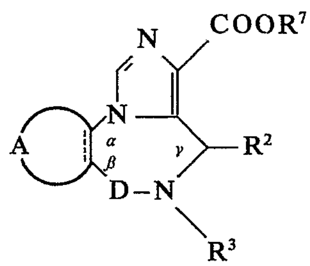

(XI)

worin A, die gestrichelte Linie, D, $R^2$, $R^3$ und $R^7$ obige Bedeutung besitzen,

umestert, oder

j) in einer Verbindung der allgemeinen Formel

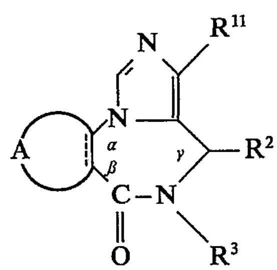

(Ib)

worin $R^{11}$ Cyano oder einen Rest der Formel $-COOR^4$ bedeutet, und A, die gestrichelte Linie, $R^2$, $R^3$ und $R^4$ obige Bedeutung besitzen,

die Carbonyl-Gruppe in die Thiocarbonyl-Gruppe überführt, und

k) erwünschtenfalls eine Verbindung der allgemeinen Formel I in ein pharmazeutisch annehmbares Säureadditionssalz überführt.

Gemäß Verfahrensvariante a) können Verbindungen der allgemeinen Formel I demnach aus Verbindungen der allgemeinen Formel II und Isocyanessigestern der allgemeinen Formel III hergestellt werden. Die in Formel II durch das Symbol X bezeichnete Abgangsgruppe ist beispielsweise eine leicht abspaltbare Phosphinylgruppe, z. B. eine Gruppe der Formel

$$-O\overset{\overset{\displaystyle O}{\|}}{P}(OR^4)_2 \quad \text{oder} \quad -O\overset{\overset{\displaystyle O}{\|}}{P}(NR^5R^6)_2$$

worin $R^4$ niederes Alkyl und $R^5$ und $R^6$ je niederes Alkyl, Allyl, Phenyl oder substituiertes Phenyl oder zusammen mit dem Stickstoffatom einen unsubstituierten oder substituierten heterocyclischen Ring mit 3—8 Gliedern (wie Morpholin) bedeuten,

ein Halogenatom, eine Alkylthiogruppe, eine Aralkylthiogruppe, eine N-Nitrosoalkylaminogruppe, eine Alkoxygruppe, eine Mercaptogruppe und dergleichen (wenn X eine Mercaptogruppe bedeutet, so handelt es sich bei der entsprechenden Verbindung der Formel II um die Iminothiol-Form des entsprechenden Thiolactams). Die Umsetzung der Verbindungen der Formeln II und III erfolgt in einem inerten Lösungsmittel, wie Dimethylformamid, Hexamethylphosphorsäuretriamid, Dimethylsulfoxid, Tetrahydrofuran oder irgend einem anderen geeigneten organischen Lösungsmittel und in Gegenwart einer Base, die genügend stark basisch ist, um das Anion des Isocyanessigesters der Formel III zu bilden. Als Basen eignen sich Alkalimetallalkoxide, wie Natriummethoxid oder Kalium-t-butoxid, Alkalimetallhydride, wie Natriumhydrid, Alkalimetallamide, wie Lithiumamid oder Lithiumdiisopropylamid, tertiäre Amine, wie Triäthylamin, und dergleichen. Die Reaktionstemperatur liegt zweckmäßigerweise zwischen etwa —40° C und etwa Raumtemperatur.

Gemäß Verfahrensvariante b) können Verbindungen der allgemeinen Formel I dadurch hergestellt werden, daß man Verbindungen der allgemeinen Formel IV mit einem Formylierungsmittel behandelt. Als Formylierungsmittel eignen sich für diesen Verfahrensaspekt niedere Alkylester der ortho-Ameisensäure und technische Äquivalente davon, beispielsweise ortho-Amide, wie N,N-Dimethylformamid-dimethylacetal, N,N,N',N',N'',N''-Hexamethylmethantriamin und dergleichen. Die Reaktion von Verbindungen der allgemeinen Formel IV mit dem Formylierungsmittel erfolgt zweckmäßigerweise in Gegenwart eines sauren Katalysators, beispielsweise einer organischen oder anorganischen Säure, wie p-Toluolsulfonsäure, Phosphorsäure und dergleichen, und bei Raumtemperatur oder oberhalb davon, beispielsweise zwischen etwa 25° und etwa 150° C.

Gemäß Verfahrensvariante c) können Verbindungen der allgemeinen Formel I durch Dehydrierung von Verbindungen der allgemeinen Formeln V oder VI hergestellt werden. Bevorzugte Reagenzien für diese Dehydrierung umfassen Mangandioxid, Palladium auf Kohle und elementarer Sauerstoff, wobei Luft-Sauerstoff bereits genügt, doch kann auch beispielsweise Kaliumpermanganat verwendet werden. Lösungsmittel, welche für diese Dehydrierung verwendet werden können, umfassen chlorierte Kohlenwasserstoffe, wie Methylenchlorid und Chloroform, aromatische Kohlenwasserstoffe, Dimethylformamid usw. Die Dehydrierung erfolgt bei Raumtemperatur oder oberhalb davon, zweckmäßigerweise zwischen etwa 25° und etwa 200° C.

Gemäß Verfahrensvariante d) können Verbindungen der allgemeinen Formel I hergestellt werden, indem man in Verbindungen der allgemeinen Formel VII die Carboxamido-Gruppe in die Nitril-Gruppe überführt. Diese Reaktion wird in an sich bekannter Weise mit einem geeigneten wasserentziehenden Mittel, wie Phosphorpentoxid oder dergleichen, in einem inerten organischen Lösungsmittel, wie Benzol, Toluol oder dergleichen, in einem Temperaturbereich von etwa 50° C bis Siedetemperatur des Reaktionsgemisches, durchgeführt. Vorzugsweise verwendet man handelsübliches an einem inerten Träger gebundenes Phosphorpentoxid als wasserentziehendes Mittel, und arbeitet in siedendem Toluol.

Gemäß Verfahrensvariante e) können Verbindungen der allgemeinen Formel I dadurch hergestellt werden, daß man Verbindungen der Formel Ia an der sekundären Aminogruppe in 5-Stellung entsprechend substituiert. Diese Substitution erfolgt nach an sich bekannten Methoden unter Verwendung eines den gewünschten Substituenten $R^{31}$ abgebenden Mittels, beispielsweise entsprechende organische Sulfonsäure-Alkylester (z. B. p-Toluolsulfonsäure-methylester), entsprechende Dialkylsulfate wie Dimethylsulfat und Diäthylsulfat, entsprechende Alkylhalogenide, wie Methyljodid, Äthyljodid oder Äthylbromid, und dergleichen. Die Verbindung der allgemeinen Formel Ia wird dabei zweckmäßigerweise in Form eines Alkalimetall-Salzes eingesetzt; man erreicht dies zweckmäßigerweise dadurch, daß man die Reaktion in Gegenwart einer starken Base ablaufen läßt oder die Verbindung der Formel Ia vor der Reaktion mit dem Alkylierungsmittel in ein Alkalimetallsalz überführt. Geeignete Basen sind Alkalimetall-alkoxide, wie Natriummethoxid oder Kalium-t-butoxid, Alkalimetall-hydride,

wie Natriumhydrid, Alkalimetall-amide, wie Lithiumamid oder Lithiumdiisopropylamid, und dergleichen. Die Reaktion wird zweckmäßigerweise in Gegenwart eines inerten organischen Lösungsmittels durchgeführt. Hierfür eignen sich z. B. Dimethylformamid, Dimethylsulfoxid, Essigester, niedere Alkanole und dergleichen; viele andere Lösungsmittel und auch Lösungsmittelgemische sind ebenfalls geeignet und ihre Auswahl bietet dem Fachmann keinerlei Schwierigkeiten. Die Reaktionstemperatur ist in ziemlich weiten Grenzen variierbar und wird in der Regel zwischen etwa Raumtemperatur und etwa der Siedetemperatur des Reaktionsgemisches liegen.

Gemäß Verfahrensvariante f) können Verbindungen der allgemeinen Formel I hergestellt werden, indem man in Verbindungen der allgemeinen Formel VIII die Schutzgruppe Z abspaltet. Es kommen dabei nur Schutzgruppen in Frage, die unter milden sauren Bedingungen abgespalten werden können, beispielsweise mit verdünnten wäßrigen Mineralsäuren, wie verdünnte Salzsäure oder verdünnte Schwefelsäure, Trifluoressigsäure oder dergleichen, gegebenenfalls unter Zusatz eines Lösungsvermittlers, wie Tetrahydrofuran, Dioxan, Essigsäure, N,N-Dimethylformamid oder dergleichen. Zweckmäßigerweise arbeitet man bei Temperaturen von etwa Raumtemperatur bis Siedetemperatur der Reaktionsmischung, wobei letzteres bevorzugt wird. Eine besonders geeignete Schutzgruppe ist die 2,4-Dimethoxybenzol-Gruppe, die zweckmäßigerweise mit Trifluoressigsäure abgespalten wird, vorzugsweise bei der Siedetemperatur des Reaktionsgemisches.

Gemäß Verfahrensvariante g) können Verbindungen der allgemeinen Formel I hergestellt werden, indem man in Verbindungen der allgemeinen Formel IX nach an sich bekannten Methoden die Oxim-Gruppe in die Nitril-Gruppe umwandelt. Diese Reaktion wird zweckmäßigerweise mit einem geeigneten wasserentziehenden Mittel, beispielsweise Carbonsäureanhydrid (z. B. Acetanhydrid und Propionsäureanhydrid), Sulfonsäurehalogenid (z. B. p-Toluolsulfonsäurechlorid) in Gegenwart einer Base (Triäthylamin oder dergleichen), und dergleichen, durchgeführt, wobei die Reaktionstemperatur von der verwendeten Methode abhängt. Vorzugsweise verwendet man Acetanhydrid und arbeitet bei der Siedetemperatur des Reaktionsgemisches.

Gemäß Verfahrensvariante h) können Verbindungen der allgemeinen Formel I nach an sich bekannten Methoden durch Oxidation der Hydroxylgruppe in Verbindungen der allgemeinen Formel X hergestellt werden. Als Oxidationsmittel eignen sich bei solchen Oxidationen übliche und jedem Fachmann bekannte Reagenzien, wie Mangandioxid, Kaliumpermanganat, Jones-Reagens und dergleichen. Geeignete Lösungsmittel sind, je nach verwendetem Oxidationsmittel, beispielsweise halogenierte Kohlenwasserstoffe, wie Methylenchlorid, Chloroform und dergleichen, aromatische Kohlenwasserstoffe, wie Benzol, Toluol und dergleichen, Dimethylformamid, Aceton, Wasser, usw. Die Oxidation erfolgt bei Temperaturen von etwa 0°C bis Siedetemperatur des Reaktionsgemisches, je nach verwendeter Methode. In einer bevorzugten Ausführungsform verwendet man Mangandioxid in einem halogenierten Kohlenwasserstoff, wie Methylenchlorid, Chloroform und dergleichen, zweckmäßigerweise bei Raumtemperatur.

Gemäß Verfahrensvariante i) können Verbindungen der allgemeinen Formel I durch Umesterung von Verbindungen der allgemeinen Formel XI hergestellt werden, d. h. daß in Verbindungen der allgemeinen Formel XI der mit $R^7$ bezeichnete Alkylrest gegen einen Rest $R^4$ ausgetauscht wird, wobei natürlich $R^7$ und $R^4$ jeweils verschieden sind. Sofern $R^7$ in Formel XI Methyl, Äthyl oder Isopropyl bedeutet, fallen die Verbindungen der Formel XI unter die eingangs definierte Formel I. $R^7$ kann jedoch natürlich auch eine andere niedere Alkylgruppe bedeuten.

Diese Umesterung erfolgt in an sich bekannter Weise durch Umsetzen einer Verbindung der allgemeinen Formel XI mit einem dem gewünschten Rest $R^4$ entsprechenden Alkohol, d. h. Methanol bzw. Äthanol bzw. Isopropanol bzw. Äthylenglykol, bei Raumtemperatur oder unter Erwärmen auf eine Temperatur von etwa 25 bis 150°C. Vorzugsweise wird die Umesterung in Gegenwart einer Base durchgeführt, wobei sich im vorliegenden Fall insbesondere Kaliumcyanid oder ähnliche schwache Basen eignen. Als Lösungsmittel dient vorzugsweise der dem Rest $R^4$ im gewünschten Endprodukt der Formel I entsprechende Alkohol; die Umesterung kann jedoch auch in einem inerten organischen Lösungsmittel erfolgen, beispielsweise in einem aromatischen Kohlenwasserstoff, wie Benzol oder Xylol, einem Äther, wie Dioxan, Tetrahydrofuran oder Äthylenglykol-dimethyläther, in Dimethylformamid, in Dimethylsulfoxid oder dergleichen. Bei dieser Umesterung kann man nicht nur einen niedriger siedenden Alkohol durch einen höher siedenden Alkohol, sondern auch einen höher siedenden Alkohol durch einen niedriger siedenden Alkohol ersetzen; es ist beispielsweise ohne weiteres möglich Äthyl-5,6-dihydro-5-methyl-6-oxo-4H-imidazo[1,5-a][1,4]benzodiazepin-3-carboxylat mittels Methanol in Methyl-5,6-dihydro-5-methyl-6-oxo-4H-imidazo[1,5-a][1,4]benzodiazepin-3-carboxylat überzuführen.

Die Umesterung kann indessen ohne weiteres auch mehrstufig durchgeführt werden, beispielsweise dadurch, daß man die eingesetzte Verbindung der Formel XI zur entsprechenden freien Carbonsäure hydrolysiert, aus dieser ein reaktionsfähiges funktionelles Derivat (z. B. ein Säurechlorid oder dergleichen) herstellt und anschließend dieses reaktionsfähige Carbonsäurederivat mit dem der Bedeutung von $R^4$ in der gewünschten Verbindung der Formel I entsprechenden Alkohol zur Reaktion bringt.

Gemäß Verfahrensvariante j) können Verbindungen der allgemeinen Formel Ib in entsprechende Verbindungen der Formel I, worin D $>C=S$ bedeutet, übergeführt werden, und zwar durch Behandeln

mit einem Schwefelungsmittel, was in an sich bekannter Weise erfolgen kann. Beispielsweise kann man als Schwefelungsmittel Phosphorpentasulfid verwenden, wobei dieses vorzugsweise im Überschuß eingesetzt wird und die Reaktion vorteilhafterweise in einem inerten organischen Lösungsmittel, wie Dioxan, Methylenchlorid oder dergleichen, in Gegenwart von Triäthylamin bei einer Temperatur von etwa 50°C bis zur Rückflußtemperatur des Reaktionsgemisches erfolgt. Als Schwefelungsmittel eignen sich indessen auch Verbindungen wie 2,4-Bis(p-methoxyphenyl)-1,3,2,4-dithiaphosphetan-2,4-disulfid; derartige Schwefelungsmittel werden ungefähr in der berechneten Menge eingesetzt, und die Reaktion erfolgt in Gegenwart eines inerten Lösungsmittels, wie Toluol, Xylol, zweckmäßigerweise bei Rückflußtemperatur des Reaktionsgemisches oder in Hexamethyl-phosphorsäuretriamid zwischen etwa 60 und 110°C.

Gemäß Verfahrensvariante k) können Verbindungen der allgemeinen Formel I erfindungsgemäß in pharmazeutisch annehmbare Säureadditionssalze übergeführt werden. Die Herstellung von solchen pharmazeutisch annehmbaren Säureadditionssalze erfolgt nach allgemein üblichen Methoden. Es kommen sowohl Salze mit anorganischen als auch Salze mit organischen Säuren in Betracht, beispielsweise Hydrochloride, Hydrobromide, Sulfate, Methansulfonate, p-Toluolsulfonate, Oxalate und dergleichen.

Die als Ausgangsstoffe verwendeten Verbindungen der allgemeinen Formel II können ausgehend von Verbindungen der Formel

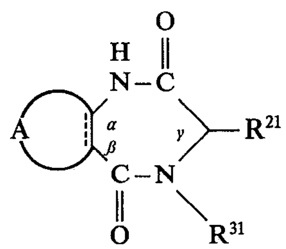

(XII)

worin A, die gestrichelte Linie, $R^{21}$ und $R^{31}$ obige Bedeutung besitzen,

hergestellt werden, und zwar nach Methoden, welche an sich bekannt sind, vgl. z. B. die Belgischen Patentschriften No. 802 233, 833 249 und 865 653, die Amerikanische Patentschrift No. 3 681 341 und J. Org. Chemistry 29, 231 (1964).

Verschiedene der weiter unten folgenden Beispiele enthalten detaillierte Angaben betreffend die Herstellung von Verbindungen der allgemeinen Formel II aus Verbindungen der allgemeinen Formel XII.

Die Verbindungen der Formel XII ihrerseits sind bekannt oder nach an sich bekannten Methoden leicht herstellbar, beispielsweise durch Umsetzen eines entsprechenden Carbonsäureanhydrids der allgemeinen Formel

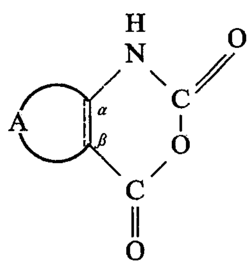

(XIII)

worin A und die gestrichelte Linie obige Bedeutung besitzen,

mit einer Aminosäure der allgemeinen Formel

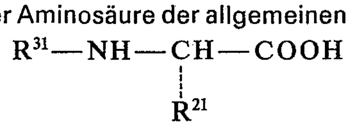

(XIV)

worin $R^{21}$ und $R^{31}$ obige Bedeutung besitzen.

Verbindungen der allgemeinen Formel XII, worin $R^{21}$ Wasserstoff und $R^{31}$ niederes Alkyl bedeuten, können aber auch ausgehend von Verbindungen der allgemeinen Formel

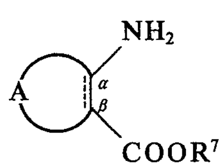

(XV)

worin A, die gestrichelte Linie und $R^7$ obige Bedeutung besitzen, hergestellt werden, beispielsweise durch Behandeln einer solchen Verbindung mit einem reaktiven Derivat einer $\alpha$-Halogenessigsäure, z. B. $\alpha$-Chloressigsäurechlorid, und Umsetzen des erhaltenen Zwischenproduktes mit einem niederen Alkylamin, wie Methylamin, Äthylamin und dergleichen. Man erhält somit Verbindungen der allgemeinen Formel

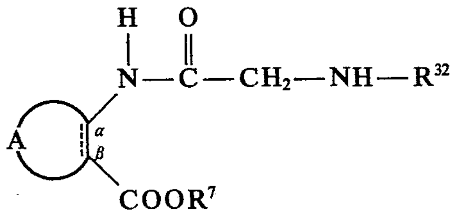

(XVI)

worin A, die gestrichelte Linie und $R^7$ obige Bedeutung besitzen, und $R^{32}$ niederes Alkyl bedeuten. Durch Cyclisieren von Verbindungen der allgemeinen Formel XVI gelangt man zu Verbindungen der allgemeinen Formel XII, worin $R^{21}$ Wasserstoff und $R^{31}$ niederes Alkyl bedeuten. Diese Cyclisierung erfolgt beispielsweise durch kurzzeitiges Erhitzen einer entsprechenden Verbindung der allgemeinen Formel XVI auf Temperaturen von etwa 100 bis etwa 300° C.

Es ist auch möglich eine Verbindung der Formel XV mit einem reaktiven Derivat einer Carbonsäure der allgemeinen Formel

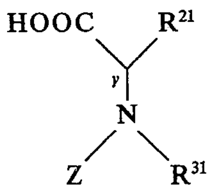

(XVII)

worin $R^{21}$, $R^{31}$ und Z obige Bedeutung besitzen, beispielsweise einem Carbonsäurechlorid oder dergleichen, umzusetzen. Nach Entfernen der mit Z bezeichneten Schutzgruppe aus einer so erhaltenen Verbindung der allgemeinen Formel

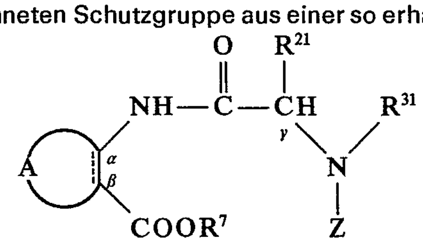

(XVIII)

worin A, die gestrichelte Linie, $R^{21}$, $R^{31}$, $R^7$ und Z obige Bedeutung besitzen, und Cyclisieren, in Analogie zur Herstellung von Verbindungen der Formel XII aus solchen der Formel XVI, erhält man eine Verbindung der allgemeinen Formel XII.

Um eine Verbindung der allgemeinen Formel

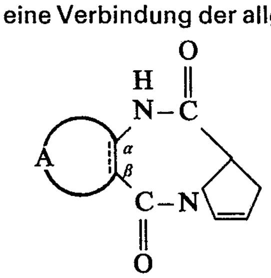

(XIIa)

worin A und die gestrichelte Linie obige Bedeutung besitzen, zu erhalten, kann man auch in an sich bekannter Weise in einer Verbindung der allgemeinen Formel

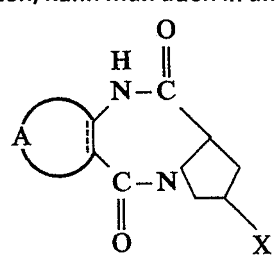

(XIX)

worin A und die gestrichelte Linie obige Bedeutung besitzen, und X eine Abgangsgruppe bedeutet,

die mit X bezeichnete Abgangsgruppe eliminieren. Als Abgangsgruppen kommen beispielsweise Sulfonsäuregruppen, wie Methansulfonyloxy, p-Toluolsulfonyloxy oder dergleichen, Halogenatome, wie Chlor, Brom und Jod, oder dergleichen, in Frage. Die Abspaltung erfolgt mit einer Base, wie Natriumhydrid, in einem inerten organischen Lösungsmittel, wie Dimethylformamid.

Verbindungen der Formel XIX können in Analogie zur Herstellung von Verbindungen der allgemeinen Formel XII aus Verbindungen der allgemeinen Formel XIII und XIV oder aus solchen der Formel XVIII hergestellt werden.

Verbindungen der allgemeinen Formeln IV, V und VI lassen sich nach an sich bekannten Methoden (vgl. die Belgischen Patentschriften Nos. 833 248 und 839 364) ausgehend von Verbindungen der allgemeinen Formel II gemäß nachfolgendem Reaktionsschema, worin A, die gestrichelte Linie, $R^{21}$, $R^{31}$, $R^{41}$ und X obige Bedeutung besitzen, herstellen:

Reaktionsschema

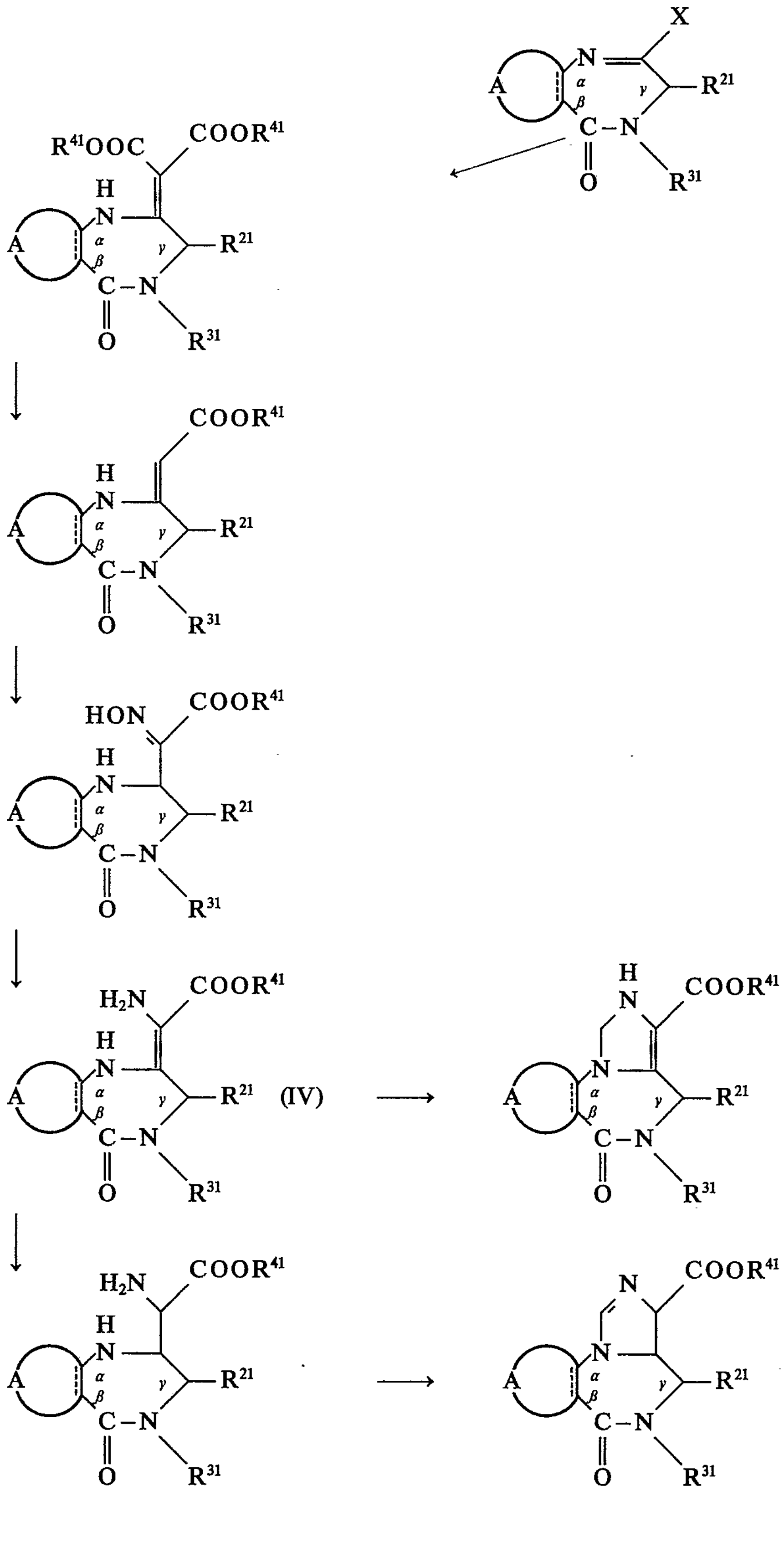

(II)

(IV)

(V)

(VI)

Verbindungen der allgemeinen Formel VII können durch Behandeln von Verbindungen der allgemeinen Formel

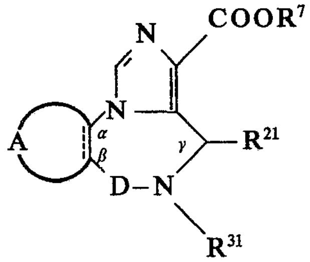

(XIa)

worin A, die gestrichelte Linie, D, $R^{21}$, $R^{31}$ und $R^7$ obige Bedeutung besitzen, mit Ammoniak nach an sich bekannten und jedem Fachmann geläufigen Methoden leicht hergestellt werden. Die Amidbildung kann indessen ohne weiteres auch mehrstufig durchgeführt werden, beispielsweise dadurch, daß man die eingesetzte Verbindung der allgemeinen Formel XIa zur entsprechenden freien Carbonsäure hydrolysiert, aus dieser ein reaktionsfähiges funktionelles Derivat (z. B. ein Säurechlorid, ein Säureimidazolid oder dergleichen) herstellt und anschließend dieses reaktionsfähige Carbonsäurederivat in an sich bekannter Weise mit Ammoniak umsetzt.

Sofern $R^7$ in Formel XIa Methyl, Äthyl oder Isopropyl bedeutet, fallen die Verbindungen der allgemeinen Formel XIa unter die eingangs definierte allgemeine Formel I. $R^7$ kann aber ohne weiteres auch eine andere niedere Alkylgruppe bedeuten.

Verbindungen der Formel VIII können ausgehend von Verbindungen der allgemeinen Formel

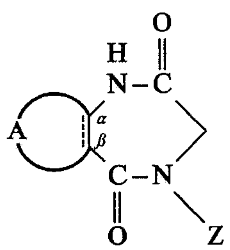

(XX)

worin A, die gestrichelte Linie und Z obige Bedeutung besitzen, in Analogie zu den weiter oben beschriebenen Methoden zur Herstellung von Verbindungen der allgemeinen Formel I, worin $R^2$ Wasserstoff und $R^3$ niederes Alkyl bedeuten, hergestellt werden, und zwar in Analogie zu Verfahrensvarianten a), b), c), d), g), h), i) und j) und zu den für die Herstellung der entsprechenden Ausgangsprodukte beschriebenen Methoden.

Die als Ausgangsstoffe verwendeten Verbindungen der allgemeinen Formel IX sind aus Carbonsäureestern der allgemeinen Formel XI leicht zugänglich. Beispielsweise kann man einen Carbonsäureester der obigen allgemeinen Formel XI mit einem Reduktionsmittel, wie Lithiumborhydrid, in einem inerten organischen Lösungsmittel, wie Diäthyläther, Tetrahydrofuran, Dimethoxyäthan oder dergleichen, zum entsprechenden primären Alkohol reduzieren, den man anschließend mit einem milden Oxidationsmittel, wie Mangandioxid oder dergleichen, in einem inerten organischen Lösungsmittel, wie Methylenchlorid, Chloroform oder dergleichen, in den entsprechenden Aldehyd der allgemeinen Formel

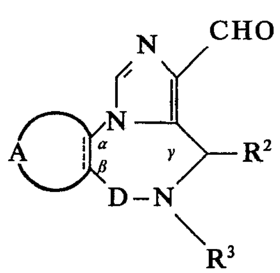

(XXI)

worin A, die gestrichelte Linie, D, $R^2$ und $R^3$ obige Bedeutung besitzen, umwandeln kann.

Durch Behandeln eines Aldehyds der allgemeinen Formel XXI mit Hydroxylamin erhält man das entsprechende Oxim der allgemeinen Formel IX. Zweckmäßigerweise verwendet man Hydroxylaminhydrochlorid als Reagens und arbeitet in einem inerten Lösungsmittel, wie beispielsweise Wasser, Methanol oder Äthanol, Mischungen davon mit Wasser oder dergleichen, in Gegenwart einer Base, wie Kalium- und Natriumcarbonat, Kalium- oder Natriumhydroxid, Triäthylamin oder dergleichen;

oder in einem basischen Lösungsmittel, wie Pyridin, Triäthylamin oder dergleichen, in einem Temperaturbereich von etwa Raumtemperatur bis Siedetemperatur des Reaktionsgemisches.

Verbindungen der allgemeinen Formel X sind aus Verbindungen der allgemeinen Formel XXI leicht zugänglich. Nach allgemein bekannten und jedem Fachmann geläufigen Methoden kann man einen Aldehyd der allgemeinen Formel XXI mit einer den Rest $R^7$ liefernden metallorganischen Verbindung umsetzen. Als metallorganische Verbindungen kommen in erster Linie Grignard-Verbindungen, wie Methyl-magnesiumjodid,Äthyl-magnesiumjodid, Isopropyl-magnesiumbromid, n-Propyl-magnesiumbromid, n-Butyl-magnesiumchlorid und dergleichen, und Lithiumalkyl-Verbindungen, wie Methyllithium, Äthyllithium, Isopropyllithium, n-Propyllithium, n-Butyllithium und dergleichen, in Frage. Als Lösungsmittel eignen sich Äther, wie Diäthyläther, Tetrahydrofuran, t-Butyl-methyläther, Mischungen davon, und dergleichen, und bei Verwendung von Lithiumalkyl-Verbindungen auch Kohlenwasserstoffe, wie Pentan, Hexan, Heptan, Benzol, Toluol und dergleichen. Zweckmäßigerweise arbeitet man bei der Siedetemperatur des Reaktionsgemisches, man kann aber auch unterhalb davon arbeiten, beispielsweise bei Raumtemperatur.

Verbindungen der allgemeinen Formel XI, worin $R^7$ nicht Methyl, Äthyl und Isopropyl bedeutet, können in Analogie zu den weiter oben beschriebenen Methoden zur Herstellung von Verbindungen der allgemeinen Formel I, worin $R^1$ einen Rest $-COOR^{41}$ bedeutet und $R^{41}$ obige Bedeutung besitzt, hergestellt werden; und zwar in Analogie zu Verfahrensvarianten a), b), c), e), f) und j) und zu den für die Herstellung der entsprechenden Ausgangsprodukte beschriebenen Methoden; sie sind neu und bilden ebenfalls Gegenstand der vorliegenden Erfindung.

Auch die als Ausgangsstoffe verwendeten Verbindungen der allgemeinen Formeln II, IV, V, VI, VII, VIII, IX und X sind neu.

Wie eingangs erwähnt, sind die Verbindungen der allgemeinen Formel I neu und besitzen äußerst wertvolle pharmakodynamische Eigenschaften. Sie weisen nur eine geringe Toxizität auf, und es hat sich gezeigt, daß sie eine ausgeprägte Affinität zu den zentralen Benzodiazepin-Rezeptoren haben und die zentral dämpfenden, muskelrelaxierenden, ataktischen, blutdrucksenkenden und atemdepressiven Eigenschaften von tranquilisierend wirksamen 1,4-Benzodiazepinen zu antagonisieren vermögen.

Die Affinität von Verbindungen der allgemeinen Formel I zu den zentralen Benzodiazepin-Rezeptoren wurde nach der in Life Science 20, 2101—2110 (1977) und Science 198, 849—851 (1977) beschriebenen Methode festgestellt. Nach dieser Methode ermittelt man die Hemmung der Bindung von tritiiertem Diazepam an die spezifischen Benzodiazepin-Rezeptoren im cerebralen Cortex durch die jeweiligen Testsubstanzen. Man bezeichnet als $IC_{50}$ (»50% inhibiting concentration«) diejenige Konzentration der jeweiligen Testsubstanz, welche eine 50%ige Hemmung der spezifischen Bindung des tritiierten Diazepams an die spezifischen Benzodiazepin-Rezeptoren im cerebralen Cortex bewirkt.

Eine der typischen Eigenschaften von tranquilisierend wirksamen 1,4-Benzodiazepinen in Versuchstieren ist ihre ausgeprägte anticonvulsive Wirkung, welche beispielsweise im bekannten und allgemein anerkannten Pentetrazol-Test nachgewiesen werden kann. Diese Eigenschaft wurde verwendet, um den nachstehend beschriebenen Test auszuarbeiten, der es erlaubt, Verbindungen zu ermitteln, die die zentralen Eigenschaften von tranquilisierend wirksamen 1,4-Benzodiazepinen zu antagonisieren vermögen.

In diesem Test verabreicht man Mäusen eine Stunde vor dem Pentetrazol (120 mg/kg, i.p.) 5 mg/kg (i.p.) Diazepam (d. h. eine supramaximale Dosis, die im Pentetrazol-Test an mehr als 900 Mäusen alle Versuchstiere vor krampfartigen Anfällen schützte) und 15 Minuten vor dem Pentetrazol die zu testende Verbindung p.o. Die antagonistische Wirksamkeit der untersuchten Verbindungen, d. h. ihr Vermögen, die Wirkung des Diazepams im Pentetrazol-Test aufzuheben, wird ermittelt, indem man die Mäuse auszählt, die in diesem Test krampfartige Anfälle erleiden.

In der nachstehenden Tabelle werden die Resultate dargestellt, welche mit repräsentativen Vertretern der durch die allgemeinen Formel I definierten Verbindungsklasse in dem vorstehend geschilderten Versuch erhalten worden sind. Angegeben wird für jede der darin figurierenden Verbindung der $ED_{50}$-Wert. Als $ED_{50}$ bezeichnet man die Menge der jeweiligen Testverbindung in mg/kg (p.o.), die bei 50% der Tiere den Diazepam-Effekt in obigem Versuch aufhebt. Außerdem enthält die Tabelle die oben definierten $IC_{50}$-Werte für alle darin angegebenen Testverbindungen sowie Angaben über die akute Toxizität einiger dieser Verbindungen ($DL_{50}$ in mg/kg bei einmaliger oraler Verabreichung an Mäusen).

Tabelle

Imidazo [1,5-a][1,4]benzodiazepin-Derivate, d. h. Verbindungen der Formel I, worin A die Gruppe (a) bedeutet:

| $R^1$ | $R^2$ | $R^3$ | Konfiguration | $R^5$ | $R^6$ | D | $IC_{50}$ in pM/1 | $ED_{50}$ in mg/kg p. o. | $DL_{50}$ in mg/kg p. o. |
|---|---|---|---|---|---|---|---|---|---|
| $-COOC_2H_5$ | H | $-CH_3$ | — | F | H | C=O | 2,5 | 5,8 | >5000 |
| $-COOC_2H_5$ | $-(CH_2)_3-$ | | S | H | H | C=O | 6,4 | 9,3 | 1250 – 2500 |
| $-COOC_2H_5$ | H | $-CH_3$ | — | H | H | C=O | 3,0 | 10,9 | >5000 |
| $-COOC_2H_5$ | H | $-CH_3$ | — | H | H | C=S | 6,0 | 8,9 | |
| $-COOCH_3$ | H | $-CH_3$ | — | H | H | C=O | 9,0 | 13,5 | |
| $-COOCH(CH_3)_2$ | H | $-CH_3$ | — | H | H | C=O | 12,0 | 7,0 | |
| $-COOCH_3$ | H | $-CH_3$ | — | F | H | C=O | 6,0 | 4,9 | |
| $-COOCH(CH_3)_2$ | H | $-CH_3$ | — | F | H | C=O | 3,9 | 6,6 | >5000 |
| $-COOC_2H_5$ | H | $-CH_3$ | — | H | F | C=O | 2,0 | 2,1 | >5000 |
| $-COOC_2H_5$ | H | $-CH_3$ | — | H | Cl | C=O | 3,0 | 0,48 | |
| $-COOC_2H_5$ | H | $-CH_3$ | — | H | $-CH_3$ | C=O | 5,0 | 7,1 | |
| $-COOCH_3$ | $-(CH_2)_3-$ | | S | H | H | C=O | 32,0 | 3,5 | 2000 – 4000 |
| $-COOCH(CH_3)_2$ | $-(CH_2)_3-$ | | S | H | H | C=O | 3,0 | 18,6 | |
| $-COOC_2H_5$ | $-(CH_2)_3-$ | | S | F | H | C=O | 6,0 | 8,9 | |
| $-COOC_2H_5$ | $-(CH_2)_3-$ | | S | H | Cl | C=O | 1,7 | 1,0 | 750 – 1500 |
| $-COOC_2H_5$ | $-(CH_2)_3-$ | | S | H | $-CH_3$ | C=O | 5,0 | 6,5 | |
| $-COOC_2H_5$ | H | $-CH_3$ | — | F | H | C=S | 2,5 | 6,3 | |
| $-CN$ | H | $-CH_3$ | — | H | H | C=O | 100,0 | 2,6 | 1250 |
| $-CN$ | H | $-CH_3$ | — | F | H | C=O | 93,0 | 4,2 | |
| $-CN$ | $-(CH_2)_3-$ | | S | H | H | C=O | 31,0 | 12,1 | |
| $-COOC_2H_5$ | $-CH=CH-CH_2-$ | | R/S | H | H | C=O | 2,3 | 3,9 | |
| $-COOC_2H_5$ | $-CH=CH-CH_2-$ | | R/S | H | F | C=O | 2,1 | 1,8 | 500 – 1000 |

Imidazo[1,5-a]thieno[2,3-f][1,4]diazepin-Derivate, d. h. Verbindungen der Formel I, worin A die Gruppe (b) bedeutet:

| $R^1$ | $R^2$ | $R^3$ | D | Konfiguration | $IC_{50}$ in µM/l | $ED_{50}$ in mg/kg p.o. |
|---|---|---|---|---|---|---|
| $-COOCH_3$ | $-(CH_2)_3-$ | | C=0 | S | 2,1 | 0,22 |
| $-COOC_2H_5$ | $-(CH_2)_3-$ | | C=0 | S | 0,9 | 1,3 |
| $-COOC_2H_5$ | H | $-CH_3$ | C=0 | — | 1,1 | 12,6 |
| $-COOCH_3$ | H | $-CH_3$ | C=0 | — | 1,2 | 0,83 |

Auch aus den nachstehend beschriebenen Versuchen geht hervor, daß eine Verbindung der Formel I, das Äthyl-5,6-dihydro-5-methyl-6-oxo-4H-imidazo[1,5-a][1,4]benzodiazepin-3-carboxylat (nachstehend »Testsubstanz« genannt), die zentralen Wirkungen des bekannten 1,4-Benzodiazepin-Derivats Diazepam antagonisiert:

Für diese Versuche wurden männliche Albinoratten (Körpergewicht etwa 165 g) verwendet. Verabreicht man diesen Tieren Diazepam in einer Dosis von 5 mg/kg i.v., so schlafen sie sofort ein, zeigen keinen Aufrichtereflex mehr, schlafen mindestens 60 Minuten in Seitenlage und wachen nach etwa $1^1/_2$ Stunden langsam auf; verabreicht man den Tieren Diazepam in einer Dosis von 10 mg/kg i.p., so schlafen sie innerhalb weniger Minuten ein, schlafen während ca. 2—3 Stunden und wachen dann langsam wieder auf. Gibt man den Tieren 5 oder 15 Minuten nach der Verabreichung von 5 mg/kg i.v. Diazepam oder 30 Minuten nach Verabreichung von 10 mg/kg i.p. Diazepam die Testsubstanz in einer Dosis von 5 mg/kg i.v., so stehen die Tiere sofort auf und laufen umher, wobei diese Wachphase während 30 Minuten bis 1 Stunde anhält.

Gibt man den Tieren die Testsubstanz in einer Dosis von 20 mg/kg i.p. so bleiben die Tiere wach und zeigen ein normales Verhalten; verabreicht man ihnen dann nach 30 Minuten Diazepam in einer Dosis von 5 mg/kg i.v., so zeigen sie in den ersten 2 Minuten nach der Injektion leichte Sedation und Ataxie, aber diese Symptome verschwinden rasch, und die Wachphase hält während mindestens 30 Minuten an.

Aus den nachstehend beschriebenen Versuchen geht hervor, daß eine Verbindung der allgemeinen Formel I, das Äthyl-(S)-11,12,13,13a-tetrahydro-9-oxo-9H-imidazo[1,5-a]pyrrolo[2,1-c][1,4]benzodiazepin-1-carboxylat (nachstehend »Testsubstanz« genannt), die zentralen Wirkungen des bekannten 1,4-Benzodiazepin-Derivats Flunitrazepam antagonisiert:

Es wurden vier Versuche an wachen, curarisierten, künstlich beatmeten Ratten durchgeführt. Als Meßgröße diente das bipolar abgeleitete EEG des dorsalen Hippocampus. 10 Minuten nach Ableitungsbeginn wurde Flunitrazepam in einer Dosis von 0,1 mg/kg intravenös verabreicht. Die Testsubstanz wurde in einer Dosis von 1 mg/kg einem Tier 10 Minuten und 3 Tieren 30 Minuten nach der Verabreichung von Flunitrazepam ebenfalls intravenös gegeben. Die anfallenden EEG's wurden nach den Regeln der Fouriertransformation fortlaufend im Frequenzbereich analysiert, wobei sich jedes der errechneten Spektren auf 1 Minute bezog.

Das Normal-EEG aus dem Hippocampus der wachen, curarisierten Ratte ist durch einen stabilen Thetarhythmus von 3,75—4,25 Hertz gekennzeichnet, der mindestens 3 Stunden nach Beginn der künstlichen Beatmung erhalten bleibt. Flunitrazepam unterdrückt, in der angegebenen Dosierung, diesen Thetarhythmus etwa 15 Minuten lang vollständig. Danach erscheint ein auf 2,75—3,25 Hertz verlangsamter Rhythmus, der sich kontinuierlich beschleunigt und 1 bis 1,5 Stunden nach der Injektion wieder die Normalfrequenz von 4 Hertz erreicht.

Die Testsubstanz führte in der angegebenen Dosierung und zu den angegebenen Zeiten nach Flunitrazepam gegeben, zu einer sofortigen, langanhaltenden Normalisierung des beschriebenen Thetarhythmus bei 4 Hertz. Wurde die Testsubstanz 10 Minuten nach Flunitrazepam gegeben, so rief sie den durch Flunitrazepam unterdrückten Rhythmus wieder hervor, wobei dieser von Anfang an die Normalfrequenz aufwies. Wurde die Testsubstanz 30 Minuten nach Flunitrazepam gegeben, so beschleunigte sie den bereits, jedoch verlangsamt, wiederaufgetretenen Rhythmus sofort auf seine Normalfrequenz von 4 Hertz.

Wie bereits erwähnt, antagonisieren Verbindungen der Formel I die zentral dämpfenden, muskelrelaxierenden, ataktischen, blutdrucksenkenden und atemdepressiven Eigenschaften von tranquilisierend wirksamen 1,4-Benzodiazepinen. Letztere stehen in der Therapie in vielfältigem Gebrauch und werden oft in hohen Dosierungen angewandt, so daß die oben erwähnten Wirkungen auch als Nebenwirkungen stark hervortreten können. Die Verbindungen der Formel I können bei Intoxikationen, bei welchen übermäßige Einnahme von tranquilisierend wirksamen 1,4-Benzodiazepinen beteiligt ist, als Antidot verwendet werden. Sie eignen sich auch zur Abkürzung einer durch tranquilisierend wirksame 1,4-Benzodiazepine eingeleiteten Anästhesie in der Chirurgie und in der Geburtshilfe. Bei Neugeborenen kann eine eventuelle Atemdepression, die auf die Gabe von tranquilisierend wirksamen 1,4-Benzodiazepinen an die Mutter zurückgeht, aufgehoben werden. Die Verbindungen der Formel I können auch dazu verwendet werden, bei 1,4-Benzodiazepinen, die in anderen Indikationsgebieten eingesetzt werden, die in einem solchen Fall unerwünschten Wirkungen auf das zentrale Nervensystem zu unterdrücken. Beispiele derartiger, in anderen Indikationsgebieten einsetzbarer 1,4-Benzodiazepine sind die in den britischen Patentschriften No. 1 444 529 und 1 474 305 beschriebenen schistosomiazid wirksamen 1,4-Benzodiazepin-Derivate der allgemeinen Formeln

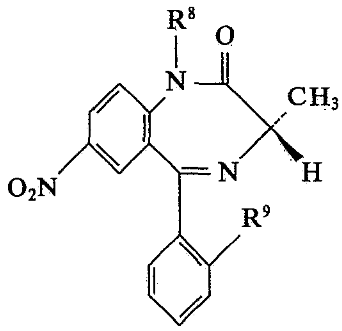

(XXII)

worin $R^8$ Wasserstoff oder niederes Alkyl und $R^9$ Halogen bedeuten,

und

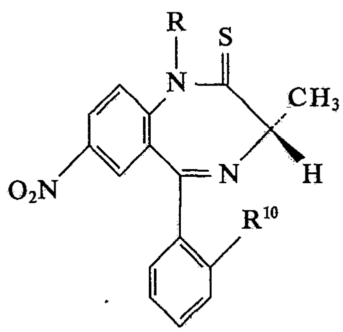

(XXIII)

worin R Wasserstoff oder niederes Alkyl und $R^{10}$ Wasserstoff, Halogen oder Trifluormethyl bedeuten,

wie (+)-5-(o-Chlorphenyl)-1,3-dihydro-3-methyl-7-nitro-2H-1,4-benzodiazepin-2-on.

Die nachfolgenden Experimente zeigen, daß repräsentative Vertreter der von der allgemeinen Formel I umfaßten Stoffklasse, welche die starken, jedoch unerwünschten zentralen Wirkungen des hochwirksamen Schistosomiazids (+)-5-(o-Chlorphenyl)-1,3-dihydro-3-methyl-7-nitro-2H-1,4-benzodiazepin-2-on auf das zentrale Nervensystem unterdrücken, dessen schistosomiazide Wirkung in keiner Weise beeinträchtigen.

Mäuse und Goldhamster werden subcutan mit 60 Cercarien von Schistosoma mansoni infiziert. Ca. 42 Tage nach der Infektion werden sie mit einer einmaligen Dosis der zu prüfenden Präparate oral behandelt. Pro Präparat und Dosierung werden 5 Tiere eingesetzt. Als Kontrolle dienen 10 unbehandelte Tiere. Die Tiere werden 2 (Hamster) bzw. 3 (Mäuse) Wochen nach der Behandlung getötet und seziert. Wurmpaare in Mesenterialvenen, Pfortader und Leber werden herauspräpariert, gezählt und der Zustand der Würmer (lebend-tot) wird registriert. Eine schistosomiazide Wirkung eines Präparats zeigt sich im Auftreten toter Würmer in den Gefäßen der Leber. In unbehandelten Kontrolltieren finden sich nie tote Würmer. Zur Auswertung errechnet man den prozentualen Anteil toter Wurmpaare in den Gefäßen der Leber bei infizierten, behandelten Tieren.

Zur Prüfung der in vitro Wirkung von Präparaten werden Wurmpaare von Schistosoma mansoni aus Mäusen isoliert und in einem Nährmedium bei 37°C inkubiert. Präparate werden als Lösung oder als Suspension beigegeben. Die Beweglichkeit der Würmer während der Versuchsdauer von 120 Stunden unter dem Mikroskop beobachtet und registriert. Eine schistosomiazide Wirkung eines Präparats zeigt sich am mehr oder weniger raschen Verlust der Motilität der Würmer. Kontrollwürmer in Nährmedium ohne Präparatzusatz behalten ihre normale Beweglichkeit während der ganzen Versuchsdauer von 120 Stunden.

Die folgenden repräsentativen Verbindungen der Formel I wurden in den oben beschriebenen Versuchen auf mögliche Beeinträchtigung der schistosomiaziden Wirkung von (+)-5-(o-Chlorphenyl)-1,3-dihydro-3-methyl-7-nitro-2H-1,4-benzodiazepin-2-on (Verbindung S) geprüft.

Äthyl-8-fluor-5,6-dihydro-5-methyl-6-oxo-4H-imidazo[1,5-a][1,4]benzo-

diazepin-3-carboxylat (Verbindung A);

Äthyl-(S)-11,12,13,13a-tetrahydro-3-oxo-9H-imidazo[1,5-a]pyrrolo[2,1-c] [1,4]-benzodiazepin-1-carboxylat (Verbindung B);

Äthyl-5,6-dihydro-5-methyl-6-oxo-4H-imidazo[1,5-a][1,4]benzodiazepin-3-carboxylat (Verbindung C)

und

Äthyl-5,6-dihydro-5-methyl-6-thioxo-4H-imidazo[1,5-a][1,4]benzodiazepin-3-carboxylat (Verbindung D).

Wie aus den folgenden Zusammenstellungen über die Versuchsergebnisse am Versuchstier und im in vitro Versuch hervorgeht, wurde die schistosomiazide Wirkung von (+)-5-(o-Chlorphenyl)-1,3-dihydro-3-methyl-7-nitro-2H-1,4-benzodiazepin-2-on (Verbindung S) von keiner der getesteten Verbindungen der allgemeinen Formel I nachteilig beeinflußt.

Versuchsergebnisse an Maus und Hamster:

| Wirts-spezies | Dosierung in mg/kg p.o. | | Dosierungs verhältnis | schistoso-miazide Wirkung in % |
|---|---|---|---|---|
| | Verbindung S | Verbindung A | | |
| Maus | 75 | — | — | 99 |
| | — | 750 | — | 0 |
| | 75 | 225 | 1 : 3 | 98 |
| | 75 | 750 | 1 : 10 | 93 |
| | — | — | — | 0 |
| Hamster | 75 | — | — | 100 |
| | 75 | 225 | 1 : 3 | 100 |
| | — | — | — | 0 |
| | Verbindung S | Verbindung B | | |
| Maus | 75 | 300 | 1 : 4 | 91 |
| Hamster | 75 | 300 | 1 : 4 | 91 |
| | Verbindung S | Verbindung C | | |
| Maus | 75 | 150 | 1 : 2 | 99 |
| Hamster | 75 | 150 | 1 : 2 | 100 |
| | Verbindung S | Verbindung D | | |
| Maus | 75 | 150 | 1 : 2 | 93 |
| | 75 | 750 | 1 : 10 | 98 |
| Hamster | 75 | 150 | 1 : 2 | 100 |

Ergebnisse der in vitro Experimente:

| Präparatkonzentration in µg/ml Verbindung S | Verbindung A | Konzentrations-Verhältnis | Wirkung*) |
|---|---|---|---|
| 25 | — | — | a |
| — | 75 | — | b |
| — | 750 | — | b |
| 25 | 75 | 1 : 3 | a |
| 25 | 750 | 1 : 10 | a |
| — | — | — | b |
| Verbindung S | Verbindung B | | |
| — | 100 | — | b |
| 25 | 100 | 1 : 4 | a |
| Verbindung S | Verbindung C | | |
| — | 100 | — | b |
| 25 | 50 | 1 : 2 | a |
| Verbindung S | Verbindung D | | |
| — | 50 | — | b |
| — | 250 | — | b |
| 25 | 50 | 1 : 2 | a |
| 25 | 250 | 1 : 10 | a |

Wirkung:
a = Wurmpaare innert 15 Minuten bewegungslos;
b = Wurmpaare während der Versuchsdauer von 120 Stunden normal beweglich.

Die Verbindungen der allgemeinen Formel I und ihre pharmazeutisch annehmbaren Säureadditionssalze können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von Tabletten, Lacktabletten, Dragées und Hartgelatinekapseln können die Verfahrensprodukte mit pharmazeutisch inerten, anorganischen oder organischen Trägern verarbeitet werden. Als solche Träger kann man für Tabletten, Dragées und Hartgelatinekapseln z. B. Lactose, Maisstärke oder Derivate davon, Talk, Stearinsäure oder deren Salze etc. verwenden.

Für Weichgelatinekapseln eignen sich als Träger z. B. pflanzliche Öle, Wachse, Fette, halbfeste und flüssige Polyole etc.

Zur Herstellung von Lösungen und Sirupen eignen sich als Träger z. B. Wasser, Polyole, Saccharose, Invertzucker, Glukose und dergleichen.

Für Injektionslösungen eignen sich als Träger z. B. Wasser, Alkohole, Polyole, Glyzerin, pflanzliche Öle etc.

Für Suppositorien eignen sich als Träger z. B. natürliche oder gehärtete Öle, Wachse, Fette, halbflüssige oder flüssige Polyole und dergleichen.

Die pharmazeutischen Präparate können daneben noch Konservierungsmittel, Lösevermittler, Stabilisierungsmittel, Netzmittel, Emulgatoren, Süßmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Man kann Verbindungen der allgemeinen Formel I und pharmazeutisch annehmbare Säureadditionssalze davon bei der Bekämpfung bzw. Verhütung von Krankheiten verwenden, insbesondere bei der Antagonisierung der zentral dämpfenden, muskelrelaxierenden, ataktischen, blutdrucksenkenden und atemdepressiven Eigenschaften von tranquilisierend wirksamen 1,4-Benzodiazepinen. Insbesondere kann man Verbindungen der allgemeinen Formel I in Kombination mit den weiter oben erwähnten schistosomiazid wirksamen Verbindungen der allgemeinen Formel XXII und/oder XXIII bei der Bekämpfung der Schistosomiasis verwenden. Als Verbindung der Formel I verwendet man dabei vorzugsweise Äthyl-8-fluor-5,6-dihydro-5-methyl-6-oxo-4H-imidazo[1,5-a][1,4]benzodiazepin-3-carboxylat und als schistosomiazid wirksame Verbindung vorzugsweise (+)-5-(o-Chlorphenyl)-1,3-dihydro-3-methyl-7-nitro-2H-1,4-benzodiazepin-2-on. Dabei können die Verbindungen der Formel I oder deren pharmazeutisch annehmbare Säureadditionssalze vor, gleichzeitig mit oder nach der Verabreichung bzw. Einnahme von tranquilisierend wirksamen 1,4-Benzodiazepinen verabreicht werden. Wird die Verbindung der Formel I oder ein pharmazeutisch annehmbares Säureadditionssalz davon gleichzeitig mit dem tranquilisierend wirksamen 1,4-Benzodiazepin verabreicht, so kann dies durch Verabreichung als ad-hoc-Kombination oder in Form einer pharmazeutischen Kombination erfolgen, welche eine Verbindung der allgemeinen Formel I oder ein pharmazeutisch annehmbares Säureadditionssalz davon und ein tranquilisierend wirksamens 1,4-Benzodiazepin-Derivat enthält; derartige pharmazeutische Kombinationen sind ebenfalls Gegenstand der vorliegenden Erfindung. Die Dosierung der Verbindungen der Formel I und ihrer pharmazeutisch annehmbaren Säureadditionssalze kann innerhalb weiter Grenzen variieren und ist natürlich in jedem einzelnen Fall den individuellen Gegebenheiten anzupassen. Im allgemeinen dürfte eine Tagesdosis von etwa 2 bis etwa 500 mg angemessen sein.

Wie eingangs erwähnt, sind Arzneimittel, enthaltend eine Verbindung der allgemeinen Formel I oder ein pharmazeutisch annehmbares Säureadditionssalz davon, ebenfalls Gegenstand der vorliegenden Erfindung. Solche Arzneimittel können dadurch hergestellt werden, daß man eine oder mehrere Verbindungen der allgemeinen Formel I oder pharmazeutisch annehmbare Säureadditionssalze davon und gegebenenfalls einen oder mehrere andere therapeutisch wertvolle Stoffe in eine galenische Darreichungsform bringt; in diesem Zusammenhang sei nochmals auf die weiter oben erwähnten pharmazeutischen Kombinationen hingewiesen, welche ebenfalls Gegenstand der vorliegenden Erfindung sind. Insbesondere bilden pharmazeutische Kombinationen, enthaltend eine Verbindung der allgemeinen Formeln I und eine Verbindung der allgemeinen Formel XXII und/oder XXIII, vorzugsweise Kombinationen, enthaltend Äthyl-8-fluor-5,6-dihydro-5-methyl-6-oxo-4H-imidazo[1,5-a][1,4]benzodiazepin-3-carboxylat und (+)-5-(o-Chlorphenyl)-1,3-dihydro-3-methyl-7-nitro-2H-1,4-benzodiazepin-2-on, Gegenstand der vorliegenden Erfindung. Derartige Kombinationen eignen sich zur Bekämpfung von Schistosomiasis.

In den nachfolgenden Beispielen, welche die vorliegende Erfindung näher erläutern, ihren Umfang jedoch in keiner Weise beschränken sollen, sind sämtliche Temperaturen in Celsiusgraden angegeben.

## Beispiel 1

a) Man löst 24 g (132,5 mMol) 5-Fluorisatosäureanhydrid in 140 ml Dimethylsulfoxid und versetzt mit 11,8 g (132,5 mMol) Sarcosin. Die Lösung wird bis zum Ende der Gas-Abspaltung bei 100° gerührt (Dauer: ca. 1,5 Stunden) und anschließend auf ca. 1,2 l Wasser gegossen. Nach 10minütigem Rühren kristallisiert ein Festkörper aus. Die Kristalle werden abgenutscht, mit 1 l Wasser gewaschen und getrocknet. Man erhält 7-Fluor-3,4-dihydro-4-methyl-2H-1,4-benzodiazepin-2,5(1H)-dion vom Schmelzpunkt 262—263° C.

b) Unter einer Argonatmosphäre versetzt man eine Lösung von 6,5 g (32 mMol) 7-Fluor-3,4-dihydro-4-methyl-2H-1,4-benzodiazepin-2,5(1H)-dion in 30 ml trockenem Dimethylformamid mit 4,3 g (38 mMol) Kalium-t-butylat. Die Temperatur steigt dabei auf 35°. Nach 10 Minuten kühlt man auf —30° und tropft, bei —30° bis —20°, 5,8 g (34 mMol) Diäthylchlorphosphat zu. Die Lösung wird anschließend 10 Minuten bei —20° gerührt.

Separat werden 4 g (35 mMol) Kalium-t-butylat in 10 ml Dimethylformamid gelöst und bei ca. —40° mit 4 g (35 mMol) Isocyanessigsäureäthylester versetzt. Diese Lösung wird bei —10° bis —20° zum obigen Reaktionsgemisch getropft. Man rührt dann während 1 Stunde ohne Kühlung, gibt 3,2 ml Eisessig zu, gießt auf ca. 400 ml Wasser und extrahiert dreimal mit je 150 ml Essigester. Die vereinigten organischen Auszüge werden fünfmal mit je 200 ml Wasser gewaschen, über Magnesiumsulfat getrocknet und eingedampft. Aus dem öligen Rückstand erhält man durch Säulen-Chromatographie an Kieselgel und anschließendes Umkristallisieren aus Essigester und Äther Äthyl-8-fluor-5,6-dihydro-5-methyl-6-oxo-4H-imidazo[1,5-a][1,4]benzodiazepin-3-carboxylat vom Schmelzpunkt 199—200°.

Beispiel 2

a) Eine Mischung aus 20,82 g (100 mMol) 7-Fluor-3,4-dihydro-4-methyl-2H-1H-1,4-benzodiazepin-2,5(1H)-dion und 200 ml Chloroform wird mit 121,18 g (1 Mol) Dimethylanilin und 23 g (150 mMol) Phosphoroxychlorid während 2,5 Stunden unter Rückfluß zum Sieden erhitzt. Die Lösung wird auf ein Gemisch an 71 g Natriumbicarbonat und 500 ml Wasser gegossen und 1/2 Stunde gerührt. Man extrahiert zweimal mit Chloroform, wäscht die Chloroform-Phasen mit Wasser und trocknet über Magnesiumsulfat. Nach Entfernung des Lösungsmittels erhält man 162,2 g einer gelben Lösung des entsprechenden Iminchlorids in Dimethylanilin.

Separat werden 8,41 g (75 mMol) Kalium-t-butylat in 30 ml Dimethylformamid gelöst und bei ca. 40° mit 8,48 g (75 mMol) Isocyanessigsäureäthylester versetzt. Zu dieser Lösung gibt man tropfenweise bei —5° bis 0°, 81,1 g der obigen Iminchlorid-Lösung in Dimethylanilin hinzu und rührt das erhaltene Gemisch während 10 Minuten ohne Kühlung bevor man es mit 7,5 ml Essigsäure neutralisiert. Man gießt auf Wasser und extrahiert dreimal mit Chloroform. Die Chloroform-Auszüge wäscht man fünfmal mit Wasser, trocknet über Magnesiumsulfat und dampft ein. Durch Umkristallisieren des Rohproduktes aus Alkohol erhält man Äthyl-8-fluor-5,6-dihydro-5-methyl-6-oxo-4H-imidazo[1,5-a][1,4]benzodiazepin-3-carboxylat vom Schmelzpunkt 200—203°.

b) Die restlichen 81,1 g der unter a) erhaltenen Iminchlorid-Lösung in Dimethylanilin werden bei 0° einer vorgekühlten Lösung von 7,59 g (75 mMol) Tiräthylamin und 8,48 g (75 mMol) Isocyanessigsäureäthylester in 30 ml Dimethylformamid zugetropft. Nach Rühren über Nacht bei Raumtemperatur wird die hellbraune Suspension mit Wasser verdünnt und dreimal mit Chloroform ausgeschüttelt. Die vereinigten Chloroform-Auszüge wäscht man fünfmal mit Wasser, trocknet über Magnesiumsulfat und dampft ein. Der Rückstand wird aus Alkohol umkristallisiert und ergibt Äthyl-8-fluor-5,6-dihydro-5-methyl-6-oxo-4H-imidazo[1,5-a][1,4]benzodiazepin-3-carboxylat vom Schmelzpunkt 201—203°.

Beispiel 3

Eine Mischung aus 3,5 g (11,5 mMol) Äthyl-8-fluor-5,6-dihydro-5-methyl-6-oxo-4H-imidazo[1,5-a][1,4]benzodiazepin-3-carboxylat, 0,15 g Kaliumcyanid und 40 ml Methanol wird während 2,5 Stunden bei Siedetemperatur gerührt. Das Lösungsmittel wird am Rotationsverdampfer abdestilliert und der Rückstand in Chloroform aufgenommen. Nach Abfiltrieren von unlöslichem Material wird das Filtrat eingeengt. Durch Umkristallisieren des Rückstandes aus Essigester erhält man Methyl-8-fluor-5,6-dihydro-5-methyl-6-oxo-4H-imidazo[1,5-a][1,4]benzodiazepin-3-carboxylat vom Schmelzpunkt 195—196,5°.

Beispiel 4

100 ml 2-Propanol werden mit 100 mg Natriumhydrid versetzt. Dazu gibt man 5 g Äthyl-8-fluor-5,6-dihydro-5-methyl-6-oxo-4H-imidazo[1,5-a][1,4]benzodiazepin-3-carboxylat und erhitzt während 1 Stunde unter Rückfluß zum Sieden. Anschließend läßt man auf Raumtemperatur abkühlen, nutscht die ausgeschiedenen Kristalle ab, wäscht mit 2-Propanol und Wasser und erhält Isopropyl-8-fluor-5,6-dihydro-5-methyl-6-oxo-4H-imidazo[1,5-a][1,4]benzodiazepin-3-carboxylat vom Schmelzpunkt 243—244°.

Beispiel 5

Eine Misschung aus 6,08 g (20 mMol) Äthyl-8-fluor-5,6-dihydro-5-methyl-6-oxo-4H-imidazo[1,5-a][1,4]benzodiazepin-3-carboxylat, 100 mg Kaliumcyanid und 60 ml Äthylenglykol wird während 6 Stunden bei 130° gerührt und anschließend eingedampft. Nach Aufnehmen des Rückstandes in Chloroform wird mit Wasser gewaschen, über Magnesiumsulfat getrocknet und eingedampft. Durch Umkristallisieren des Rohproduktes aus Essigester erhält man 2-Hydroxyäthyl-8-fluor-5,6-dihydro-5-methyl-6-oxo-4H-imidazo[1,5-a][1,4]benzodiazepin-3-carboxylat vom Schmelzpunkt 202—204°.

Beispiel 6

10,7 g (35,3 mMol) Äthyl-8-fluor-5,6-dihydro-5-methyl-6-oxo-4H-imidazo[1,5-a][1,4]benzodiazepin-3-carboxylat und 7,13 g (17,6 mMol) 2,4-Bis(p-methoxyphenyl)-1,3,2,4-dithiadiphosphetan-2,4-disulfid in 100 ml Toluol werden während 22 Stunden unter Rückfluß zum Sieden erhitzt. Das Reaktionsgemisch wird anschließend bis zur beginnenden Kristallisation im Vakuum eingeengt und 1 Stunde im Eisbad stehen gelassen. Das auskristallisierte Material wird abgenutzt und mit wenig Toluol gewaschen. Das Gemisch wird an Kieselgel mit Essigester/Chloroform (1 : 9) aufgetrennt. Durch anschließendes Umkristallisieren aus Essigester erhält man reines Äthyl-8-fluor-5,6-dihydro-5-methyl-6-thioxo-4H-imidazo[1,5-a][1,4]benzodiazepin-3-carboxylat vom Schmelzpunkt 157—159°.

## Beispiel 7

a) 15,0 g (49,5 mMol) Äthyl-8-fluor-5,6-dihydro-5-methyl-6-oxo-4H-imidazo[1,5-a][1,4]benzodiazepin-3-carboxylat werden unter Argon in 4,50 ml siedendem absolutem Tetrahydrofuran (über Alox filtriert) gelöst und bei 50° einer Lösung von 1,1 g (49,5 mMol) Lithiumborhydrid in 50 ml absolutem Tetrahydrofuran zugetropft. Es wird 4 Stunden bei 40° und 2$^1/_2$ Stunden bei Siedetemperatur gerührt. Anschließend zersetzt man bei 40° mit 20 ml Wasser und 20 ml konz. Salzsäure/$H_2O$ (1 : 1) und rührt über Nacht bei Raumtemperatur. Nach Entfernen des Tetrahydrofurans im Vakuum wird der Rückstand mit 14 ml konz. Ammoniak versetzt. Man läßt in der Kälte kristallisieren, nutscht das ausgefallene Material ab und wäscht mit Wasser. Durch Umkristallisieren aus Äthanol erhält man 8-Fluor-4,5-dihydro-3-(hydroxymethyl)-5-methyl-6H-imidazo[1,5-a][1,4]benzodiazepin-6-on vom Schmelzpunkt 221—223°.

b) Eine Suspension von 4,93 g (18,9 mMol) 8-Fluor-4,5-dihydro-3-(hydroxymethyl)-5-methyl-6H-imidazo[1,5-a][1,4]benzodiazepin-6-on in 220 ml Methylenchlorid wird mit 30 g Mangandioxid versetzt und während 1$^1/_2$ Stunden bei Raumtemperatur gerührt. Nach Filtrieren über Dicalit wird das Filtrat im Vakuum eingedampft und der Rückstand aus Äthanol umkristallisiert. Es resultiert dabei 8-Fluor-5,6-dihydro-5-methyl-6-oxo-4H-imidazo[1,5-a][1,4]benzodiazepin-3-carboxaldehyd vom Schmelzpunkt 224—226°.

c) Zu einem Gemisch aus 3,0 g (11,6 mMol) 8-Fluor-5,6-dihydro-5-methyl-6-oxo-4H-imidazo[1,5-a][1,4]benzodiazepin-3-carboxaldehyd, 0,99 g (14,5 mMol) N-Hydroxylamin-hydrochlorid und 60 ml Wasser gibt man tropfenweise eine Lösung von 4,14 g (14,5 mMol) Natriumcarbonat. 10 $H_2O$ in 20 ml Wasser, rührt das Reaktionsgemisch während 1 Stunde bei 70°, kühlt im Eisbad ab, nutscht das ausgefallene Material ab und wäscht es mit Wasser. Der noch feuchte Stoff wird aus Äthanol umkristallisiert und liefert 8-Fluor-5,6-dihydro-5-methyl-6-oxo-4H-imidazo[1,5-a][1,4]benzodiazepin-3-carboxaldehyd-3-oxim vom Schmelzpunkt 247—250°.

d) 1,65 g (6 mMol) 8-Fluor-5,6-dihydro-5-methyl-6-oxo-4H-imidazo[1,5-a][1,4]benzodiazepin-3-carboxaldehyd-3-oxim werden in 15 ml Essigsäureanhydrid während 4$^1/_2$ Stunden unter Rückfluß zum Sieden erhitzt und eingedampft, der Rückstand wird in 100 ml Chloroform aufgenommen, einmal mit gesättigter Natriumhydrogencarbonat-Lösung und einmal mit Wasser gewaschen. Die Chloroform-Lösung wird über Magnesiumsulfat getrocknet und eingeengt. Nach Säulenchromatographie und Umkristallisieren aus Essigester resultieren 8-Fluor-5,6-dihydro-5-methyl-6-oxo-4H-imidazo[1,5-a][1,4]benzodiazepin-3-carbonitril vom Schmelzpunkt 236—237°.

## Beispiel 8

a) Eine Lösung von 23,7 g 5-Fluorisatosäureanhydrid und 29,5 g N-(2,4-Dimethoxybenzyl)-glycin in 130 ml Dimethylsulfoxid wird während 1$^1/_2$ Stunden auf 100° erhitzt. Man kühlt auf Raumtemperatur ab und gießt das Reaktionsgemisch auf 400 ml Wasser. Das ausgefallene Produkt wird abgenutscht und mit Wasser gewaschen. Man erhält dabei 4-(2,4-Dimethoxybenzyl)-7-fluor-3,4-dihydro-2H-1,4-benzodiazepin-2,5(1H)-dion vom Schmelzpunkt 190—192°.

b) 27 g (78 mMol) 4-(2,4-Dimethoxybenzyl)-7-fluor-3,4-dihydro-2H-1,4-benzodiazepin-2,5(1H)-dion werden in 75 ml trockenem Dimethylformamid vorgelegt und mit 9,6 g (86 mMol) Kalilum-t-butylat versetzt. Die Temperatur steigt auf 40° und die Lösung wird während 10 Minuten bei Raumtemperatur gerührt, bevor man sie auf —30° abkühlt. Bei dieser Temperatur gibt man tropfenweise14,2 g (82 mMol) Diäthylchlorphosphat hinzu und rührt 10 Minuten bei —20°.

Inzwischen löst man 9,7 g (86 mMol) Kalium-t-butylat in 20 ml trockenem Dimethylformamid, kühlt diese Lösung auf —50° ab und versetzt sie mit 9,8 g (86 mMol) Isocyanessigester. Diese Lösung wird sofort bei —20° bis —10° zum obigen Reaktionsgemisch zugetropft. Man rührt während 1 Stunde ohne Kühlung, gibt 7,8 ml Eisessig zu, gießt auf 1 l Wasser und extrahiert dreimal mit je 200 ml Essigester. Die vereinigten organischen Auszüge werden fünfmal mit je 250 ml Wasser gewaschen, über Magnesiumsulfat getrocknet und eingedampft. Durch Umkristallisation aus 150 ml Essigester erhält man Äthyl-5-(2,4-dimethoxybenzyl)-8-fluor-5,6-dihydro-5-methyl-6-oxo-4H-imidazo[1,5-a][1,4]benzodiazepin-3-carboxylat vom Schmelzpunkt 159—160°.

c) 13,0 g (29,6 mMol) Äthyl-5-(2,4-dimethoxybenzyl)-8-fluor-5,6-dihydro-5-methyl-6-oxo-4H-imidazo[1,5-a][1,4]benzodiazepin-3-carboxylat werden in 45 ml Trifluoressigsäure 4 Stunden unter Rückfluß zum Sieden erhitzt. Nach Eindampfen der dunkelroten Suspension im Vakuum wird der Rückstand mit Wasser versetzt und mit ca. 100 ml 15%iger Kaliumcarbonat-Lösung alkalisch gestellt. Das ausgefallene Material wird abgenutscht und mit Wasser gewaschen. Durch Umkristallisieren aus ca. 500 ml Äthanol erhält man Äthyl-8-fluor-5,6-dihydro-5-methyl-6-oxo-4H-imidazo[1,5-a][1,4]benzodiazepin-3-carboxylat vom Schmelzpunkt 298°.

Beispiel 9

0,1 g (2 mMol) Natriumhydrid (55%ige Öldispersion) werden in 10 ml trockenem Dimethylformamid suspendiert und mit 0,5 g (1,7 mMol) Äthyl-8-fluor-5,6-dihydro-5-methyl-6-oxo-4H-imidazo[1,5-a]-[1,4]benzodiazepin-3-carboxylat versetzt. Nach beendeter Gasentwicklung gibt man 0,13 ml (2 mMol) Methyljodid hinzu und rührt während 2 Stunden bei Raumtemperatur. Das Reaktionsgemisch wird auf ca. 60 ml Wasser gegossen und dreimal mit je 30 ml Chloroform extrahiert. Die vereinigten Chloroform-Auszüge werden mit ca. 30 ml Wasser gewaschen, über Magnesiumsulfat getrocknet und eingedampft. Durch Umkristallisieren aus Essigester erhält man Äthyl-8-fluor-5,6-dihydro-5-methyl-6-oxo-4H-imidazo[1,5-a][1,4]benzodiazepin-3-carboxylat vom Schmelzpunkt 196—197°.

Beispiel 10

a) Eine Mischung aus 34,5 g (0,22 Mol) 6-Fluor-2-nitrotoluol, 30,7 g (0,22 Mol) Kaliumcarbonat, 105,4 g (0,66 Mol) Kaliumpermanganat und 3,3 l Wasser wird bis zur Entfärbung des Permanganats auf 100° erhitzt (ca. $2^1/_2$ Stunden). Nach Abkühlen und Entfernen des nicht umgesetzten 6-Fluor-2-nitrotoluols durch Extraktion mit Essigester wird die wäßrige Phase mit Salzsäure auf pH 1 gestellt und dreimal mit Essigester ausgeschüttelt. Die vereinigten organischen Auszüge werden über Magnesiumsulfat getrocknet und eingeengt, wobei 6-Fluor-2-nitrobenzoesäure erhalten wird. Nach Umkristallisieren aus Essigester/n-Hexan schmilzt das Produkt bei 146—148°.

b) Eine Lösung von 20,0 g (0,108 Mol) 6-Fluor-nitrobenzoesäure in einem Gemisch von 200 ml Methanol und 27 ml konz. Salzsäure wird nach Zugabe von 2,7 g 10%iger Palladiumkohle bei 35—40° unter leichtem Überdruck hydriert. Nach Abfiltrieren des Katalysators und Einengen des Filtrates wird das Rohprodukt aus Methanol/Äther umkristallisiert. Man erhält 6-Fluor-anthranilsäure-hydrochlorid vom Schmelzpunkt 176—178° (Zersetzung).

c) In eine Lösung von 23 g (0,148 Mol) 6-Fluor-anthranilsäure-hydrochlorid in einem Gemisch aus 300 ml Tetrahydrofuran und 150 ml 4N Salzsäure leitet man während 3 Stunden 35—40° Phosgen ein. Nach Entfernen des Phosgens wird mit 500 ml Wasser verdünnt und vom Niederschlag abfiltriert. Man erhält rohes 6-Fluor-isatosäureanhydrid vom Schmelzpunkt 265—267° (Zersetzung).

d) 7,2 g (0,04 Mol) 6-Fluorisatosäureanhydrid und 3,9 g (0,044 Mol) Sarcosin werden zu 10 ml Dimethylsulfoxid gegeben und 30 Minuten auf 100° erhitzt. Nach Abkühlen und Verdünnen mit 15 ml Wasser wird der ausgefallene Stoff abfiltriert. Man erhält nach dem Trocknen 6-Fluor-3,4-dihydro-4-methyl-2H-1,4-benzodiazepin-2,5(1H)-dion vom Schmelzpunkt 214—217° (Zersetzung).

e) Unter einer Argonatmosphäre werden 3,18 g (15,2 mMol) 6-Fluor-3,4-dihydro-4-methyl-2H-1,4-benzodiazepin-2,5(1H)-dion in 20 ml Dimethylformamid mit 0,59 g (15,2 mMol) Natriumhydrid (60%iges Öl) Dispersion versetzt und während 1 Stunde gerührt. Die erhaltene Lösung wird auf —30° gekühlt und tropfenweise bei dieser Temperatur mit 2,63 g (15,2 mMol) Diäthylchlorphosphat versetzt. Man rührt anschließend 10 Minuten bei —20°.

Inzwischen werden separat 1,79 g (16 mMol) Kalium-t-butylat in 3 ml Dimethylformamid gelöst und bei —40 bis —50° mit 1,81 g Isocyanessigsäureäthylester (16 mMol) versetzt. Die erhaltene orangefarbene Lösung wird bei —15° dem obigen Reaktionsgemisch zugetropft. Man rührt 10 Minuten ohne Kühlung, neutralisiert mit 1,50 ml Essigsäure und gießt die dunkelbraune Lösung auf Wasser. Nach dreimaligem Extrahieren mit je 60 ml Chloroform werden die vereinigten Chloroform-Phasen fünfmal mit je 150 ml Wasser gewaschen und eingedampft. Durch Umkristallisieren des Rohproduktes aus Essigester erhält man Äthyl-7-fluor-5,6-dihydro-5-methyl-6-oxo-4H-imidazo[1,5-a][1,4]benzodiazepin-3-carboxylat vom Schmelzpunkt 196—198,5°.

Beispiel 11

a) 7,2 g (0,04 Mol) 6-Fluorisatosäureanhydrid und 5 g (0,044 Mol) (S)-3,4-Dehydroprolin werden zu 10 ml Dimethylsulfoxid gegeben und 45 Minuten auf 100° erhitzt. Nach Abkühlen und Verdünnen mit 15 ml Wasser werden die ausgefallenen Kristalle abfiltriert. Nach Trocknen resultiert (S)-6-Fluor-3,11a-dihydro-5H-pyrrolo[2,1-c][1,4]benzodiazepin-5,11(10H)-dion vom Schmelzpunkt 238—240°.

b) Eine Mischung aus 4,64 g (S)-6-Fluor-3,11a-dihydro-5H-pyrrolo[2,1-c][1,4]benzodiazepin-5,11-(10H)-dion (20 mMol) und 25 ml Dimethylformamid wird unter Argon mit 0,80 g (22 mMol) Natriumhydrid (60%ige Öldispersion) versetzt und 1 Stunde zwischen 0 und 10° gerührt. Die erhaltene Lösung wird auf —30° abgekühlt und mit 3,79 g (22 mMol) Diäthylchlorphosphat versetzt und 10 Minuten bei —20° gerührt.

Inzwischen wird eine Lösung von 2,24 g Kalium-t-butylat (22 mMol) in 5 ml Dimethylformamid in einem Aceton-Trockeneis-Bad mit 2,26 g Isocyanessigsäureäthylester (20 mMol) versetzt. Die erhaltene Lösung wird bei —20 bis —10° dem obigen Reaktionsgemisch tropfenweise zugegeben. Man rührt während 10 Minuten ohne Kühlung, neutralisiert mit 2 ml Essigsäure und gießt auf Wasser. Man extrahiert dreimal mit je 50 ml Chloroform, wäscht die vereinigten organischen Phasen fünfmal mit je

200 ml Wasser, trocknet über Magnesiumsulfat und entfernt das Lösungsmittel. Durch Säulenchromatographie des Rohproduktes und anschließendes Umkristallisieren aus Essigester erhält man Äthyl-8-fluor-11,13a-dihydro-9-oxo-9H-imidazo[1,5-a]pyrrolo[2,1-c][1,4]benzodiazepin-1-carboxylat vom Schmelzpunkt 200—201°.

Beispiel 12

Unter einer Argonatmosphäre werden 2,86 g (12,2 Mol) (S)-(+)-7-Fluor-1,2,3,11a-tetrahydro-5H-pyrrolo[2,1-c][1,4]benzodiazepin-5,11(10H)-dion in 15 ml trockenem Dimethylformamid vorgelegt und mit 1,5 g (13,1 mMol) Kalium-t-butylat versetzt. Die Lösung wird 10 Minuten gerührt und auf —30° abgekühlt. Bei dieser Temperatur tropft man 2,18 g (12,6 mMol) Diäthylchlorphosphat zu und rührt 10 Minuten bei —20°.

Separat löst man 1,5 g (13,2 mMol) Kalium-t-butylat in 5 ml Dimethylformamid, kühlt diese Lösung auf —50° und versetzt sie mit 1,5 g (13,3 mMol) Isocyanessigsäureäthylester. Diese Lösung wird sofort bei —20 bis —10° zum obigen Reaktionsgemisch getropft. Man rührt anschließend während 1 Stunde ohne Kühlung, neutralisiert mit 1,2 ml Eisessig, gießt auf 300 ml Wasser und extrahiert dreimal mit je 100 ml Essigester. Die vereinigten organischen Auszüge werden fünfmal mit je 200 ml Wasser gewaschen, über Magnesiumsulfat getrocknet und eingedampft. Der halbkristalline Rückstand wird aus 30 ml Essigester umkristallisiert, dabei resultiert Äthyl-(S)-7-fluor-11,12,13,13a-tetrahydro-9-oxo-9H-imidazo[1,5-a]pyrrolo[2,1-c][1,4]benzodiazepine-1-carboxylat vom Schmelzpunkt 194—195°.

Beispiel 13

a) Man rührt 29,1 g (0,14 Mol) 6-Chlor-isatosäureanhydrid mit 13,12 g (0,14 Mol) Sarcosin in 150 ml Dimethylsulfoxid während 1 Stunde bei 110°. Die erhaltene Lösung wird eingeengt und der Rückstand aus Alkohol umkristallisiert. Man erhält 6-Chlor-3,4-dihydro-4-methyl-2H-1,4-benzodiazepin-2,5(1H)-dion vom Schmelzpunkt 237—238°.

b) Eine Lösung von 10 g (44,5 mMol) 6-Chlor-3,4-dihydro-4-methyl-2H-1,4-benzodiazepin-2,5(1H)-dion in 100 ml Dimethylformamid wird unter einer Argonatmosphäre mit 5,50 g (49 mMol) Kalium-t-butylat versetzt und 20 Minuten gerührt. Man kühlt die erhaltene Lösung auf —30° ab und tropft bei dieser Temperatur 3,45 g (49 mMol) Diäthylchlorphosphat zu. Das Gemisch wird anschließend 10 Minuten bei —20° gerührt.

Inzwischen kühlt man eine Lösung von 5,50 g (40 mMol) Kalium-t-butylat in 10 ml Dimethylformamid in einem Aceton/Trockeneis-Bad und versetzt mit 5,54 g (49 mMol) Isocyanessigsäureäthylester. Die dunkelrote Lösung wird bei —10 bis —20° dem obigen Reaktionsgemisch zugegeben und dieses eine halbe Stunde ohne Kühlung gerührt bevor man es mit 5 ml Eisessig neutralisiert und auf ca. 300 ml Wasser gießt. Die orange Lösung wird dreimal mit Chloroform extrahiert. Die organische Phase wird fünfmal mit Wasser gewaschen, über Magnesiumsulfat getrocknet und eingedampft. Durch Chromatographieren und Umkristallisieren aus Essigester erhält man Äthyl-7-chlor-5,6-dihydro-5-methyl-6-oxo-4H-imidazo[1,5-a][1,4]benzodiazepin-3-carboxylat vom Schmelzpunkt 229—230°.

Beispiel 14

a) Man rührt 10 g (50,6 mMol) 6-Chlor-isatosäureanhydrid mit 5,82 g (50,6 mMol) L-Prolin in 80 ml Dimethylsulfoxid während 2 Stunden bei 110°. Die Lösung wird eingedampft und der Rückstand aus Essigester auskristallisiert. Man erhält (S)-6-Chlor-1,2,3,11a-tetrahydro-5H-pyrrolo[2,1-c][1,4]benzodiazepin-5,11(10H)-dion vom Schmelzpunkt 264—266°.

b) Eine Lösung von 4,0 g (16 mMol) (S)-6-Chlor-1,2,3,11a-tetrahydro-5H-pyrrolo[2,1-c][1,4]benzodiazepin-5,11(10H)-dion in 30 ml Dimethylformamid versetzt man unter Argonatmosphäre mit 0,57 g (16 Mol) Natriumhydrid (60%ige Öldispersion) und rührt 1 Stunde bei Raumtemperatur. Zur so erhaltenen Suspension gibt man tropfenweise bei —20° 3,02 g (17,5 mMol) Diäthylchlorphosphat und rührt während 10 Minuten bei dieser Temperatur.

Inzwischen kühlt man eine Lösung von 1,96 g (17,5 mMol) Kalium-t-butylat in 5 ml Dimethylformamid in einem Aceton/Trockeneis-Bad ab und versetzt mit 1,92 g (17 mMol) Isocyanessigsäureäthylester. Diese Lösung wird bei —10 bis —20° dem obigen Reaktionsgemisch langsam zugegeben. Man rührt eine halbe Stunde ohne Kühlung, neutralisiert mit 1,6 ml Essigsäure und gießt auf ca. 200 ml Wasser. Die orange-farbene Lösung wird dreimal mit Chloroform extrahiert. Die organische Phase wird fünfmal mit Wasser gewaschen, über Magnesiumsulfat getrocknet und eingedampft. Durch Chromatographieren und anschließendes Umkristallisieren aus Essigester erhält man Äthyl-(S)-8-chlor-11,12,13,13a-tetrahydro-9-oxo-9H-imidazo[1,5-a]pyrrolo[2,1-c][1,4]benzodiazepin-1-carboxylat vom Schmelzpunkt 182,5—184°.

## Beispiel 15

a) Das als Ausgangsstoff verwendete N-(2,4-Dimethoxybenzyl)-glycin wird hergestellt durch Umsetzen von Glycin mit 2,4-Dimethoxybenzaldehyd in Gegenwart von Natronlauge, Reduzieren mit Palladium auf Kohle in Methanol und anschließendes Neutralisieren mit 2N Salzsäure. Die erhaltene wäßrige Lösung wird eingeengt. 41,9 g dieses Gemisches aus N-(2,4-Dimethoxybenzyl)-glycin und Natriumchlorid werden in 300 ml Dimethylsulfoxid mit 23,18 g (142 mMol) Isatosäureanhydrid während 1,5 Stunden bei 110° gerührt. Man gießt das Reaktionsgemisch auf ca. 2 l Wasser und rührt eine halbe Stunde. Die ausgefallenen Kristalle werden abgenutscht, mit Wasser gewaschen und getrocknet. Durch Umkristallisieren aus Essigester erhält man 3,4-Dihydro-4-(2,4-dimethoxybenzyl)-2H-1,4-benzodiazepin-2,5-(1H)-dion vom Schmelzpunkt 151—152,5°.

b) Eine Lösung von 110 g (0,33 Mol) 3,4-Dihydro-4-(2,4-dimethoxybenzyl)-2H-1,4-benzodiazepin-2,5(1H)-dion in 330 ml trockenem Dimethylformamid wird mit 45,74 g (0,40 Mol) Kalium-t-butylat versetzt. Man kühlt die Lösung auf —30° ab und tropft über einen Zeitraum von 20 Minuten 61 g (0,35 Mol) Diäthylchlorphosphat zwischen —30 und —20° zu und rührt 10 Minuten bei —20°.

Inzwischen werden separat 41,6 g (0,37 Mol) Kalium-t-butylat in 90 ml Dimethylformamid gelöst, auf ca. —50° gekühlt und mit 42 g (0,37 Mol) Isocyanessigsäureäthylester versetzt. Die so erhaltene orange-farbene Lösung wird bei —20 bis —10° dem obigen Reaktionsgemisch zugetropft. Man rührt eine halbe Stunde, neutralisiert mit 33 ml Essigsäure, gießt auf ca. 1,7 l Wasser und extrahiert dreimal mit Chloroform. Die vereinigten Chloroform-Phasen werden fünfmal mit Wasser gewaschen, über Magnesiumsulfat getrocknet und eingedampft. Durch Umkristallisieren aus Essigester erhält man Äthyl-5,6-dihydro-5-(2,4-dimethoxybenzyl)-6-oxo-4H-imidazo[1,5-a][1,4]benzodiazepin-3-carboxylat vom Schmelzpunkt 136—138°.

c) 90 g (214 mMol) Äthyl-5,6-dihydro-5-(2,4-dimethoxybenzyl)-6-oxo-4H-imidazo[1,5-a][1,4]benzodiazepin-3-carboxylat werden in 300 ml Trifluoressigsäure 3 Stunden unter Rühren zum Rückfluß erhitzt. Nach Eindampfen des Ansatzes im Vakuum versetzt man den Rückstand mit Wasser und stellt mit 10%iger Kaliumcarbonatlösung alkalisch. Das ausgefallene Material wird abgenutscht, mit Wasser gewaschen und im Vakuum getrocknet. Durch Kristallisieren aus Chloroform/Hexan erhält man Äthyl-5,6-dihydro-6-oxo-4H-imidazo[1,5-a][1,4]benzodiazepin-3-carboxylat vom Schmelzpunkt 248—250°.

## Beispiel 16

70 mg (1,6 mMol) Natriumhydrid (55%ige Öldispersion) werden in 5 ml trockenem Dimethylformamid suspendiert und mit 135 mg (0,5 mMol) Äthyl-5,6-dihydro-6-oxo-4H-imidazo[1,5-a][1,4]benzodiazepin-3-carboxylat versetzt. Nach beendeter Gasentwicklung gibt man 0,15 ml (2,3 mMol) Methyljodid dazu und rührt während 1 Stunde bei Raumtemperatur. Das Reaktionsgemisch wird auf ca. 50 ml Wasser gegossen, mit Eisessig neutralisiert und dreimal mit je ca. 30 ml Chloroform ausgeschüttelt. Die vereinigten Chloroformphasen werden mit ca. 20 ml Wasser gewaschen, über Magnesiumsulfat getrocknet und eingedampft. Nach Säulenchromatographie und Umkristallisieren aus Essigester resultiert Äthyl-5,6-dihydro-5-methyl-6-oxo-4H-imidazo[1,5-a][1,4]benzodiazepin-3-carboxylat vom Schmelzpunkt 166—167°.

## Beispiel 17

Unter einer Argonatmosphäre werden 19,0 g (0,10 Mol) 3,4-Dihydro-4-methyl-2H-1,4-benzodiazepin-2,5(1H)-dion in 100 ml trockenem Dimethylformamid vorgelegt. Man gibt 15,5 g (0,12 Mol) Kalium-t-butylat zu, wobei die Temperatur von 25° auf 39° steigt. Man kühlt auf Raumtemperatur und tropft zwischen 18—22° 18,2 g (0,105 Mol) Diäthylchlorphosphat zu.

Separat werden 11,2 g (0,10 Mol) Kalium-t-butylat in 30 ml Dimethylformamid gelöst. Diese Lösung wird auf ca. —50° gekühlt und unter Argon mit 11,3 g (0,10 Mol) Isocyanessigsäureäthylester versetzt. Anschließend wird diese Lösung bei 18—23° unter Kühlung zum obigen Reaktionsgemisch getropft. Man rührt während 1 Stunde bei Raumtemperatur, gibt 5 ml Essigsäure zu, gießt dann auf 500 ml Wasser und extrahiert zweimal mit je 200 ml Chloroform. Die vereinigten Chloroform-Auszüge werden dreimal mit je 300 ml Wasser gewaschen, über Magnesiumsulfat getrocknet und eingedampft. Zum öligen Rückstand gibt man 150 ml Essigester und läßt bei 0° kristallisieren. Man nutscht die ausgeschiedenen Kristalle ab, wäscht sie mit kaltem Essigester und erhält Äthyl-5,6-dihydro-5-methyl-6-oxo-4H-imidazo[1,5-a][1,4]benzodiazepin-3-carboxylat vom Schmelzpunkt 163—165°. Nach Umkristallisieren aus 50 ml Essigester zeigt das Produkt einen Schmelzpunkt von 164—165°.

## Beispiel 18

a) Unter einer Argonatmosphäre werden 21,5 g (75,4 mMol) Äthyl-5,6-dihydro-5-methyl-6-oxo-4H-imidazo[1,5-a][1,4]benzodiazepin-3-carboxylat unter Rühren in 250 ml absolutem Tetrahydrofuran

(über Alox basisch I filtriert) heiß gelöst, auf ca. 30° abgekühlt und tropfenweise mit einer Lösung von 1,66 g (75,4 mMol) Lithiumborhydrid in 25 ml absolutem Tetrahydrofuran versetzt. Das Reaktionsgemisch wird 6 Stunden unter Rückfluß zum Sieden erhitzt, auf Raumtemperatur abgekühlt und mit 50 ml 3N wäßriger Salzsäure zersetzt. Man rührt weitere 2 Stunden bei 60° und entfernt das Tetrahydrofuran im Vakuum. Der Rückstand wird mit konz. Ammoniak alkalisch gestellt und 2 Stunden im Eisbad stehen gelassen. Das Rohprodukt wird abgenutscht, mit viel Wasser gewaschen und aus Äthanol umkristallisiert. Man erhält 4,5-Dihydro-3-(hydroxymethyl)-5-methyl-6H-imidazo[1,5-a][1,4]benzodiazepin-6-on vom Schmelzpunkt 224—226°.

b) In einer Argonatmosphäre rührt man ein Gemisch von 12,2 g (50 mMol) 4,5-Dihydro-3-(hydroxymethyl)-5-methyl-6H-imidazo[1,5-a][1,4]benzodiazepin-6-on, 80,0 g Mangan-IV-oxid und 500 ml absolutes Methylenchlorid 1 Stunde bei Raumtemperatur und nutscht anschließend das Mangan-IV-oxid über Dicalit ab unter Nachspülen mit Methylenchlorid. Das Filtrat wird im Vakuum zur Trockene eingedampft.

Nach Erwärmen des Rückstandes in ca. 150 ml Essigester erhält man 5,6-Dihydro-5-methyl-6-oxo-4H-imidazo[1,5-a][1,4]benzodiazepin-3-carboxaldehyd vom Schmelzpunkt 205—207°.

c) Zu einer Suspension von 7,2 g (29,8 mMol) 5,6-Dihydro-5-methyl-6-oxo-4H-imidazo[1,5-a][1,4]benzodiazepin-3-carboxaldehyd und 2,53 g (37,3 mMol) Hydroxylamin-hydrochlorid in 250 ml Wasser gibt man tropfenweise eine Lösung von 10,6 g (37,3 mMol) Natriumcarbonat. 10 $H_2O$ in 40 ml Wasser. Das Reaktionsgemisch wird 6 Stunden bei 70° gerührt und auf Raumtemperatur abgekühlt. Das ausgefallene Rohprodukt wird abgenutscht, mit Wasser gewaschen und noch feucht aus ca. 60 ml Dimethylformamid umkristallisiert. Man erhält dabei 5,6-Dihydro-5-methyl-6-oxo-4H-imidazo[1,5-a][1,4]benzodiazepin-3-carboxaldehyd-3-oxim vom Schmelzpunkt 268—274° (Zersetzung).

d) Eine Lösung von 4,8 g (18,7 mMol) 5,6-Dihydro-5-methyl-6-oxo-4H-imidazo[1,5-a][1,4]benzodiazepin-3-carboxaldehyd-3-oxim in 50 ml Essigsäureanhydrid wird 28 Stunden unter Rückfluß zum Sieden erhitzt. Nach Eindampfen des Reaktionsgemisches im Vakuum wird der Rückstand in 150 ml Chloroform aufgenommen, zweimal mit je 30 ml gesättigter Natriumhydrogencarbonatlösung und mit 30 ml Wasser gewaschen. Die organische Phase wird über Magnesiumsulfat getrocknet und eingedampft. Durch Säulenchromatographie an Kieselgel und anschließendem Umkristallisieren aus Essigester erhält man 5,6-Dihydro-5-methyl-6-oxo-4H-imidazo[1,5-a][1,4]benzodiazepin-3-carbonitril vom Schmelzpunkt 184—186°.

## Beispiel 19

a) In einer Argonatmosphäre werden 1,22 g Magnesium-Späne (50,2 mMol) mit 60 ml absolutem Diäthyläther überschichtet und mit 2—3 Tropfen Äthyljodid versetzt. Nach Anspringen der Grignard-Reaktion wird bei Siedetemperatur eine Lösung von 4,3 ml (51 mMol) Äthyljodid in 10 ml absolutem Diäthyläther zugetropft. Nach vollständigem Auflösen des Magnesiums versetzt man tropfenweise mit einer 30° warmen Lösung von 0,65 g (40 mMol) 5,6-Dihydro-5-methyl-6-oxo-4H-imidazo[1,5-a][1,4]benzodiazepin-3-carboxaldehyd in 200 ml absolutem Tetrahydrofuran. Die gelbe Suspension wird ca. $5^1/_2$ Stunden unter Rückfluß zum Sieden erhitzt, auf Raumtemperatur abgekühlt und auf 600 ml Eiswasser gegossen. Es wird über Dicalit filtriert unter Nachspülen mit Tetrahydrofuran. Nach Einengen des Filtrates im Vakuum wird der Rückstand in 250 ml Chloroform aufgenommen. Die Chloroformphase wird zweimal mit je 60 ml Wasser gewaschen, über Magnesiumsulfat getrocknet und eingeengt. Das Rohprodukt wird an Kieselgel mit Chloroform/Methanol (19:1, V/V) als Elutionsmittel chromatographiert. Nach Umkristallisieren aus ca. 40 ml Essigester erhält man 4,5-Dihydro-3-(1-hydroxypropyl)-5-methyl-6H-imidazo[1,5-a][1,4]benzodiazepin-6-on vom Schmelzpunkt 145—147°.

b) Ein Gemisch von 1,84 g (6,8 mMol) 4,5-Dihydro-3-(1-hydroxypropyl)-5-methyl-6H-imidazo[1,5-a][1,4]benzodiazepin-6-on, 16 g Mangan-IV-oxid und 150 ml Methylenchlorid wird während 2 Stunden unter Argonatmosphäre bei Raumtemperatur gerührt. Das Mangan-IV-oxid wird über ein Glasfilterfilter abgenutscht und das Filtrat im Vakuum eingedampft. Nach Umkristallisieren des Rückstandes aus 100 ml Essigester resultieren 4,5-Dihydro-5-methyl-3-propionyl-6H-imidazo[1,5-a][1,4]benzodiazepin-6-on vom Schmelzpunkt 215—216°.

## Beispiel 20

a) Eine unter Argon gerührte Mischung aus 910 ml Malonsäurediäthylester und 3,0 l Tetrahydrofuran wird auf 0° abgekühlt und portionenweise mit 350 g Natriumhydrid (55%ige Öldispersion) versetzt, so daß die Temperatur 15° nicht übersteigt. Danach rührt man über Nacht bei Raumtemperatur, kühlt das Rekationsgemisch auf 0—5° ab und gibt über einen Zeitraum von 15 Minuten 290 ml Diäthylchlorphosphat tropfenweise hinzu. Nach weiteren 2 Stunden bei Raumtemperatur versetzt man langsam mit einer ca. 45° warmen Suspension von 190 g 4-Methyl-3H-1,4-benzodiazepin-2,5(1H,4H)-dion in 2 l Tetrahydrofuran (Zugabezeit ca. 1 Stunde). Das Reaktionsgemisch wird über Nacht bei Raumtemperatur gerührt, danach auf 5° abgekühlt und mit 350 ml Eisessig tropfenweise versetzt. Dabei entsteht ein

dicker Brei, welcher durch Zugabe von 500 ml Wasser wieder gut rührfähig wird. Das Tetrahydrofuran wird aus dem Reaktionsgemisch abdestilliert und der zum Teil kristalline Rückstand zwischen Äther und Wasser verteilt. Die organische Phase wird mit Wasser gewaschen, getrocknet und eingeengt. Der rotbraune Rückstand wird in 2 l siedendem n-Hexan gelöst und danach abgekühlt. Man läßt über Nacht bei 2° stehen und nutscht das entstandene Kristallisat ab. Die hellgelben Kristalle werden anschließend in 800 ml Toluol heiß gelöst und durch Zugabe von 600 ml n-Hexan und Kratzen zur Kristallisation gebracht. Nach dem Stehen über Nacht in der Kälte werden die Kristalle abgenutscht und im Vakuum bei 50° getrocknet. Man erhält Diäthyl-(1,3,4,5-tetrahydro-4-methyl-5-oxo-2H-1,4-benzodiazepin-2-yliden)malonat als weiße Kristalle vom Schmelzpunkt 139°.

b) Eine Mischung aus 33,2 g (0,1 Mol) Diäthyl-(1,3,4,5-tetrahydro-4-methyl-5-oxo-2H-1,4-benzodiazepin-2-yliden)-malonat, 8,0 g (0,2 Mol) Natriumhydroxid und 400 ml abs. Äthanol wird während 3 Stunden zum Rückfluß erhitzt. Man läßt anschließend bei 0° kristallisieren, nutscht den ausgefallenen Festkörper ab und transferiert ihn in einem Scheidetrichter. Dazu gibt man 100 ml Chloroform und 25 ml Wasser. Dann trennt man die wässerige Phase ab und wäscht nochmals mit 25 ml Wasser. Die organische Phase wird über Magnesiumsulfat getrocknet und eingedampft. Man erhält kristallines Äthyl-(1,3,4,5-tetrahydro-4-methyl-5-oxo-2H-1,4-benzodiazepin-2-yliden)acetat vom Schmelzpunkt 154—155°.

c) 32,2 g (0,124 Mol) Äthyl-(1,3,4,5-tetrahydro-4-methyl-5-oxo-2H-1,4-benzodiazepin-2-yliden)acetat werden in 300 ml Essigsäure gelöst und bei Raumtemperatur mit 12,8 g (0,186 Mol) Natriumnitrit versetzt. Man rührt noch 10 Minuten bei Raumtemperatur, gießt dann auf 1 l Wasser und extrahiert dreimal mit je 200 ml Chloroform. Die vereinigten Chloroformphasen werden mit 100 ml Wasser gewaschen, über Magnesiumsulfat getrocknet und eingedampft. Der Rückstand wird aus Essigester umkristallisiert. Man erhält Äthyl-4,5-dihydro-4-methyl-5-oxo-3H-1,4-benzodiazepin-2glyoxylat-$\alpha$-oxim vom Schmelzpunkt 173—174°.

d) 4,34 g (15 mMol) Äthyl-4,5-dihydro-4-methyl-5-oxo-3H-1,4-benzodiazepin-2-glyoxylat-$\alpha$-oxim werden in 60 ml Tetrahydrofuran gelöst, mit 30 ml Äthanol und 1,0 g Palladium auf Kohle (5%) versetzt und bei Normaldruck und Raumtemperatur hydriert. Nach Aufnahme der theoretischen Menge Wasserstoff (1 Stunde) wird vom Katalysator abfiltriert und im Vakuum eingedampft. Man erhält dabei $\alpha$-Amino-1,3,4,5-tetrahydro-5-oxo-2H-1,4-benzodiazepin-2-yliden-essigsäure-äthylester als blaßgelbes, oxydationsempfindliches Öl.

e) 4,1 g $\alpha$-Amino-1,3,4,5-tetrahydro-5-oxo-2H-1,4-benzodiazepin-2-yliden-essigsäure-äthylester werden in 25 ml Essigester gelöst, mit 3,1 ml (18,1 mMol) N,N-Dimethylformamid-diäthylacetal versetzt und 1/2 Stunde unter Rückfluß zum Sieden erhitzt. Nach dem Abkühlen wird das ausgefallene Material abgenutscht und aus Äthanol umkristallisiert. Durch Chromatographieren der Mutterlauge erhält man eine zweite Portion Äthyl-5,6-dihydro-5-methyl-6-oxo-4H-imidazo[1,5-a][1,4]benzodiazepin-3-carboxylat von gleicher Reinheit; Schmelzpunkt 166—168°.

## Beispiel 21

1,10 g $\alpha$-Amino-1,3,4,5-tetrahydro-4-methyl-5-oxo-2H-1,4-benzodiazepin-2-yliden-essigsäure-äthylester werden in 15 ml Toluol gelöst und mit 1,0 ml Orthoameisensäuretriäthylester versetzt. Das Gemisch wird während 50 Minuten zum Rückfluß erhitzt, anschließend abgekühlt und im Vakuum eingedampft. Der kristalline Rückstand wird in 25 ml Essigester suspendiert, abfiltriert und getrocknet. Man erhält Äthyl-5,6-dihydro-5-methyl-6-oxo-4H-imidazo[1,5-a][1,4]benzodiazepin-3-carboxylat vom Schmelzpunkt 165°.

## Beispiel 22

a) 1,10 g $\alpha$-Amino-1,3,4,5-tetrahydro-4-methyl-5-oxo-2H-1,4-benzodiazepin-2-yliden-essigsäureäthylester werden in 20 ml Methylenchlorid gelöst, mit 0,65 ml 37%iger wäßriger Formaldehyd-Lösung versetzt und 30 Minuten bei Raumtemperatur gerührt. Das Reaktionsgemisch wird dreimal mit je 10 ml Wasser gewaschen. Die organische Phase wird abgetrennt, getrocknet und im Vakuum eingedampft. Der Rückstand wird in Essigester gelöst und mit Aktivkohle entfärbt. Nach Abfiltrieren der Kohle und Einengen des Filtrates erhält man ein blaßgelbes Öl.

b) Dieses Öl wird in 10 ml Methylenchlorid gelöst und zusammen mit 3,5 g Mangandioxid 30 Minuten bei Raumtemperatur gerührt. Nach Filtrieren der Suspension unter Nachspülen mit Methylenchlorid wird im Vakuum eingedampft. Man erhält ein gelbes Öl, das nach Zugabe von Essigester und Äther kristallines Äthyl-5,6-dihydro-5-methyl-6-oxo-4H-imidazo[1,5-a][1,4]benzodiazepin-3-carboxylat vom Schmelzpunkt 162—164° liefert.

## Beispiel 23

1,0 g $\alpha$-Amino-1,3,4,5-tetrahydro-4-methyl-5-oxo-2H-1,4-benzodiazepin-2-yliden-essigsäureäthylester werden in 20 ml Methylenchlorid gelöst, mit 0,65 ml 37%iger wäßriger Formaldehyd-Lösung

versetzt und während 2 Stunden bei Raumtemperatur in Gegenwart von Luft gerührt. Die Lösung wird anschließend zweimal mit je 10 ml Wasser gewaschen. Die organische Phase wird abgetrennt, getrocknet und mit Aktivkohle entfärbt. Nach Abfiltrieren der Kohle wird im Vakuum eingedampft und der Rückstand an 20 g Kieselgel chromatographiert. Man eluiert zuerst mit Chloroform und anschließend mit Chloroform/Äthanol (985 : 15). Die Fraktionen, welche Äthyl-5,6-dihydro-5-methyl-6-oxo-4H-imidazo[1,5-a][1,4]benzodiazepin-3-carboxylat als Hauptkomponente enthalten, werden vereinigt und im Vakuum eingedampft. Der Rückstand wird in Äthanol gelöst und mit Diisopropyläther zur Kristallisation gebracht. Man erhält Äthyl-5,6-dihydro-5-methyl-6-oxo-4H-imidazo[1,5-a][1,4]benzodiazepin-3-carboxylat vom Schmelzpunkt 162—164°.

Beispiel 24

285 mg (1 mMol) Äthyl-5,6-dihydro-5-methyl-6-oxo-4H-imidazo[1,5-a][1,4]benzodiazepin-3-carboxylat und 66 mg Kaliumcyanid werden in 10 ml Methanol während 24 Stunden unter Rückfluß zum Sieden erhitzt. Dann engt man am Rotationsverdampfer ein, verteilt den Rückstand zwischen Wasser und Chloroform, extrahiert noch zweimal mit Chloroform, trocknet die Chloroformphase über Magnesiumsulfat und dampft ein. Man erhält dünnschichtchromatographisch einheitliches Methyl-5,6-dihydro-5-methyl-6-oxo-4H-imidazo[1,5-a][1,4]benzodiazepin-3-carboxylat vom Schmelzpunkt 212—214°.

Beispiel 25

285 mg (1 mMol) Äthyl-5,6-dihydro-5-methyl-6-oxo-4H-imidazo[1,5-a][1,4]benzodiazepin-3-carboxylat und 50 mg Kaliumcyanid werden in 10 ml iso-Propanol während 72 Stunden bei 60° gerührt. Dann engt man am Rotationsverdampfer ein, verteilt den Rückstand zwischen Wasser und Chloroform, extrahiert noch zweimal mit Chloroform, trocknet die Chloroformphase über Magnesiumsulfat und dampft ein. Man erhält dünnschichtchromatographisch einheitliches Isopropyl-5,6-dihydro-5-methyl-6-oxo-4H-imidazo[1,5-a][1,4]benzodiazepin-3-carboxylat vom Schmelzpunkt 190—192°.

Beispiel 26

Eine Mischung aus 1,42 g (5 mMol) Äthyl-5,6-dihydro-5-methyl-6-oxo-4H-imidazo[1,5-a][1,4]benzodiazepin-3-carboxylat, 100 mg Kaliumcyanid und 10 ml Äthylenglykol wird während 6 Stunden bei 130° gerührt, anschließend mit Chloroform verdünnt, mit Wasser gewaschen, über Magnesiumsulfat getrocknet und eingedampft. Durch Umkristallisieren des Rückstandes aus Essigester erhält man 2-Hydroxyäthyl-5,6-dihydro-5-methyl-6-oxo-4H-imidazo[1,5-a][1,4]benzodiazepin-3-carboxylat vom Schmelzpunkt 222—223°.

Beispiel 27

670 mg (2,3 mMol) Äthyl-5,6-dihydro-5-methyl-6-oxo-4H-imidazo[1,5-a][1,4]benzodiazepin-3-carboxylat und 497 mg (1,23 mMol) 2,4-Bis(p-methoxyphenyl)-1,3,2,4-dithiadiphosphetan-2,4-disulfid werden in 5 ml absolutem Toluol während $2^1/_2$ Stunden unter Rückfluß zum Sieden erhitzt. Man chromatographiert an Kieselgel mit Essigester und erhält Äthyl-5,6-dihydro-5-methyl-6-oxo-4H-imidazo[1,5-a][1,4]benzodiazepin-3-carboxylat vom Schmelzpunkt 164—165°.

Beispiel 28

a) Ein Gemisch von 14,0 g (79 mMol) 6-Methyl-isatosäureanhydrid, 7,04 g (79 mMol) Sarcosin und 80 ml Dimethylsulfoxid wird während 3 Stunden bei 110° gerührt. Die rot gefärbte Lösung wird im Hochvakuum eingedampft. Der Rückstand wird mit 80 ml Äthanol versetzt, wobei das Produkt auskristallisiert. Man erhält 3,4-Dihydro-4,6-dimethyl-2H-1,4-benzodiazepin-2,5(1H)-dion vom Schmelzpunkt 200—202°.

b) Eine Lösung von 9 g (44 mMol) 3,4-Dihydro-4,6-dimethyl-2H-1,4-benzodiazepin-2,5(1H)-dion in 100 ml Dimethylformamid wird mit 1,69 g Natriumhydrid (60%ige Öldispersion) versetzt und 1 Stunde gerührt. Die so erhaltene Suspension wird bei —20° tropfenweise mit 7,59 g (44 mMol) Diäthylchlorphosphat versetzt. Anschließend rührt man noch während 10 Minuten bei dieser Temperatur.

Inzwischen kühlt man eine Lösung von 5,60 g (50 mMol) Kalium-t-butylat in 10 ml Dimethylformamid in einem Aceeton/Trockeneis-Bad ab und versetzt mit 5,65 g (50 mMol) Isocyanessigsäureäthylester.

Diese Lösung wird bei —10 bis —20° dem obigen Reaktionsgemisch zugetropft. Man rührt das Reaktionsgemisch noch eine halbe Stunde ohne Kühlung, neutralisiert mit 5 ml Essigsäure und gießt auf ca. 300 ml Wasser. Die orangefarbene Lösung wird dreimal mit Chloroform extrahiert. Die organische Phase wird fünfmal mit Wasser gewaschen, über Magnesiumsulfat getrocknet und eingedampft. Durch Umkristallisieren des Rückstandes aus Alkohol und Äther erhält man Äthyl-5,6-dihydro-5,7-dimethyl-6-oxo-4H-imidazo[1,5-a][1,4]benzodiazepin-3-carboxylat vom Schmelzpunkt 146—147°.

Beispiel 29

a) Eine Lösung von 13,6 g (0,0768 Mol) 6-Methylisatosäureanhydrid und 8,8 g (0,0768 Mol) L-Prolin in 75 ml Dimethylsulfoxid wird während 1 Stunde auf 110° erwärmt. Anschließend dampft man im Hochvakuum zur Trockne ein und kristallisiert den Rückstand unter Zusatz von Aktivkohle aus Essigester um. Man erhält (S)-1,2,3,11a-Tetrahydro-6-methyl-5H-pyrrolo[2,1-c][1,4]benzodiazepin-5,11(10H)-dion vom Schmelzpunkt 212—214°.

b) Eine Lösung von 5,76 g (25 mMol) (S)-1,2,3,11a-Tetrahydro-6-methyl-5H-pyrrolo[2,1-c][1,4]benzodiazepin-5,11(10H)-dion in 40 ml Dimethylformamid wird unter einer Argonatmosphäre mit 0,9 g (25 mMol) Natriumhydrid (60%ige Öldispersion) versetzt und 1 Stunde gerührt. Zur erhaltenen Suspension tropft man bei —20° 4,23 g (25 mMol) Diäthylchlorphosphat zu und rührt 10 Minuten bei dieser Temperatur.

Inzwischen kühlt man eine Lösung von 2,80 g (25 mMol) Kalium-t-butylat in 7 ml Dimethylformamid in einem Aceton/Trockeneis-Bad ab und versetzt mit 2,32 g (25 mMol) Isocyanessigsäureäthylester. Diese Lösung wird bei —10 bis —20° dem obigen Rekationsgemisch zugetropft. Anschließend entfernt man die Kühlung, rührt eine halbe Stunde, neutralisiert mit 2,5 ml Essigsäure und gießt auf ca. 250 ml Wasser. Die orangefarbene Lösung wird dreimal mit Chloroform extrahiert; die organische Phase wird fünfmal mit Wasser gewaschen, über Magnesiumsulfat getrocknet und eingedampft. Durch Säulenchromatographie und anschließendes Umkristallisieren aus Essigester/Hexan resultiert Äthyl-(S)-11,12,13,13a-tetrahydro-8-methyl-9-oxo-9H-imidazo[1,5-a]pyrrolo[2,1-c][1,4]benzodiazepin-1-carboxylat vom Schmelzpunkt 152—153°.

Beispiel 30

Unter einer Argonatmosphäre versetzt man eine Lösung von 21,6 g (0,10 Mol) (S)-(+)-1,2,3,11a-Tetrahydro-5H-pyrrolo[2,1-c][1,4]benzodiazepin-5,11(10H)-dion in 100 ml trockenem Dimethylformamid mit 13,5 g (0,12 Mol) Kalium-t-butylat, wobei die Temperatur von 24° auf 46° steigt. Man kühlt auf Raumtemperatur und tropft zwischen 18—23° 18,2 g (0,105 Mol) Diäthylchlorphosphat zu.

Separat werden 11,2 g (0,10 Mol) Kalium-t-butylat in 30 ml Dimethylformamid gelöst. Diese Lösung wird auf ca. —50° gekühlt und unter Argon mit 11,3 g (0,10 Mol) Isocyanessigsäureäthylester versetzt. Anschließend wird diese Lösung bei 18—23° unter Kühlung zum obigen Reaktionsgemisch getropft. Man rührt während 1 Stunde bei Raumtemperatur, gibt 5 ml Essigsäure zu, gießt dann auf 500 ml Wasser und extrahiert zweimal mit je 200 ml Chloroform. Die vereinigten Chloroformphasen werden dreimal mit je 300 ml Wasser gewaschen, über Magnesiumsulfat getrocknet und eingedampft. Zum öligen Rückstand gibt man 150 ml Essigsäure und läßt bei 0° kristallisieren. Man nutscht die ausgeschiedenen Kristalle ab, wäscht mit kaltem Essigester und erhält Äthyl-(S)-11,12,13,13a-tetrahydro-9-oxo-9H-imidazo[1,5-a]pyrrolo[2,1-c][1,4]benzodiazepin-1-carboxylat vom Schmelzpunkt 196—197°. Die Mutterlauge wird eingedampft und der Rückstand wird in 50 ml Essigester gelöst. Daraus kristallisiert eine weitere Portion des obigen Produkts; Schmelzpunkt 196—197°.

Beispiel 31

Eine Mischung aus 311 mg (1 mMol) Äthyl-(S)-11,12,13,13a-tetrahydro-9-oxo-9H-imidazo[1,5-a]pyrrolo[2,1-c][1,4]benzodiazepin-1-carboxylat, 66 mg Kaliumcyanid und 10 ml absolutes Methanol wird während 6 Stunden unter Rückfluß zum Sieden erhitzt. Man engt ein, gibt wenig Wasser zu und extrahiert dreimal mit je 10 ml Chloroform, trocknet mit Magnesiumsulfat, dampft ein und kristallisiert aus Essigester/Hexan um. Man erhält Methyl-(S)-11,12,13,13a-tetrahydro-9-oxo-9H-imidazo[1,5-a]pyrrolo[2,1-c][1,4]benzodiazepin-1-carboxylat vom Schmelzpunkt 165—167°.

Beispiel 32

Eine Mischung aus 5,0 g (0,0161 Mol) Äthyl-(S)-11,12,13,13a-tetrahydro-9-oxo-9H-imidazo[1,5-a]pyrrolo[2,1-c][1,4]benzodiazepin-1-carboxylat und 3,46 g (0,0086 Mol) 2,4-Bis(p-methoxyphenyl)1,3,2,4-dithiadiphosphetan-2,4-disulfid wird mit 30 ml Toluol während $1^1/_2$ Stunden unter Rückfluß zum Sieden erhitzt. Diese Lösung wird an Kieselgel chromatographiert, wobei man Essigester als Elutionsmittel verwendet. Man erhält Äthyl-(S)-11,12,13,13a-tetrahydro-9-thioxo-9H-imidazo[1,5-a]pyrrolo[2,1-c][1,4]benzodiazepin-1-carboxylat, welches aus 180 ml Essigester umkristallisiert wird und dann bei 227—229° schmilzt.

Beispiel 33

934 mg (3 mMol) Äthyl-(S)-11,12,13,13a-tetrahydro-9-oxo-9H-imidazo[1,5-a]pyrrolo[2,1-c][1,4]benzodiazepin-1-carboxylat werden mit 198 mg (3 mMol) Kaliumcyanid in 30 ml trockenem 2-Propanol während 48 Stunden bei 60° gerührt. Das Reaktionsgemisch wird im Vakuum eingedampft; der Rückstand mit 30 ml 2-Propanol und 198 mg (3 mMol) Kaliumcyanid versetzt und während 22 Stunden unter Rückfluß zum Sieden erhitzt. Nach Eindampfen im Vakuum wird der Rückstand mit Wasser versetzt und dreimal mit je 30 ml Chloroform extrahiert. Die vereinigten Chloroformphasen werden dreimal mit je 20 ml Wasser gewaschen, über Magnesiumsulfat getrocknet und eingedampft. Durch Umkristallisieren aus Essigester/n-Hexan erhält man Isopropyl-(S)-11,12,13,13a-tetrahydro-9-oxo-9H-imidazo[1,5-a]pyrrolo[2,1-c][1,4]benzodiazepin-1-carboxylat vom Schmelzpunkt 207—208°.

Beispiel 34

a) Eine Mischung aus 175 g (0,93 Mol) 3-Amino-2-thiophencarbonsäuremethylester-hydrochlorid, 1,8 l n-Butanol und 77 g Natronlauge werden während 30 Minuten unter Rückfluß zum Sieden erhitzt. Nach Einengen der erhaltenen Suspension am Rotationsverdampfer wird das resultierende Gemisch aus Natriumsalz der 3-Amino-2-thiophencarbonsäure und von Kochsalz direkt für die nächste Stufe eingesetzt. Dazu wird das Gemisch mit 800 ml Wasser, 280 ml konz. Salzsäure und 230 ml Tetrahydrofuran versetzt. Durch diese Mischung leitet man bei 15—25° während $2^1/_2$ Stunden Phosgen ein und anschließend während 15 Minuten Luft. Der ausgefallene Festkörper wird abgenutscht, mit Wasser gewaschen und getrocknet. Man erhält 2H-Thieno[3,2-d][1,3]oxazin-2,4(1H)-dion vom Schmelzpunkt 220—221°.

b) Eine Lösung von 34,3 g (202 mMol) 2H-Thieno[3,2-d][1,3]oxazin-2,4(1H)-dion und 23,3 g (202 mMol) L-Prolin in 200 ml Dimethylsulfoxid wird 1 Stunde bei 110° gerührt. Die erhaltene braune Lösung wird auf 2 l Wasser gegossen und über Nacht bei Raumtemperatur gerührt. Das ausgefallene Produkt wird abgenutscht, am Rotationsverdampfer getrocknet und mit ca. 200 ml siedenden Essigester gewaschen. Man erhält dabei (S)-5a,6,7,8-Tetrahydro-5H-pyrrolo[1,2]thieno[3,2-e][1,4]diazepin-5,10(4)-dion vom Schmelzpunkt 244—247°.

c) Unter einer Argonatmosphäre werden 7 g (31,5 mMol) (S)-5a,6,7,8-Tetrahydro-5H-pyrrolo-[1,2]thieno[3,2-e][1,4]diazepin-5,10(4)-dion in 30 ml Dimethylformamid suspendiert und bei —50° mit 3,92 g (35 mMol) Kalium-t-butylat versetzt. Man rührt die Lösung 10 Minuten bei —50°, tropft bei dieser Temperatur 6,0 g (35 mMol) Diäthylchlorphosphat zu und rührt während einer halben Stunde.

Separat werden 3,92 g (35 mMol) Kalium-t-butylat in 7 ml Dimethylformamid gelöst, im Aceton/Trockeneis-Bad gekühlt und mit 3,95 g (35 mMol) Isocyanessigsäureäthylester versetzt. Die erhaltene orangefarbene Lösung wird bei —50° zum obigen Reaktionsgemisch zugetropft. Anschließend rührt man noch 10 Minuten bei —50 bis —60°, neutralisiert mit 3,2 ml Essigsäure und gießt auf ca. 250 ml Wasser.Man extrahiert zweimal mit je 200 ml Chloroform, wäscht die vereinigten Chloroform-Phasen fünfmal mit je 300 ml Wasser, trocknet über Magnesiumsulfat und dampft ein. Durch Säulenchromatographie und anschließendes Umkristallisieren aus Essigester erhält man Äthyl-(S)-10,11,12,12a-tetrahydro-8-oxo-8H-imidazo[5,1-c]pyrrolo[1,2-a]thieno[3,2-e][1,4]diazepin-1-carboxylat vom Schmelzpunkt 212,5—213°.

Beispiel 35

1,50 g Äthyl-(S)-10,11,12,12a-tetrahydro-8-oxo-8H-imidazo[5,1-c]pyrrolo[1,2-a]thieno[3,2-e][1,4]diazepin-1-carboxylat werden zusammen mit 100 mg pulverisiertem Kaliumcyanid in 10 ml Methanol während 20 Stunden bei 50° gerührt. Die Lösung wird eingeengt und der Rückstand in Chloroform aufgenommen. Das Unlösliche wird abgenutscht und das Filtrat eingedampft. Durch Umkristallisieren aus Chloroform/Hexan erhält man aus dem Rückstand Methyl-(S)-10,11,12,12a-tetrahydro-8-oxo-8H-imidazo[5,1-c]pyrrolo[1,2-a]thieno[3,2-e][1,4]diazepin-1-carboxylat vom Schmelzpunkt 192—193°.

Beispiel 36

a) Eine Mischung aus 30,0 g (177 mMol) 2H-Thieno[3,2-d][1,3]oxazin-2,4(1H)-dion und 17,3 g (195 mMol) Sarcosin in 100 ml Dimethylsulfoxid wird während $1^1/_2$ Stunden bei 110° gerührt. Die

dunkelbraune Lösung wird auf ca. 600 ml Eiswasser gegossen. Das abgeschiedene Öl wird in ca. 200 ml Essigester aufgenommen; die wäßrige Phase wird bis zur beginnenden Kristallisation im Vakuum eingedampft. Man kühlt ca. 3 Stunden im Eisbad, nutscht das ausgefallene Material ab und wäscht es mit wenig Wasser. Nach dem Trocknen am Rotationsverdampfer resultieren 3,4-Dihydro-4-methyl-2H-thieno[3,2-e][1,4]diazepin-2,5(1H)-dion vom Schmelzpunkt 270—272°.

b) Unter einer Argonatmosphäre versetzt man eine auf 15° abgekühlte Suspension von 4,8 g (24,5 mMol) 3,4-Dihydro-4-methyl-2H-thieno[3,2-e][1,4]diazepin-2,5(1H)-dion in 30 ml Dimethylformamid mit 3,28 g (29,4 mMol) Kalium-t-butylat. Nach Abkühlen der dunkelbraunen Lösung auf —40° gibt man tropfenweise 3,7 ml (25,7 mMol) Diäthylchlorphosphat bei —40 bis —30° hinzu. Man entfernt das Kühlbad und rührt während 20 Minuten, wobei die Temperatur auf —15° ansteigt.

Separat werden 3,0 g (27 mMol) Kalium-t-butylat in 8 ml Dimethylformamid gelöst, im Aceton/Trockeneis-Bad gekühlt und mit 3,1 ml (27 mMol) Isocyanessigsäureäthylester versetzt. Die orangefarbene Lösung wird bei —15 bis —10° zum obigen Reaktionsgemisch zugetropft. Nach entfernen des Kühlbades wird gerührt, bis die Temperatur auf 20° angestiegen ist. Anschließend neutralisiert man mit 2 ml Essigsäure und gießt auf ca. 150 ml Wasser und extrahiert dreimal mit je 150 ml Chloroform. Die vereinigten Chloroform-Phasen werden dreimal mit je 100 ml gesättigter Natriumchlorid-Lösung gewaschen, über Magnesiumsulfat getrocknet und im Vakuum eingedampft. Durch Säulenchromatographie und anschließendes Umkristallisieren aus Essigester/n-Hexan erhält man Äthyl-5,6-dihydro-5-methyl-6-oxo-4H-imidazo[1,5-a]thieno[2,3-f][1,4]diazepin-3-carboxylat vom Schmelzpunkt 160—162°.

## Beispiel 37

Eine Suspension von 430 mg (1,5 mMol) Äthyl-5,6-dihydro-5-methyl-6-oxo-4H-imidazo[1,5-a]thieno[2,3-f][1,4]diazepin-3-carboxylat und 99 mg (1,5 mMol) Kaliumcyanid in 15 ml absolutem Methanol wird unter Rühren während 3 Stunden zum Rückfluß erhitzt. Nach eindampfen im Vakuum versetzt man mit ca. 20 ml Eiswasser und extrahiert dreimal mit je ca. 30 ml Chloroform. Die vereinigten Chloroformphasen werden zweimal mit je ca. 20 ml Wasser gewaschen, über Magnesiumsulfat getrocknet und eingedampft. Nach Waschen in siedendem Essigester erhält man Methyl-5,6-dihydro-5-methyl-6-oxo-4H-imidazo[1,5-a]thieno[2,3-f][1,4]diazepin-3-carboxylat vom Schmelzpunkt 244—245°.

## Beispiel 38

Eine Lösung von 16,85 g (0,075 Mol) 3,4-Dihydro-7-chlor-4-methyl-2H-1,4-benzodiazepin-2,5(1H)-dion in 150 ml Tetrahydrofuran versetzt man unter einer Argonatmosphäre mit 8,98 g (0,08 Mol) Kalium-t-butylat, kühlt auf —10°, tropft 12,94 g (0,08 Mol) Diäthylchlorphosphat zu und rührt während 20 Minuten bei —10°.

Separat werden 8,98 g (0,08 Mol) Kalium-t-butylat in 30 ml Dimethylformamid gelöst. Diese Lösung wird unter Argon bei ca. —50° mit 9,05 g (0,08 Mol) Isocyanessigsäureäthylester versetzt. Anschließend wird diese Lösung zum obigen Reaktionsgemisch getropft. Man rührt während 1 Stunde bei Raumtemperatur, gibt 5 ml Essigsäure zu, gießt dann auf 500 ml Wasser und extrahiert dreimal mit je 200 ml Chloroform. Die vereinigten Chloroform-Auszüge werden zweimal mit je 200 ml Wasser gewaschen, über Magnesiumsulfat getrocknet und eingedampft. Zum Rückstand gibt man 75 ml Essigester und läßt bei 0° kristallisieren. Man nutscht die ausgeschiedenen Kristalle ab, wäscht mit kaltem Essigester und kristallisiert erneut aus 125 ml Essigester um. Dabei erhält man Äthyl-8-chlor-5,6-dihydro-5-methyl-6-oxo-4H-imidazo[1,5-a][1,4]benzodiazepin-3-carboxylat vom Schmelzpunkt 188—189°.

## Beispiel 39

Eine Lösung von 35 g (0,14 Mol) (S)-(+)-7-Chlor-1,2,3,11a-tetrahydro-5H-pyrrolo[2,1-c][1,4]benzodiazepin-5,11(10H)-dion in 170 ml trockenem Dimethylformamid versetzt man unter einer Argonatmosphäre mit 17,3 g (0,15 Mol) Kalium-t-butylat, wobei die Temperatur von 24° auf 40° steigt. Man kühlt auf —30° und tropft, zwischen —30 und —20°, 25 g (0,15 Mol) Diäthylchlorphosphat zu.

Separat werden 16,8 g Kalium-t-butylat (0,15 Mol) in 50 ml Dimethylformamid gelöst. Diese Lösung wird auf ca. —50° gekühlt und unter Argon mit 17,42 g (0,15 Mol) Isocyanessigsäureäthylester versetzt. Anschließend wird diese Lösung bei —20 bis —10° zum obigen Reaktionsgemisch zugetropft. Man rührt während 1 Stunde ohne Kühlung, gibt 14 ml Essigsäure zu, gießt anschließend auf ca. 1000 ml Wasser und extrahiert dreimal mit je 250 ml Chloroform. Die vereinigten Chloroformphasen werden fünfmal mit je 300 ml Wasser gewaschen, über Magnesiumsulfat getrocknet und eingedampft. Der Rückstand wird aus 500 ml Essigester umkristallisiert. Man erhält (S)-(+)-Äthyl-7-chlor-11,12,13,13a-tetrahydro-9-oxo-9H-imidazo[1,5-a]pyrrolo[2,1-c][1,4]-benzodiazepin-1-carboxylat vom Schmelzpunkt

242—244°.

Beispiel 40

a) Eine Lösung von 39,5 g 5-Brom-isatosäureanhydrid und 14,5 g Sarcosin in 150 ml Dimethylsulfoxid wird unter Rühren auf 100° erwärmt. Dabei setzt ab ca. 70° eine lebhafte $CO_2$-Entwicklung ein, welche nach ca. 30 Minuten beendet ist. Man rührt noch 30 Minuten bei 100° und gießt danach das Reaktionsgemisch auf 900 ml Eiswasser (Temperatur 5°) und nutscht das ausgefallene Material ab. Die Kristalle werden mit Wasser gewaschen und anschließend im Vakuumtrockenschrank über Phosphorpentoxid bei 50° getrocknet. Man erhält 7-Brom-3,4-dihydro-4-methyl-2H-1,4-benzodiazepin-2,5(1H)-dion, als hellbeige Kristalle. Eine aus Methanol umkristallisierte Probe hat einen Schmelzpunkt von 260—261°.

b) Unter einer Argonatmosphäre versetzt man eine Lösung von 17 g (63 mMol) 7-Brom-3,4-dihydro-4-methyl-2H-1,4-benzodiazepin-2,5(1H)-dion in 100 ml Dimethylformamid mit 7,5 g (67 mMol) Kalium-t-butylat. Die erhaltene Lösung wird bei —30° mit 11,56 g (67 mMol) Diäthylchlorphosphat tropfenweise versetzt und 10 Minuten bei —20° gerührt.

Inzwischen kühlt man eine Lösung von 7,5 g (67 mMol) Kalium-t-butylat in 30 ml Dimethylformamid in einem Aceton/Trockeneis-Bad ab und versetzt mit 7,58 g (67 mMol) Isocyanessigsäureäthylester. Diese Lösung wird bei —10 bis —20° dem obigen Reaktionsgemisch zugegeben. Man entfernt die Kühlung, rührt eine halbe Stunde, neutralisiert mit 8 ml Essigsäure und gießt auf ca. 600 ml Wasser. Die orangefarbene Lösung wird dreimal mit Chloroform extrahiert. Die organische Phase wird fünfmal mit Wasser gewaschen, über Magnesiumsulfat getrocknet und eingedampft. Durch Umkristallisieren aus Essigester erhält man Äthyl-8-brom-5,6-dihydro-5-methyl-6-oxo-4H-imidazo[1,5-a][1,4]benzodiazepin-3-carboxylat vom Schmelzpunkt 194—195°.

Beispiel 41

a) Eine Suspension von 12,2 g (0,063 Mol) 3-Carbomethoxy-4-aminothiophen-hydrochlorid in 100 ml Methylenchlorid wird mit 10 ml (0,126 Mol) Chloracetylchlorid und anschließend tropfenweise mit 17,6 ml (0,126 Mol) Triäthylamin versetzt, wobei die Temperatur unter 25° gehalten wird. Man gießt die Lösung auf Wasser und extrahiert zweimal mit je 50 ml Methylenchlorid. Die vereinten Methylenchlorid-Auszüge werden dreimal mit je 50 ml Wasser gewaschen, über Magnesiumsulfat getrocknet und eingedampft. Nach Chromatographie an Kieselgel mit Methylenchlorid als Elutionsmittel erhält man kristallines 3-Carbomethoxy-4-[(chloracetyl)amino]thiophen vom Schmelzpunkt 98—100°.

b) Eine Lösung von 13,25 g (0,057 Mol) 3-Carbomethoxy-4-[(chloracetyl)amino]thiophen in einem Gemisch aus 60 ml Dimethylformamid und 60 ml Toluol wird nach Zugabe von 15,7 g Kaliumcarbonat und 0,05 g Kaliumjodid unter stetem Einleiten von Methylamin auf 50° erwärmt. Nach 1 Stunde wird das Gemisch auf Eiswasser gegossen und dreimal mit Toluol extrahiert. Die vereinigten Toluolphasen werden zweimal mit Wasser gewaschen, über Magnesiumsulfat getrocknet und eingedampft. Das Rohprodukt wird an Kieselgel mit Chloroform als Elutionsmittel chromatographiert. Man erhält dabei 3-Carbomethoxy-4-/[(methylamino)acetyl]amino/thiophen als Oel. Das entsprechende Hydrochlorid schmilzt bei 234—236° (Zersetzung).

c) Insgesamt 9,5 g (0,036 Mol) 3-Carbomethoxy-4-/[(methylamino)acetyl]amino/thiophen werden in 2 g Portionen unter Schutzgas und Rühren während 5 Minuten auf 250° erhitzt. Die Rohprodukte aus den verschiedenen Ansätzen werden mit Methanol zusammengespült und eingeengt. Durch mehrfache fraktionierte Kristallisationen aus Dimethylformamid/Äther erhält man reines 3,4-Dihydro-4-methyl-5H-thieno[3,4-e]-[1,4]diazepin-2,5(1H)-dion vom Schmelzpunkt 263—265°.

d) Eine Mischung aus 1,90 g (9,7 mMol) 3,4-Dihydro-4-methyl-5H-thieno[3,4-e]-1,4-diazepin-2,5(1H)-dion und 15 ml Dimethylformamid wird unter Argon mit 0,35 g (9,7 mMol) Natriumhydrid (60-proz. Oeldispersion) versetzt und 1 Stunde gerührt. Dieser Lösung werden bei —30° 1,40 ml (9,7 mMol) Diäthylchlorphosphat zugetropft. Man rührt 10 Minuten bei — 20°.

Inzwischen wird eine Lösung von 1,08 g (9,7 mMol) Kalium-t-butylat in 3 ml Dimethylformamid auf — 50° gekühlt und mit 1,09 g (9,7 mMol) Isocyanessigsäureäthylester versetzt. Diese Lösung wird dem obigen Reaktionsgemisch bei — 10 und — 20° zugetropft und das erhaltene Gemisch eine halbe Stunde gerührt. Man neutralisiert es mit 1 ml Essigsäure, gießt auf ca. 200 ml Wasser und extrahiert dreimal mit Chloroform. Die vereinigten Chloroform-Phasen werden fünfmal mit Wasser gewaschen, über Magnesiumsulfat getrocknet und eingedampft. Durch Säulenchromatographie des Rohproduktes und anschließendes Umkristallisieren aus Essigester erhält man Äthyl-5,6-dihydro-5-methyl-6-oxo-4H-imidazo[1,5-a]thieno[3,4-f][1,4]diazepin-3-carboxylat vom Schmelzpunkt 207,5—208,5°.

Beispiel 42

a) Eine Lösung von 46,7 g (150 mMol) Äthyl-(S)-11,12,13,13a-tetrahydro-9-oxo-9H-imidazo[1,5-a]pyrrolo[2,1-c]-[1,4]benzodiazepin-1-carboxylat in 300 ml trockenem Tetrahydrofuran wird bei ca. 35° tropfenweise mit einer Lösung von 8,3 g (377 mMol) Lithiumborhydrid in 110 ml trockenem Tetrahydrofuran versetzt. Das Reaktionsgemisch wird während ca. 40 Stunden unter Rückfluß zum Sieden erhitzt, auf Raumtemperatur abgekühlt, mit 110 ml 12-proz. Salzsäure und 20 ml konz. Salzsäure versetzt und nochmals während 2 Stunden unter Rückfluß zum Sieden erhitzt. Nach Entfernen des Tetrahydrofurans im Vakuum wird der Rückstand mit konz. Ammoniak alkalisch gestellt. Das ausgefallene Material wird abgenutscht und mit Wasser gewaschen. Durch Umkristallisieren aus Äthanol erhält man (S)-11,12,13,13a-Tetrahydro-1-(hydroxymethyl)-9H-imidazo[1,5-a]pyrrolo-[2,1-c][1,4]benzodiazepin-9-on vom Schmelzpunkt 212—214°.

b) Eine Suspension von 12,1 g (45 mMol) (S)-11,12,13,13a-Tetrahydro-1-(hydroxymethyl)-9H-imidazo[1,5-a]pyrrolo[2,1-c][1,4]benzodiazepin-9-on in 300 ml Methylenchlorid wird mit 70 g Mangan-IV-oxid versetzt und während ca. 3/4 Stunden bei Raumtemperatur gerührt. Nach Filtrieren über Dicalit wird das Filtrat im Vakuum eingedampft und der Rückstand mit ca. 100 ml siedendem Essigester gewaschen. Man erhält (S)-11,12,13,13a-Tetrahydro-9-oxo-9H-imidazo[1,5-a]pyrrolo[2,1-c][1,4]benzodiazepin-1-carboxaldehyd vom Schmelzpunkt 206—208°.

c) Eine Suspension von 2,67 g (10 mMol) (S)-11,12,13,13a-Tetrahydro-9-oxo-9H-imidazo[1,5-a]pyrrolo[2,1-c][1,4]-benzodiazepin-1-carboxaldehyd und 0,85 g (12,5 mMol) Hydroxylamin-hydrochlorid in 60 ml Wasser wird bei Raumtemperatur tropfenweise mit einer Lösung von 3,57 g (12,5 mMol) Natriumcarbonat. 10 $H_2O$ in 10 ml Wasser versetzt. Das Gemisch wird während 4 Stunden bei 70° gerührt. Nach Abkühlen wird das ausgefallene Material abgenutscht, mit Wasser gewaschen und direkt aus 50 ml Dimethylformamid umkristallisiert. Es resultiert (S)-11,12,13,13a-Tetrahydro-9-oxo-9H-imidazo[1,5-a]pyrrolo[2,1-c][1,4]benzodiazepin-1-carboxaldehyd-1-oxim vom Schmelzpunkt 285°.

d) Eine Lösung von 5,24 g (18,5 mMol) (S)-11,12,13,13a-Tetrahydro-9-oxo-9H-imidazo[1,5-a]pyrrolo[2,1-c][1,4]-benzodiazepin-1-carboxaldehyd-1-oxim in 50 ml Essigsäureanhydrid wird während 3 Stunden unter Rückfluß zum Sieden erhitzt und anschließend im Vakuum eingedampft. Den Rückstand nimmt man in Chloroform auf, wäscht die Lösung einmal mit ca. 30 ml gesättigter Natriumhydrogencarbonat-Lösung und einmal mit ca. 30 ml Wasser, trocknet über Magnesiumsulfat und dampft ein. Durch Säulenchromatographie und anschließendes Umkristallisieren aus Essigester erhält man (S)-11,12,13,13a-Tetrahydro-9-oxo-9H-imidazo[1,5-a]pyrrolo-[2,1-c][1,4]benzodiazepin-1-carbonitril vom Schmelzpunkt 223—225°.

## Beispiel 43

Eine Mischung aus 1,92 g (9 mMol) (S)-3,11a-Dihydro-5H-pyrrolo[2,1-c][1,4]benzodiazepin-5,11-(10H)-dion und 15 ml Dimethylformamid wird mit 0,33 g (9 mMol) Natriumhydrid (60-proz. Oeldispersion) versetzt und eine halbe Stunde gerührt. Bei — 30° tropft man 1,55 g (9 mMol) Diäthylchlorphosphat zu und rührt 10 Minuten bei — 20°.

Separat wird eine Lösung von 1 g (9 mMol) Kalium-t-butylat in 3 ml Dimethylformamid auf ca. — 45° gekühlt und mit 1,02 g (9 mMol) Isocyanessigsäureäthylester versetzt. Die erhaltene orangefarbene Lösung wird bei — 20 bis — 10° dem obigen Reaktionsgemisch zugetropft und das erhaltene Gemisch eine halbe Stunde ohne Kühlung gerührt. Anschließend neutralisiert man mit 1 ml Essigsäure, gießt auf Wasser und extrahiert dreimal mit Chloroform. Die vereinigten Chloroform-Auszüge werden fünfmal mit Wasser gewaschen, über Magnesiumsulfat getrocknet und eingedampft. Durch Chromatographieren des Rohproduktes und Umkristallisieren des erhaltenen Materials aus Essigester/Hexan resultiert Äthyl-(R,S)-11,13a-dihydro-9-oxo-9H-imidazo[1,5-a]pyrrolo-[2,1-c][1,4]benzodiazepin-1-carboxylat vom Schmelzpunkt 184-185,5°.

## Beispiel 44

Eine Mischung aus 3,12 g (10 mMol) Äthyl-(S)-10,11,12,12a-tetrahydro-8-oxo-8H-imidazo[5,1-c]pyrrolo[1,2-a]-thieno[3,2-e][1,4]diazepin-1-carboxylat, 80 mg pulverisiertem Kaliumcyanid und 30 ml Äthylenglykol wird über Nacht bei 100° gerührt. Nach Entfernen des Äthylenglykols wird der Rückstand durch Säulenchromatographie gereinigt. Man erhält 2-Hydroxyäthyl-(S)-10,11,12,12a,-tetrahydro-8-oxo-8H-imidazo[5,1-c]pyrrolo[1,2-a]thieno[3,2-e][1,4]diazepin-1-carboxylat vom Schmelzpunkt 182—184°.

## Beispiel 45

a) Eine Lösung von 2,32 g (10 mMol) (2R,11aS)-1,2,3,11a-Tetrahydro-2-hydroxy-5H-pyrrolo[2,1-c][1,4]benzodiazepin-5,11(10H)-dion in 20 ml Pyridin wird mit 0,86 ml (11 mMol) Methansulfochlorid versetzt und während 4 Stunden bei Raumtemperatur gerührt. Nach Eindampfen im Vakuum wird der Rückstand mit Wasser versetzt und dreimal mit je ca. 60 ml Chloroform extrahiert. Die vereinigten Chloroform-Auszüge werden zweimal mit je ca. 30 ml Wasser gewaschen über Magnesiumsulfat getrocknet und eingedampft. Durch Umkristallisieren aus Äthanol erhält man (2R,11aS)-2,3,5,10,11,11a-Hexahydro-5,11-dioxo-1H-pyrrolo[2,1-c][1,4]benzodiazepin-3-yl-methansulfonat vom Schmelzpunkt 179-181°.

b) Eine Suspension von 5,92 g (135,7 mMol) Natriumhydrid (55-proz. Oeldispersion) in 150 ml trockenem Dimethylformamid wird mit 17,0 g (54,8 mMol) (2R,11aS)-2,3,5,10,11,11a-Hexahydro-5,11-dioxo-1H-pyrrolo[2,1-c][1,4]benzodiazepin-3-yl-methansulfonat versetzt und während ca. 16 Stunden bei Raumtemperatur und ca. 16 Stunden bei 40° gerührt. Man versetzt mit Wasser, gießt auf ca. 300 ml Eiswasser, neutralisiert mit Eisessig und läßt 2 Stunden im Eisbad stehen. Das ausgefallene Material wird abgenutscht, mit Wasser gewaschen und aus Dioxan umkristallisiert. Dabei erhält man (R,S)-1,11a-Dihydro-5H-pyrrolo-[2,1-c][1,4]benzodiazepin-5,11(10H)-dion vom Schmelzpunkt 236—238°.

c) Eine Suspension von 1,01 g (23,2 mMol) Natriumhydrid (55-proz. Oeldispersion) in 35 ml trockenem Dimethylformamid wird mit 4,15 g (19,4 mMol) (R,S)-1,11a-Dihydro-5H-pyrrolo[2,1-c][1,4]benzodiazepin-5,11(10H)-dion versetzt. Nach beendeter Gasentwicklung kühlt man auf —40° ab und versetzt tropfenweise 3,1 ml (20,2 mMol) Diäthylchlorphosphat. Nach Entfernen des Kühlbades versetzt man die Reaktionsmischung bei —20 bis —15° tropfenweise mit einer in einem Aceton/Trockeneis-Bad abgekühlten Lösung von 2,33 g (21,3 mMol) Kalium-t-butylat und 2,7 ml (21,3 mMol)Isocyanessigsäureäthylester in 8 ml trockenem Dimethylformamid. Wenn die Temperatur 20° erreicht, wird mit Eisessig neutralisiert, auf ca. 200 ml Wasser gegossen und über Nacht stehen gelassen. Das ausgefallene Produkt wird abgenutscht, mit Wasser gewaschen und getrocknet. Durch Umkristallisieren aus Essigester erhält man Äthyl-(R,S)-13,13a-dihydro-9-oxo-9H-imiazo[1,5-a]pyrrolo[2,1-c][1,4]benzodiazepin-1-carboxylat vom Schmelzpunkt 178°.

## Beispiel 46

a) Eine Mischung aus 10,0 g Äthyl-5,6-dihydro-5-methyl-6-oxo-4H-imidazo[1,5-a][1,4]benzodiazepin-3-carboxylat, 200 ml Methanol, 0,5 g Ammoniumchlorid und 30 g Ammoniak (100%) wird in einem Autoklaven unter Stickstoff (40 bar) bei einer Temperatur von 120° während 12 Stunden gerührt. Nach dem Erkalten und Ablassen des Überdruckes wird das Reaktionsgemisch zur Trockene eingedampft. Nach Verteilen des Rückstandes zwischen Methylenchchlorid und Wasser wird die organische Phase getrocknet und eingedampft. Der Rückstand wird aus Essigester umkristallisiert, man erhält dabei 5,6-Dihydro-5-methyl-6-oxo-4H-imidazo[1,5-a][1,4]-benzodiazepin-3-carboxamid als weiße Kristalle vom Schmelzpunkt 274—275°.

b) 0,5 g 5,6-Dihydro-5-methyl-6-oxo-4H-imidazo[1,5-a]-[1,4]benzodiazepin-3-carboxamid werden zusammen mit 0,5 g Sicapent in 150 ml Toluol während 40 Stunden unter Rühren zum Rückfluß erhitzt. Nach jeweils 16, 19 bzw. 24 Stunden versetzt man mit 0,5 g Sicapent. Das Reaktionsgemisch wird anschließend abgekühlt und mit Wasser versetzt. Die Mischung wird mit 28-proz. Natronlauge auf pH 9 eingestellt. Nach Abtrennen der organischen Phase wird die alkalische, wässerige Phase noch zweimal mit je 250 ml Essigester ausgeschüttelt. Die organischen Auszüge werden zweimal mit gesättigter Kochsalzlösung gewaschen, getrocknet und eingedampft. Durch Kristallisation des Rückstandes aus Methylenchlorid/Hexan wird ein Teil des nicht umgesetzten Ausgangsmaterials zurückgewonnen. Die Mutterlauge wird eingedampft und an 100 g Kieselgel unter Eluieren mit Essigester und Alkohol chromatographiert. Durch Umkristallisieren des so erhaltenen Stoffes aus Aceton erhält man 5,6-Dihydro-5-methyl-6-oxo-4H-imidazo[1,5-a][1,4]benzodiazepin-3-carbonitril vom Schmelzpunkt 184—185°.

## Beispiel 47

a) Eine Mischung aus 12,1 g 5-(Trifluormethyl)-isatin, 50 ml Essigsäure und 50 ml Essigsäureanhydrid wird zwischen 80 und 90° portionsweise mit 10,0 g Chromtrioxid versetzt. 5 Minuten nach der letzten Zugabe kühlt man auf Raumtemperatur ab, nutscht die ausgeschiedenen Kristalle ab und wäscht diese mit Wasser. Man erhält dabei 5-(Trifluormethyl)-isatosäureanhydrid vom Schmelzpunkt 268— 266°.

b) Eine Suspension von 5,76 g (24,9 mMol) 5-(Trifluormethyl)-isatosäureanhydrid und 2,44 g (27,4 mMol) Sarcosin in 8 ml Dimethylsulfoxid wird während 4 Stunden bei 100° gerührt, anschließend auf 70 ml Wasser gegossen und im Vakuum zur Trockene eingedampft. Durch Säulenchromatographie an Kieselgel mit Essigester/n-Hexan (9:1) als Elutionsmittel und Umkristallisieren des erhaltenenRohproduktes aus Ähanol erhält man 3,4-Dihydro-4-methyl-7-(trifluormethyl)-2H-1,4-benzodiazepin-2,5-(1H)-dion vom Schmelzpunkt 203—206°.

c) Eine Lösung von 2,36 g (9,1 mMol) 3,4-Dihydro-4-methyl-7-(trifluormethyl)-2H-1,4-benzodiazepin-2,5(1H)-dion in 10 ml trockenem Dimethylformamid wird bei 5° mit 1,22 g (10,9 mMol) Kalium-t-butylat und bei —40° tropfenweise mit 1,4 ml (9,5 mMol) Diäthylchlorphosphat versetzt. Separat wird eine Lösung von 1,12 g (10 mMol) Kalium-t-butylat in 5 ml trockenem Dimethylformamid unter Kühlen in einem Aceton/Trockeneis-Bad mit 1,2 ml (10 mMol) Isocyanessigsäureäthylester versetzt. Die so erhaltene orangefarbene Lösung wird nun bei —20 bis —10° zum obigen Reaktionsgemisch getropft. Man entfernt das Kühlbad, neutralisiert nach ca. 15 Minuten mit Eisessig und gießt das Reaktionsgemisch auf 80 ml Eiswasser. Man extrahiert dreimal mit je 60 ml Chloroform, wäscht die vereinigten Chloroform-Auszüge dreimal mit je 60 ml Wasser, trocknet diese über Magnesiumsulfat und dampft ein. Durch Säulenchromatographie und Umkristallisieren des erhaltenen Rohproduktes aus Essigester erhält man Äthyl-5,6-dihydro-5-methyl-6-oxo-8-(trifluormethyl)-4H-imidazo[1,5-a][1,4]benzodiazepin-3-carboxylat vom Schmelzpunkt 179—180°.

## Beispiel A

Es werden Tabletten folgender Zusammensetzung hergestellt:

| | pro Tablette |
|---|---|
| Äthyl-(S)-11,12,13,13a-tetrahydro-9-oxo-9H-imidazo[1,5-a]pyrrolo[2,1-c][1,4]benzodiazepin-1-carboxylat | 10 mg |
| Milchzucker | 90 mg |
| Maisstärke | 29 mg |
| Mikrokristalline Cellulose | 70 mg |
| Magnesiumstearat | 1 mg |
| Total | 200 mg |

## Beispiel B

Es werden Kapseln folgender Zusammensetzungen hergestellt:

| | pro Kapsel |
|---|---|
| Äthyl-(S)-11,12,13,13a-tetrahydro-9-oxo-9H-imidazo[1,5-a]pyrrolo[2,1-c][1,4]benzodiazepin-1-carboxylat | 10 mg |
| Milchzucker | 165 mg |
| Maisstärke | 30 mg |
| Talk | 5 mg |
| Total | 210 mg |

Äthyl-(S)-11,12,13,13a-tetrahydro-9-oxo-9H-imidazo[1,5-a]pyrrolo[2,1-c][1,4]benzodiazepin-1-carboxylat, Milchzucker und Maisstärke werden zunächst in einem Mischer und dann in einer Zerkleinerungsmaschine vermengt. Man bringt das Gemisch wieder in den Mischer zurück, gibt den Talk zu und vermengt gründlich. Das Gemisch wird maschinell in Hartgelatinekapseln abgefüllt.

## Beispiel C

Es werden Injektionslösungen folgender Zusammensetzung hergestellt:

| | pro ml |
|---|---|
| Äthyl-(S)-11,12,13,13a-tetrahydro-9-oxo-9H-imidazo[1,5-a]pyrrolo[2,1-c][1,4]benzodiazepin-1-carboxylat | 5,0 mg |
| Benzylalkohol | 0,015 ml |
| Propylenglykol | 0,4 ml |
| Äthanol (95%ig) | 0,1 ml |
| Natriumbenzoat | 48,8 mg |
| Benzoesäure | 1,2 mg |
| Wasser für Injektion q.s. ad | 1,0 ml |

Zur Herstellung von 10 000 ml Injektionslösung löst man 50 g Äthyl-(S)-11,12,13,13a-tetrahydro-9-oxo-9H-imidazo[1,5-a]pyrrolo[2,1-c][1,4]benzodiazepin-1-carboxylat in 150 ml Benzylalkohol und gibt 4000 ml Propylenglykol und 1000 ml Äthanol zu. Dann löst man 12 g Benzoesäure in dem obigen Gemisch und gibt eine Lösung von 488 g Natriumbenzoat in 300 ml Wasser (für Injektion) zu. Die erhaltene Lösung wird durch Zugabe von Wasser (für Injektion) auf ein Volumen von 10 000 ml gebracht, filtriert und in Ampullen geeigneter Größe abgefüllt; man füllt das Restvolumen der Ampullen mit Stickstoff, schmilzt die Ampullen zu und sterilisiert sie während 30 Minuten im Autoklaven bei

0,7 Atm.

Beispiel D

Es werden Suppositorien folgender Zusammensetzung hergestellt:

| | | pro Supp. |
|---|---|---|
| Äthyl-(S)-11,12,13,13a-tetrahydro-9-oxo-9H-imidazo[1,5-a]pyrrolo[2,1-c][1,4]benzodiazepin-1-carboxylat | | 0,010 g |
| Cacaobutter (Smp. 36—37°) | | 1,245 g |
| Carnauba-Wachs | | 0,045 g |
| | Total | 1,3 g |

Cacaobutter und Carnauba-Wachs werden in einem Glas- oder Stahlgefäß geschmolzen, gründlich vermengt und auf 45° abgekühlt. Hierauf gibt man fein pulverisiertes Äthyl-(S)-11,12,13,13a-tetrahydro-9-oxo-9H-imidazo[1,5-a]pyrrolo[2,1-c][1,4]benzodiazepin-1-carboxylat zu und rührt, bis es vollständig dispergiert ist. Man gießt die Mischung in Suppositorienformen geeigneter Größe, läßt abkühlen, nimmt dann die Suppositorien aus den Formen und verpackt sie einzeln in Wachspapier oder Metallfolien.

Beispiel E

Es werden Kapseln folgender Zusammensetzung hergestellt:

| | | mg/Kapsel |
|---|---|---|
| (+)-5-(o-Chlorphenyl)-1,3-dihydro-3-methyl-7-nitro-2H-1,4-benzodiazepin-2-on | | 10,0 |
| Äthyl-8-fluor-5,6-dihydro-5-methyl-6-oxo-4H-imidazo[1,5-a][1,4]benzodiazepin-3-carboxylat | | 100,0 |
| Milchzucker krist. | | 100,0 |
| Maisstärke weiß | | 27,5 |
| Talk | | 10,0 |
| Magnesiumstearat | | 2,5 |
| | Total | 250,0 |

Die beiden Wirkstoffe werden mit den Hilfsstoffen gut vermischt und zu je 250,0 mg in Steckkapseln geeigneter Größe abgefüllt.

Beispiel F

Es werden Tabletten folgender Zusammensetzung hergestellt:

| | | mg/Tablette |
|---|---|---|
| (+)-5-(o-Chlorphenyl)-1,3-dihydro-3-methyl-7-nitro-2H-1,4-benzodiazepin-2-on | | 30,0 |
| Äthyl-8-fluor-5,6-dihydro-5-methyl-6-oxo-4H-imidazo[1,5-a][1,4]benzodiazepin-3-carboxylat | | 30,0 |
| Milchzucker pulvis | | 15,0 |
| Maisstärke weiß | | 19,5 |
| Povidon K30 | | 3,5 |
| Maisstärke weiß | | 10,0 |
| Magnesiumstearat | | 2,0 |
| | Total | 110,0 |

Die beiden Wirkstoffe, Milchzucker pulvis und die erste Portion Maisstärke weiß werden gemischt und und gesiebt. Diese Mischung wird mit einer Lösung von Povidon K30 in Wasser befeuchtet, geknetet, granuliert, getrocknet und gesiebt. Dem Granulat werden die zweite Portion Maisstärke weiß und Magnesiumstearat zugesetzt. Nach Mischen wird die erhaltene Masse zu 110 mg schweren Tabletten verpreßt.

Beispiel G

Es werden Tabletten folgender Zusammensetzung hergestellt:

| | mg/Tablette |
|---|---|
| (+)-5-(o-Chlorphenyl)-1,3-dihydro-3-methyl-7-nitro-2H-1,4-benzodiazepin-2-on | 30 |
| Äthyl-8-fluor-5,6-dihydro-5-methyl-6-oxo-4H-imidazo[1,5-a][1,4]benzodiazepin-3-carboxylat | 100 |
| Milchzucker pulvis | 22 |
| Maisstärke weiß | 22 |
| Povidon K30 | 6 |
| Maisstärke weiß | 16 |
| Magnesiumstearat | 4 |
| Total | 200 |

Die beiden Wirkstoffe, Milchzucker pulvis und die erste Portion Maisstärke weiß werden gemischt und gesiebt. Diese Mischung wird mit einer Lösung von Povidon K30 in Wasser befeuchtet, geknetet, granuliert, getrocknet und gesiebt. Dem Granulat werden die zweite Portion Maisstärke weiß und Magnesiumstearat zugesetzt. Nach Mischen wird die erhaltene Masse zu 200 mg schweren Tabletten verpreßt.

**Patentansprüche für die Vertragsstaaten: BE, CH, DE, FR, IT, LI, LU, NL, SE**

1. Imidazodiazepin-Derivate der allgemeinen Formel

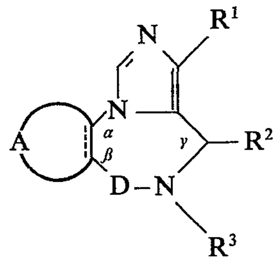

(I)

worin A zusammen mit den beiden mit $\alpha$ und $\beta$ bezeichneten Kohlenstoffatomen eine der Gruppen

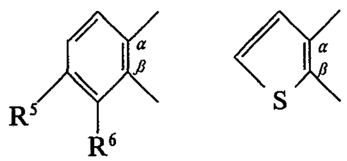

und

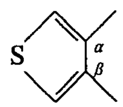

(a) (b) (c)

und die gestrichelte Linie die in Fällen (a) und (b) vorliegende Doppelbindung bedeuten, D $>C=O$ oder $>C=S$, $R^1$ Cyano, $(C_{2-7})$-Alkanoyl oder einen Rest der Formel $-COOR^4$, $R^4$ Methyl, Äthyl, Isopropyl oder 2-Hydroxyäthyl, $R^5$ Wasserstoff, Trifluormethyl oder Halogen und $R^6$ Wasserstoff, Trifluormethyl, Halogen oder $(C_{1-7})$-Alkyl bedeuten und entweder $R^2$ Wasserstoff und $R^3$ Wasserstoff oder $(C_{1-7})$-Alkyl bedeuten oder $R^2$ und $R^3$ zusammen Trimethylen oder Propenylen bedeuten und das mit $\gamma$ bezeichnete Kohlenstoffatom die S- oder R,S-Konfiguration aufweist,

und pharmazeutisch annehmbare Säureadditionssalze davon.

2. Verbindungen gemäß Anspruch 1, dadurch gekennzeichnet, daß A zusammen mit den beiden mit $\alpha$ und $\beta$ bezeichneten Kohlenstoffatomen die in Anspruch 1 definierte Gruppe (a) und die gestrichelte Linie die in diesem Fall vorliegende Doppelbindung bedeuten, D $>C=O$ oder $>C=S$, $R^1$ einen Rest der Formel $-COOR^4$, $R^2$ Wasserstoff, $R^3$ Wasserstoff oder $(C_{1-7})$-Alkyl, $R^4$ Methyl, Äthyl oder Isopropyl und $R^5$ und $R^6$ beide Wasserstoff bedeuten.

3. Verbindungen gemäß Anspruch 1, dadurch gekennzeichnet, daß A zusammen mit den beiden mit $\alpha$ und $\beta$ bezeichneten Kohlenstoffatomen die in Anspruch 1 definierte Gruppe (a) und die gestrichelte Linie die in diesem Fall vorliegende Doppelbindung bedeuten, D $>C=O$ oder $>C=S$, $R^1$ einen Rest der Formel $-COOR^4$ bedeuten, $R^2$ und $R^3$ zusammen Trimethylen bedeuten und das mit $\gamma$ bezeichnete Kohlenstoffatom die S-Konfiguration aufweist, $R^4$ Methyl, Äthyl oder Isopropyl und $R^5$ und $R^6$ beide Wasserstoff bedeuten.

4. Verbindungen gemäß Anspruch 1, dadurch gekennzeichnet, daß A zusammen mit den beiden mit $\alpha$ und $\beta$ bezeichneten Kohlenstoffatomen die in Anspruch 1 definierte Gruppe (a) und die gestrichelte Linie die in diesem Fall vorliegende Doppelbindung bedeuten, D $>C=O$ oder $>C=S$, $R^1$ einen Rest der Formel $-COOR^4$, $R^2$ Wasserstoff, $R^3$ Wasserstoff oder ($C_{1-7}$)-Alkyl, $R^4$ Methyl, Äthyl oder Isopropyl, $R^5$ Halogen und $R^6$ Wasserstoff bedeuten.

5. Verbindungen gemäß Anspruch 1, dadurch gekennzeichnet, daß A zusammen mit den beiden mit $\alpha$ und $\beta$ bezeichneten Kohlenstoffatomen die in Anspruch 1 definierte Gruppe (a) und die gestrichelte Linie die in diesem Fall vorliegende Doppelbindung bedeuten, D $>C=O$ oder $>C=S$, $R^1$ einen Rest der Formel $-COOR^4$ bedeuten, $R^2$ und $R^3$ zusammen Trimethylen bedeuten und das mit $\gamma$ bezeichnete Kohlenstoffatom die S-Konfiguration aufweist, $R^4$ Methyl, Äthyl oder Isopropyl, $R^5$ Fluor und $R^6$ Wasserstoff bedeuten.

6. Verbindungen gemäß Anspruch 1, dadurch gekennzeichnet, daß $R^1$ Cyano oder einen Rest der Formel $-COOR^4$ bedeutet, wobei $R^4$ Methyl, Äthyl oder Isopropyl bedeutet.

7. Verbindungen gemäß Anspruch 1 oder 6, dadurch gekennzeichnet, daß $R^3$ Methyl bedeutet, wenn $R^2$ Wasserstoff bedeutet.

8. Verbindungen gemäß Anspruch 1 oder 6, dadurch gekennzeichnet, daß das in Formel I mit $\gamma$ bezeichnete Kohlenstoffatom die S-Konfiguration aufweist, wenn $R^2$ und $R^3$ zusammen Trimethylen bedeuten.

9. Verbindungen gemäß einem der Ansprüche 1 und 6 bis 8, dadurch gekennzeichnet, daß A zusammen mit den beiden mit $\alpha$ und $\beta$ bezeichneten Kohlenstoffatomen die in Anspruch 1 definierte Gruppe (a) und die gestrichelte Linie die in diesem Fall vorliegende Doppelbindung bedeuten, $R^5$ Wasserstoff oder Fluor und $R^6$ Wasserstoff, Fluor, Chlor oder Methyl bedeuten, wobei mindestens eines von $R^5$ und $R^6$ Wasserstoff bedeutet.

10. Äthyl-8-fluor-5,6-dihydro-5-methyl-6-oxo-4H-imidazo[1,5-a][1,4]benzodiazepin-3-carboxylat.

11. Äthyl-7-fluor-5,6-dihydro-5-methyl-6-oxo-4H-imidazo[1,5-a][1,4]benzodiazepin-3-carboxylat.

12. Äthyl-7-chlor-5,6-dihydro-5-methyl-6-oxo-4H-imidazo[1,5-a][1,4]benzodiazepin-3-carboxylat.

13. Äthyl-(S)-8-chlor-11,12,13,13a-tetrahydro-9-oxo-9H-imidazo[1,5-a]pyrrolo[2,1-c][1,4]benzodiazepin-1-carboxylat.

14. Äthyl-(R,S)-11,13a-dihydro-9-oxo-9H-imidazo[1,5-a]pyrrolo[2,1-c][1,4]benzodiazepin-1-carboxylat.

15. Äthyl-8-fluor-11,13a-dihydro-9-oxo-9H-imidazo[1,5-a]pyrrolo[2,1-c][1,4]benzodiazepin-1-carboxylat.

16. Äthyl-5,6-dihydro-5-methyl-6-oxo-4H-imidazo[1,5-a][1,4]benzodiazepin-3-carboxylat, Methyl-5,6-dihydro-5-methyl-6-oxo-4H-imidazo[1,5-a][1,4]benzodiazepin-3-carboxylat, Isopropyl-5,6-dihydro-5-methyl-6-oxo-4H-imidazo[1,5-a][1,4]benzodiazepin-3-carboxylat, Methyl-8-fluor-5,6-dihydro-5-methyl-6-oxo-4H-imidazo[1,5-a][1,4]benzodiazepin-3-carboxylat, Isopropyl-8-fluor-5,6-dihydro-5-methyl-6-oxo-4H-imidazo[1,5-a][1,4]benzodiazepin-3-carboxylat und Äthyl-5,6-dihydro-5,7-dimethyl-6-oxo-4H-imidazo[1,5-a][1,4]benzodiazepin-3-carboxylat.

17. Äthyl-(S)-11,12,13,13a-tetrahydro-9-oxo-9H-imidazo[1,5-a]pyrolo[2,1-c][1,4]benzodiazepin-1-carboxylat, Methyl-(S)-11,12,13,13a-tetrahydro-9-oxo-9H-imidazo[1,5-a]pyrrolo[2,1-c][1,4]benzodiazepin-1-carboxylat, Isopropyl-(S)-11,12,13,13a-tetrahydro-9-oxo-9H-imidazo[1,5-a]pyrrolo[2,1-c][1,4]benzodiazepin-1-carboxylat, Äthyl-(S)-7-fluor-11,12,13,13a-tetrahydro-9-oxo-9H-imidazo[1,5-a]pyrrolo[2,1-c][1,4]benzodiazepin-1-carboxylat und Äthyl-(S)-11,12,13,13a-tetrahydro-8-methyl-9-oxo-9H-imidazo[1,5-a]pyrrolo-[2,1-c][1,4]benzodiazepin-1-carboxylat.

18. Äthyl-5,6-dihydro-5-methyl-6-thioxo-4H-imidazo[1,5-a][1,4]benzodiazepin-3-carboxylat und Äthyl-8-fluor-5,6-dihydro-5-methyl-6-thioxo-4H-imidazo[1,5-a][1,4]benzodiazepin-3-carboxylat.

19. 8-Fluor-5,6-dihydro-5-methyl-6-oxo-4H-imidazo[1,5-a][1,4]benzodiazepin-3-carbonitril, (S)-11,12,13,13a-Tetrahydro-9-oxo-9H-imidazo[1,5-a]pyrrolo[2,1-c][1,4]benzodiazepin-1-carbonitril und 5,6-Dihydro-5-methyl-6-oxo-4H-imidazo[1,5-a][1,4]benzodiazepin-3-carbonitril.

20. Methyl-(S)-10,11,12,12a-tetrahydro-8-oxo-8H-imidazo[5,1-c]pyrrolo[1,2-a]thieno[3,2-e][1,4]diazepin-1-carboxylat, Äthyl-(S)-10,11,12,12a-tetrahydro-8-oxo-8H-imidazo[5,1-c]pyrrolo[1,2-a]thieno[3,2-e][1,4]diazepin-1-carboxylat, Äthyl-5,6-dihydro-5-methyl-6-oxo-4H-imidazo[1,5-a]thieno[2,3-f][1,4]diazepin-3-carboxylat und Methyl-5,6-dihydro-5-methyl-6-oxo-4H-imidazo[1,5-a]thieno[2,3-f][1,4]diazepin-3-carboxylat.

21. Verbindungen der allgemeinen Formel

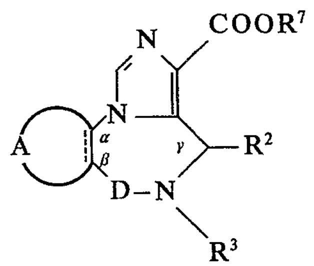

(XI)

worin A, die gestrichelte Linie, D, $R^2$ und $R^3$ die in Anspruch 1 angegebene Bedeutung besitzen, und $R^7$ $(C_{1-7})$-Alkyl bedeutet, mit der Maßgabe, daß $R^7$ nicht Methyl, Äthyl oder Isopropyl bedeutet.

22. Verbindungen gemäß Anspruch 21, dadurch gekennzeichnet, daß D $>C=O$, $R^2$ Wasserstoff und $R^3$ Methyl bedeuten, A zusammen mit den beiden mit $\alpha$ und $\beta$ bezeichneten Kohlenstoffatomen die in Anspruch 1 definierte Gruppe (a) und die gestrichelte Linie die in diesem Fall vorliegende Doppelbindung bedeuten, wobei $R^5$ Fluor und $R^6$ Wasserstoff bedeuten.

23. Verbindungen gemäß einem der Ansprüche 1—20 und pharmazeutisch annehmbare Säureadditionssalze davon als pharmazeutische Wirkstoffe.

24. Verbindungen gemäß einem der Ansprüche 1—20 und pharmazeutisch annehmbare Säureadditionssalze davon als die zentral dämpfenden, muskelrelaxierenden, ataktischen, blutdrucksenkenden und atemdepressiven Eigenschaften von tranquilisierend wirksamen 1,4-Benzodiazepinen antagonisierende Wirkstoffe.

25. Äthyl-8-fluor-5,6-dihydro-5-methyl-6-oxo-4H-imidazo[1,5-a][1,4]benzodiazepin-3-carboxylat und pharmazeutisch annehmbare Säureadditionssalze davon als pharmazeutische Wirkstoffe.

26. Äthyl-8-fluor-5,6-dihydro-5-methyl-6-oxo-4H-imidazo[1,5-a][1,4]benzodiazepin-3-carboxylat und pharmazeutisch annehmbare Säureadditionssalze davon als die zentral dämpfenden, muskelrelaxierenden, ataktischen, blutdrucksenkenden und atemdepressiven Eigenschaften von tranquilisierend wirksamen 1,4-Benzodiazepinen antagonisierende Wirkstoffe.

27. Äthyl-7-chlor-5,6-dihydro-5-methyl-6-oxo-4H-imidazo[1,5-a][1,4]benzodiazepin-3-carboxylat und pharmazeutisch annehmbare Säureadditionssalze davon als pharmazeutische Wirkstoffe.

28. Äthyl-7-chlor-5,6-dihydro-5-methyl-6-oxo-4H-imidazo[1,5-a][1,4]benzodiazepin-3-carboxylat und pharmazeutisch annehmbare Säureadditionssalze davon als die zentral dämpfenden, muskelrelaxierenden, ataktischen, blutdrucksenkenden und atemdepressiven Eigenschaften von tranquilisierend wirksamen 1,4-Benzodiazepinen antagonisierende Wirkstoffe.

29. Verfahren zur Herstellung von Verbindungen gemäß einem der Ansprüche 1—20 und von pharmazeutisch annehmbaren Säureadditionssalzen davon, dadurch gekennzeichnet, daß man

a) eine Verbindung der allgemeinen Formel

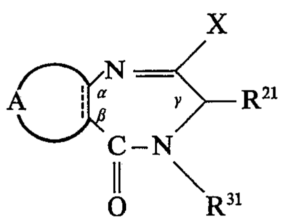

(II)

worin A und die gestrichelte Linie die in Anspruch 1 angegebene Bedeutung besitzen und X eine Abgangsgruppe bedeutet, und entweder $R^{21}$ Wasserstoff und $R^{31}$ $(C_{1-7})$-Alkyl bedeuten, oder $R^{21}$ und $R^{31}$ zusammen Trimethylen oder Propenylen bedeuten, und das mit $\gamma$ bezeichnete Kohlenstoffatom die S- oder R,S-Konfiguration aufweist,

in Gegenwart einer Base mit einem Isocyanessigester der allgemeinen Formel

$$CN-CH_2-COOR^{41} \quad (III)$$

worin $R^{41}$ Methyl, Äthyl oder Isopropyl bedeutet, umsetzt, oder

b) eine Verbindung der allgemeinen Formel

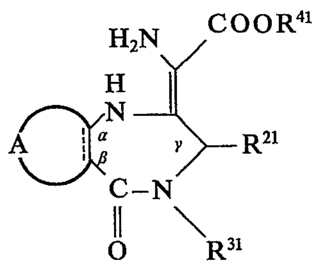

(IV)

worin A und die gestrichelte Linie die in Anspruch 1 angegebene und $R^{21}$, $R^{31}$ und $R^{41}$ obige Bedeutung besitzen,
mit einem Formylierungsmittel behandelt, oder

c) eine Verbindung der allgemeinen Formel

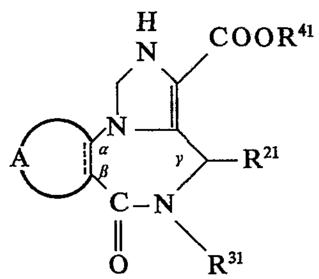

(V)

oder

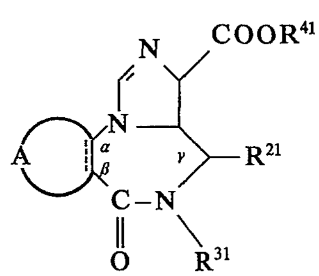

(VI)

worin A und die gestrichelte Linie die in Anspruch 1 angegebene und $R^{21}$, $R^{31}$ und $R^{41}$ obige Bedeutung besitzen,
dehydriert, oder

d) in einer Verbindung der allgemeinen Formel

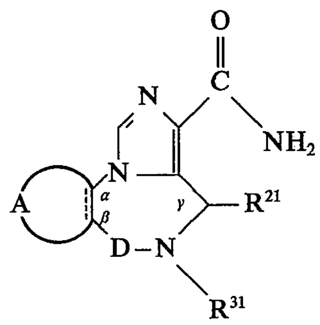

(VII)

worin A, die gestrichelte Linie und D die in Anspruch 1 angegebene und $R^{21}$ und $R^{31}$ obige Bedeutung besitzen,
die Carboxamido-Gruppe in die Nitril-Gruppe umwandelt, oder

e) eine Verbindung der allgemeinen Formel

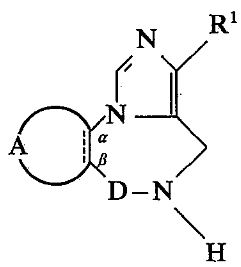

(Ia)

worin A, die gestrichelte Linie, D und $R^1$ die in Anspruch 1 angegebene Bedeutung besitzen,
an der sekundären Aminogruppe ensprechend substituiert, oder

f) in einer Verbindung der allgemeinen Formel

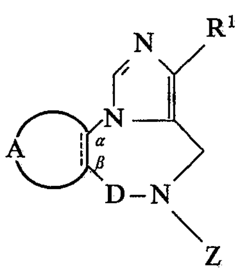

(VIII)

worin A, die gestrichelte Linie, D und $R^1$ die in Anspruch 1 angegebene Bedeutung besitzen, und Z eine Schutzgruppe bedeutet,
die Schutzgruppe abspaltet, oder

g) in einer Verbindung der allgemeinen Formel

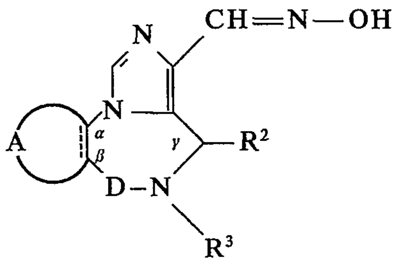

(IX)

worin A, die gestrichelte Linie, D, $R^2$ und $R^3$ die in Anspruch 1 angegebene Bedeutung besitzen, die Oxim-Gruppe in die Nitril-Gruppe überführt, oder

h) in einer Verbindung der allgemeinen Formel

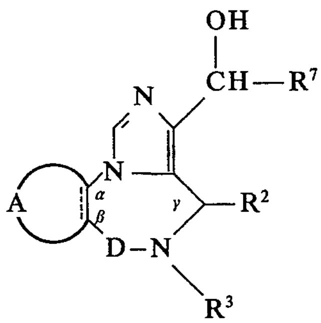

(X)

worin A, die gestrichelte Linie, D, $R^2$ und $R^3$ die in Anspruch 1 angegebene Bedeutung besitzen, und $R^7$ $(C_{1-6})$-Alkyl bedeutet,
die Hydroxyl-Gruppe in die Keto-Gruppe überführt, oder

i) eine Verbindung der allgemeinen Formel

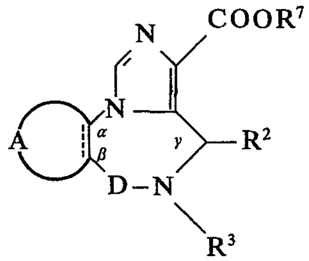

(XI)

worin A, die gestrichelte Linie, D, $R^2$ und $R^3$ die in Anspruch 1 angegebene Bedeutung besitzen, und $R^7$ $(C_{1-7})$-Alkyl bedeutet,
umestert, oder

j) in einer Verbindung der allgemeinen Formel

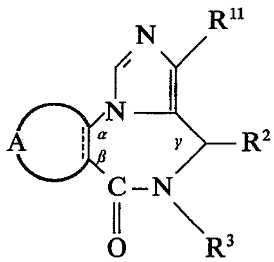

(Ib)

worin $R^{11}$ Cyano oder einen Rest der Formel $-COOR^4$ bedeutet, und A, die gestrichelte Linie, $R^2$, $R^3$ und $R^4$ die in Anspruch 1 angegebene Bedeutung besitzen,
die Carbonyl-Gruppe in die Thiocarbonyl-Gruppe überführt, und

k) erwünschtenfalls eine Verbindung der allgemeinen Formel I in ein pharmazeutisch annehmbares Säureadditionssalz überführt.

30. Verfahren gemäß Anspruch 29, dadurch gekennzeichnet, daß man Äthyl-8-fluor-5,6-dihydro-5-methyl-6-oxo-4H-imidazo[1,5-a][1,4]benzodiazepin-3-carboxylat herstellt.

31. Verfahren gemäß Anspruch 29, dadurch gekennzeichnet, daß man Äthyl-7-chlor-5,6-dihydro-5-methyl-6-oxo-4H-imidazo[1,5-a][1,4]benzodiazepin-3-carboxylat herstellt.

32. Arzneimittel, enthaltend eine Verbindung gemäß einem der Ansprüche 1 bis 20 oder pharmazeutisch annehmbare Säureadditionssalze davon.

33. Arzneimittel, welche die zentral dämpfenden, muskelrelaxierenden, ataktischen, blutdrucksenkenden und atemdepressiven Eigenschaften von tranquilisierend wirksamen 1,4-Benzodiazepinen antagonisieren, enthaltend eine Verbindung gemäß einem der Ansprüche 1 bis 20 oder pharmazeutisch annehmbare Säureadditionssalze davon.

34. Arzneimittel, enthaltend Äthyl-8-fluor-5,6-dihydro-5-methyl-6-oxo-4H-imidazo[1,5-a][1,4]benzodiazepin-3-carboxylat oder pharmazeutisch annehmbare Säureadditionssalze davon.

35. Arzneimittel, enthaltend Äthyl-7-chlor-5,6-dihydro-5-methyl-6-oxo-4H-imidazo[1,5-a][1,4]benzodiazepin-3-carboxylat oder pharmazeutisch annehmbare Säureadditionssalze davon.

36. Arzneimittel, welche die zentral dämpfenden, muskelrelaxierenden, ataktischen, blutdrucksenkenden und atemdepressiven Eigenschaften von tranquilisierend wirksamen 1,4-Benzodiazepinen antagonisieren, enthaltend Äthyl-8-fluor-5,6-dihydro-5-methyl-6-oxo-4H-imidazo[1,5-a][1,4]benzodiazepin-3-carboxylat oder pharmazeutisch annehmbare Säureadditionssalze davon.

37. Arzneimittel, welche die zentral dämpfenden, muskelrelaxierenden, ataktischen, blutdrucksenkenden und atemdepressiven Eigenschaften von tranquilisierend wirksamen 1,4-Benzodiazepinen antagonisieren, enthaltend Äthyl-7-chlor-5,6-dihydro-5-methyl-6-oxo-4H-imidazo[1,5-a][1,4]benzodiazepin-3-carboxylat oder pharmazeutisch annehmbare Säureadditionssalze davon.

38. Schistosomiazid wirksames Arzneimittel, enthaltend eine Verbindung gemäß einem der Ansprüche 1 bis 20 oder ein pharmazeutisch annehmbares Säureadditionssalz davon und eine Verbindung der

allgemeinen Formel

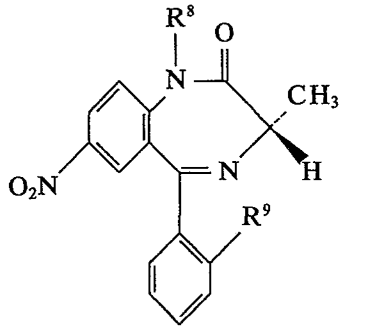

(XXII)

worin $R^8$ Wasserstoff oder $(C_{1-7})$-Alkyl und $R^9$ Halogen bedeuten, und/oder eine Verbindung der allgemeinen Formel

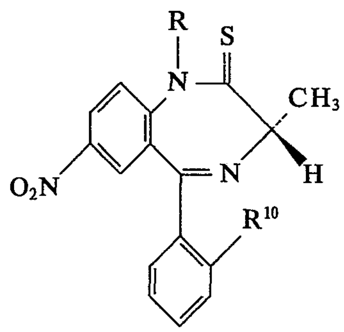

(XXIII)

worin R Wasserstoff oder $(C_{1-7})$-Alkyl und $R^{10}$ Wasserstoff, Halogen oder Trifluormethyl bedeuten.

39. Schistosomiazid wirksames Arzneimittel gemäß Anspruch 38, dadurch gekennzeichnet, daß es Äthyl-8-fluor-5,6-dihydro-5-methyl-6-oxo-4H-imidazo[1,5-a][1,4]benzodiazepin-3-carboxylat oder ein pharmazeutisch annehmbares Säureadditionssalz davon enthält.

40. Schistosomiazid wirksames Arzneimittel gemäß Anspruch 38, dadurch gekennzeichnet, daß es Äthyl-7-chlor-5,6-dihydro-5-methyl-6-oxo-4H-imidazo[1,5-a][1,4]benzodiazepin-3-carboxylat oder ein pharmazeutisch annehmbares Säureadditionssalz davon enthält.

41. Schistosomiazid wirksames Arzneimittel gemäß Anspruch 38, dadurch gekennzeichnet, daß es (+)-5-(o-Chlorphenyl)-1,3-dihydro-3-methyl-7-nitro-2H-1,4-benzodiazepin-2-on enthält.

42. Schistosomiazid wirksames Arzneimittel, enthaltend Äthyl-8-fluor-5,6-dihydro-5-methyl-6-oxo-4H-imidazo[1,5-a][1,4]benzodiazepin-3-carboxylat oder ein pharmazeutisch annehmbares Säureadditionssalz davon und (+)-5-(o-Chlorphenyl)-1,3-dihydro-3-methyl-7-nitro-2H-1,4-benzodiazepin-2-on.

43. Schistosomiazid wirksames Arzneimittel, enthaltend Äthyl-7-chlor-5,6-dihydro-5-methyl-6-oxo-4H-imidazo[1,5-a][1,4]benzodiazepin-3-carboxylat oder ein pharmazeutisch annehmbares Säureadditionssalz davon und (+)-5-(o-Chlorphenyl)-1,3-dihydro-3-methyl-7-nitro-2H-1,4-benzodiazepin-2-on.

**Patentansprüche für den Vertragsstaat: AT**

1. Verfahren zur Herstellung von Imidazodiazepin-Derivaten der allgemeinen Formel

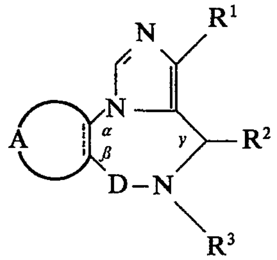

(I)

worin A zusammen mit den beiden mit $\alpha$ und $\beta$ bezeichneten Kohlenstoffatomen eine der Gruppen

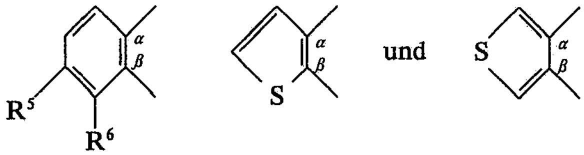

(a) (b) und (c)

und die gestrichelte Linie die in Fällen (a) und (b) vorliegende Doppelbindung bedeuten, D $>C=O$ oder $>C=S$, $R^1$ Cyano, $(C_{2-7})$-Alkanoyl oder einen Rest der Formel $-COOR^4$, $R^4$ Methyl, Äthyl, Isopropyl oder 2-Hydroxyäthyl, $R^5$ Wasserstoff, Trifluormethyl oder Halogen und $R^6$ Wasserstoff, Trifluormethyl, Halogen oder $(C_{1-7})$-Alkyl bedeuten und entweder $R^2$ Wasserstoff und $R^3$ Wasserstoff oder $(C_{1-7})$-Alkyl bedeuten oder $R^2$ und $R^3$ zusammen Trimethylen oder Propenylen bedeuten und das mit $\gamma$ bezeichnete Kohlenstoffatom die S- oder R,S-Konfiguration aufweist,

und von pharmazeutisch annehmbaren Säureadditionssalzen davon, dadurch gekennzeichnet, daß man

a) eine Verbindung der allgemeinen Formel

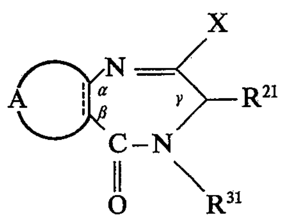

(II)

worin A und die gestrichelte Linie obige Bedeutung besitzen und X eine Abgangsgruppe bedeutet, und entweder $R^{21}$ Wasserstoff und $R^{31}$ $(C_{1-7})$-Alkyl bedeuten, oder $R^{21}$ und $R^{31}$ zusammen Trimethylen oder Propenylen bedeuten, und das mit $\gamma$ bezeichnete Kohlenstoffatom die S- oder R,S-Konfiguration aufweist,

in Gegenwart einer Base mit einem Isocyanessigester der allgemeinen Formel

$$CN-CH_2-COOR^{41} \quad (III)$$

worin $R^{41}$ Methyl, Äthyl oder Isopropyl bedeutet,

umsetzt, oder

b) eine Verbindung der allgemeinen Formel

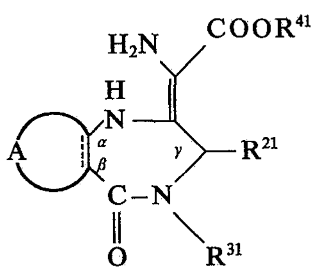

(IV)

worin A, die gestrichelte Linie, $R^{21}$, $R^{31}$ und $R^{41}$ obige Bedeutung besitzen,

mit einem Formylierungsmittel behandelt, oder

c) eine Verbindung der allgemeinen Formel

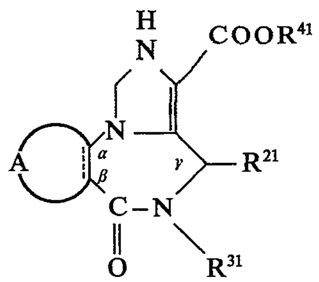

(V)

oder

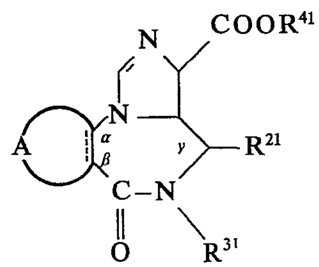

(VI)

worin A, die gestrichelte Linie, $R^{21}$, $R^{31}$ und $R^{41}$ obige Bedeutung besitzen, dehydriert, oder

d) in einer Verbindung der allgemeinen Formel

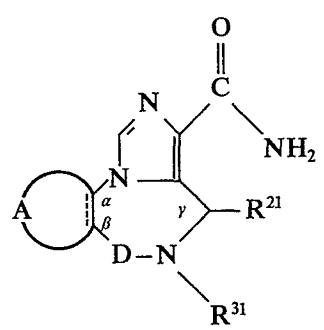

(VII)

worin A, die gestrichelte Linie, D, $R^{21}$ und $R^{31}$ obige Bedeutung besitzen, die Carboxamido-Gruppe in die Nitril-Gruppe umwandelt, oder

e) eine Verbindung der allgemeinen Formel

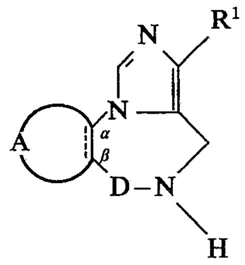

(Ia)

worin A, die gestrichelte Linie, D und $R^1$ obige Bedeutung besitzen, an der sekundären Aminogruppe entsprechend substituiert, oder

f) in einer Verbindung der allgemeinen Formel

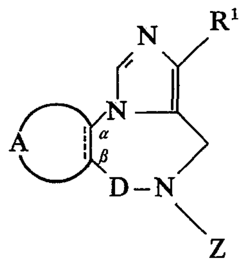

(VIII)

worin A, die gestrichelte Linie, D und $R^1$ obige Bedeutung besitzen, und Z eine Schutzgruppe bedeutet, die Schutzgruppe abspaltet, oder

g) in einer Verbindung der allgemeinen Formel

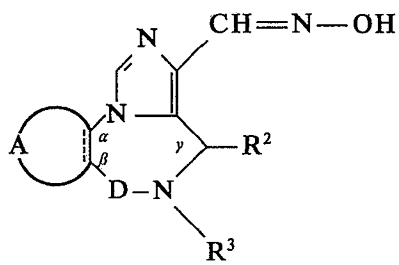

(IX)

worin A, die gestrichelte Linie, D, $R^2$ und $R^3$ obige Bedeutung besitzen, die Oxim-Gruppe in die Nitril-Gruppe überführt, oder

h) in einer Verbindung der allgemeinen Formel

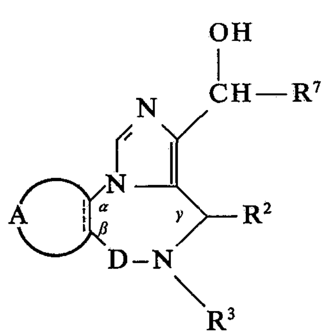

(X)

worin A, die gestrichelte Linie, D, $R^2$ und $R^3$ obige Bedeutung besitzen, und $R^7$ $(C_{1-6})$-Alkyl bedeutet, die Hydroxyl-Gruppe in die Keto-Gruppe überführt, oder

i) eine Verbindung der allgemeinen Formel

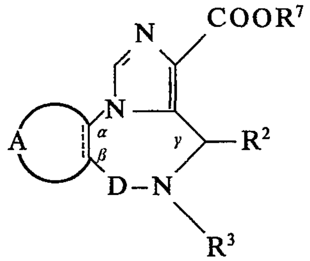

(XI)

worin A, die gestrichelte Linie, D, $R^2$ und $R^3$ obige Bedeutung besitzen, und $R^7$ $(C_{1-7})$-Alkyl bedeutet,

umestert, oder

j) in einer Verbindung der allgemeinen Formel

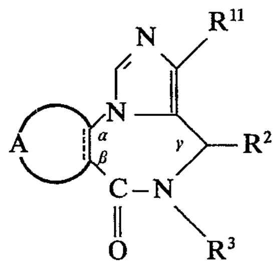

(Ib)

worin $R^{11}$ Cyano oder einen Rest der Formel $-COOR^4$ bedeutet, und A, die gestrichelte Linie, $R^2$, $R^3$ und $R^4$ obige Bedeutung besitzen,

die Carbonyl-Gruppe in die Thiocarbonyl-Gruppe überführt, und

k) erwünschtenfalls eine Verbindung der allgemeinen Formel I in ein pharmazeutisch annehmbares Säureadditionssalz überführt.

2.Verfahren gemäß Anspruch 1, dadurch gekennzeichnet, daß man Verbindungen der in Anspruch 1 definierten allgemeinen Formel I, worin A zusammen mit den beiden mit $\alpha$ und $\beta$ bezeichneten Kohlenstoffatomen die in Anspruch 1 definierte Gruppe a) und die gestrichelte Linie die in diesem Fall vorliegende Doppelbindung bedeuten, D $>C=O$ oder $>C=S$, $R^1$ einen Rest der Formel $-COOR^4$, $R^2$ Wasserstoff, $R^3$ Wasserstoff oder $(C_{1-7})$-Alkyl, $R^4$ Methyl, Äthyl oder Isopropyl und $R^5$ und $R^6$ beide Wasserstoff bedeuten, herstellt.

3. Verfahren gemäß Anspruch 1, dadurch gekennzeichnet, daß man Verbindungen der in Anspruch 1 definierten allgemeinen Formel I, worin A zusammen mit den beiden mit $\alpha$ und $\beta$ bezeichneten Kohlenstoffatomen die in Anspruch 1 definierte Gruppe a) und die gestrichelte Linie die in diesem Fall vorliegende Doppelbindung bedeutet, D $>C=O$ oder $>C=S$, $R^1$ einen Rest der Formel $-COOR^4$ bedeuten, $R^2$ und $R^3$ zusammen Trimethylen bedeuten und das mit $\gamma$ bezeichnete Kohlenstoffatom die S-Konfiguration aufweist, $R^4$ Methyl, Äthyl oder Isopropyl und $R^5$ und $R^6$ beide Wasserstoff bedeuten, herstellt.

4. Verfahren gemäß Anspruch 1, dadurch gekennzeichnet, daß man Verbindungen der in Anspruch 1 definierten allgemeinen Formel I, worin A zusammen mit den beiden mit $\alpha$ und $\beta$ bezeichneten Kohlenstoffatomen die in Anspruch 1 definierte Gruppe a) und die gestrichelte Linie die in diesem Fall vorliegende Doppelbindung bedeutet, D $>C=O$ oder $>C=S$, $R^1$ einen Rest der Formel $-COOR^4$ bedeuten, $R^2$ Wasserstoff, $R^3$ Wasserstoff oder $(C_{1-7})$-Alkyl, $R^4$ Methyl, Äthyl oder Isopropyl, $R^5$ Halogen und $R^6$ Wasserstoff bedeuten, herstellt.

5. Verfahren gemäß Anspruch 1, dadurch gekennzeichnet, daß man Verbindungen der in Anspruch 1 definierten allgemeinen Formel I, worin A zusammen mit den beiden mit $\alpha$ und $\beta$ bezeichneten Kohlenstoffatomen die in Anspruch 1 definierte Gruppe a) und die gestrichelte Linie die in diesem Fall vorliegende Doppelbindung bedeuten, D $>C=O$ oder $>C=S$, $R^1$ einen Rest der Formel $-COOR^4$ bedeuten, $R^2$ und $R^3$ zusammen Trimethylen bedeuten und das mit $\gamma$ bezeichnete Kohlenstoffatom die S-Konfiguration aufweist, $R^4$ Methyl, Äthyl oder Isopropyl, $R^5$ Fluor und $R^6$ Wasserstoff bedeuten, herstellt.

6. Verfahren gemäß Anspruch 1, dadurch gekennzeichnet, daß man Verbindungen der in Anspruch 1 definierten allgemeinen Formel I, worin $R^1$ Cyano oder einen Rest der Formel $-COOR^4$ bedeutet, wobei $R^4$ Methyl, Äthyl oder Isopropyl bedeutet, herstellt.

7. Verfahren gemäß Anspruch 1 oder 6, dadurch gekennzeichnet, daß man Verbindungen der in

Anspruch 1 definierten allgemeinen Formel I, worin $R^3$ Methyl bedeutet, wenn $R^2$ Wasserstoff bedeutet, herstellt.

8. Verfahren gemäß Anspruch 1 oder 6, dadurch gekennzeichnet, daß man Verbindungen der in Anspruch 1 definierten allgemeinen Formel I, worin das mit $\gamma$ bezeichnete Kohlenstoffatom die S-Konfiguration aufweist, wenn $R^2$ und $R^3$ zusammen Trimethylen bedeuten, herstellt.

9. Verfahren gemäß einem der Ansprüche 1 und 6 bis 8, dadurch gekennzeichnet, daß man Verbindungen der in Anspruch 1 definierten allgemeinen Formel I, worin A zusammen mit den beiden mit $\alpha$ und $\beta$ bezeichneten Kohlenstoffatomen die in Anspruch 1 definierte Gruppe a) und die gestrichelte Linie die in diesem Fall vorliegende Doppelbindung bedeuten, $R^5$ Wasserstoff oder Fluor und $R^6$ Wasserstoff, Fluor, Chlor oder Methyl bedeuten, wobei mindestens eines von $R^5$ und $R^6$ Wasserstoff bedeutet, herstellt.

10. Verfahren gemäß Anspruch 4, dadurch gekennzeichnet, daß man Äthyl-8-fluor-5,6-dihydro-5-methyl-6-oxo-4H-imidazo[1,5-a][1,4]benzodiazepin-3-carboxylat herstellt.

11. Verfahren gemäß Anspruch 1, dadurch gekennzeichnet, daß man Äthyl-7-fluor-5,6-dihydro-5-methyl-6-oxo-4H-imidazo[1,5-a][1,4]benzodiazepin-3-carboxylat herstellt.

12. Verfahren gemäß Anspruch 1, dadurch gekennzeichnet, daß man Äthyl-7-chlor-5,6-dihydro-5-methyl-6-oxo-4H-imidazo[1,5-a][1,4]benzodiazepin-3-carboxylat herstellt.

13. Verfahren gemäß Anspruch 1, dadurch gekennzeichnet, daß man Äthyl-(S)-8-chlor-11,12,13,13a-tetrahydro-9-oxo-9H-imidazo[1,5-a]pyrrolo[2,1-c][1,4]benzodiazepin-1-carboxylat herstellt.

14. Verfahren gemäß Anspruch 1, dadurch gekennzeichnet, daß man Äthyl-(R,S)-11,13a-dihydro-9-oxo-9H-imidazo[1,5-a]pyrrolo[2,1-c][1,4]benzodiazepin-1-carboxylat herstellt.

15. Verfahren gemäß Anspruch 1, dadurch gekennzeichnet, daß man Äthyl-8-fluor-11,13a-dihydro-9-oxo-9H-imidazo[1,5-a]pyrrolo[2,1-c][1,4]benzodiazepin-1-carboxylat herstellt.

**Claims for the Contracting States: BE, CH, DE, FR, IT, LI, LU, NL, SE**

1. Imidazodiazepine derivatives of the general formula

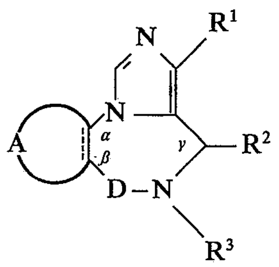

(I)

wherein A together with the two carbon atoms denoted as $\alpha$ and $\beta$ signifies one of the groups

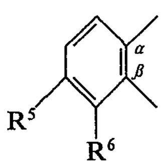

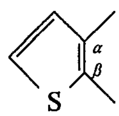

and

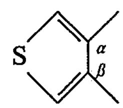

(a) (b) (c)

and the dotted line signifies the double bond present in cases (a) and (b), D signifies $>C=O$ or $>C=S$, $R^1$ signifies cyano, $(C_{2-7})$-alkanoyl or a residue of the formula $-COOR^4$, $R^4$ signifies methyl, ethyl, isopropyl or 2-hydroxyethyl, $R^5$ signifies hydrogen, trifluoromethyl or halogen and $R^6$ signifies hydrogen, trifluoromethyl, halogen or $(C_{1-7})$-alkyl and either $R^2$ signifies hydrogen and $R^3$ signifies hydrogen or $(C_{1-7})$-alkyl or $R^2$ and $R^3$ together signify trimethylene or propenylene and the carbon atom denoted as $\gamma$ has the S- or R,S-configuration,

and pharmaceutically acceptable acid addition salts thereof.

2. Compounds according to claim 1, characterized in that A together with the two carbon atoms denoted as $\alpha$ and $\beta$ signifies group (a) defined in claim 1 and the dotted line signifies the double bond present in this case, D signifies $>C=O$ or $>C=S$, $R^1$ signifies a residue of the formula $-COOR^4$, $R^2$ signifies hydrogen, $R^3$ signifies hydrogen or $(C_{1-7})$-alkyl, $R^4$ signifies methyl, ethyl or isopropyl and $R^5$ and $R^6$ both signify hydrogen.

3. Compounds according to claim 1, characterized in that A together with the two carbon atoms denoted as $\alpha$ and $\beta$ signifies group (a) defined in claim 1 and the dotted line signifies the double bond present in this case, D signifies $>C=O$ or $>C=S$, $R^1$ signifies a residue of the formula $-COOR^4$, $R^2$

and $R^3$ together signify trimethylene and the carbon atom denoted as $\gamma$ has the S-configuration, $R^4$ signifies methyl, ethyl or isopropyl and $R^5$ and $R^6$ both signify hydrogen.

4. Compounds according to claim 1, characterized in that A together with the two carbon atoms denoted as $\alpha$ and $\beta$ signifies group (a) defined in claim 1 and the dotted line signifies the double bond present in this case, D signifies $>C=O$ or $>C=S$, $R^1$ signifies a residue of the formula $-COOR^4$, $R^2$ signifies hydrogen, $R^3$ signifies hydrogen or $(C_{1-7})$-alkyl, $R^4$ signifies methyl, ethyl or isopropyl, $R^5$ signifies halogen and $R^6$ signifies hydrogen.

6. Compounds according to claim 1, characterized in that A together with the two carbon atoms denoted as $\alpha$ and $\beta$ signifies group (a) defined in claim 1 and the dotted line signifies the double bond present in this case, D signifies $>C=O$ or $>C=S$, $R^1$ signifies a residue of the formula $-COOR^4$, $R^2$ and $R^3$ together signify trimethylene and the carbon atom denoted as $\gamma$ has the S-configuration, $R^4$ signifies methyl, ethyl or isopropyl, $R^5$ signifies fluorine and $R^6$ signifies hydrogen.

6. Compounds according to claim 1, characterized in that $R^1$ signifies cyano or a residue of the formula $-COOR^4$ in which $R^4$ signifies methyl, ethyl or isopropyl.

7. Compounds according to claim 1 or 6, characterized in that $R^3$ signifies methyl when $R^2$ signifies hydrogen.

8. Compounds according to claim 1 or 6, characterized in that the carbon atom denoted as $\gamma$ has the S-configuration when $R^2$ and $R^3$ together signify trimethylene.

9. Compounds according to any one of claims 1 and 6 to 8, characterized in that A together with the two carbon atoms denoted as $\alpha$ and $\beta$ signifies the group (a) defined in claim 1 and the dotted line signifies the double bond present in this case, $R^5$ signifies hydrogen or fluorine and $R^6$ signifies hydrogen, fluorine, chlorine or methyl, whereby at least one of $R^5$ and $R^6$ signifies hydrogen.

10. Ethyl 8-fluoro-5,6-dihydro-5-methyl-6-oxo-4H-imidazo[1,5-a][1,4]benzodiazepine-3-carboxylate.

11. Ethyl 7-fluoro-5,6-dihydro-5-methyl-6-oxo-4H-imidazo[1,5-a][1,4]benzodiazepine-3-carboxylate.

12. Ethyl 7-chloro-5,6-dihydro-5-methyl-6-oxo-4H-imidazo[1,5-a][1,4]benzodiazepine-3-carboxylate.

13. Ethyl (S)-8-chloro-11,12,13,13a-tetrahydro-9-oxo-9H-imidazo[1,5-a]pyrrolo[2,1-c][1,4]benzodiazepine-1-carboxylate.

14. Ethyl (R,S)-11,13a-dihydro-9-oxo-9H-imidazo[1,5-a]pyrrolo[2,1-c][1,4]benzodiazepine-1-carboxylate.

15. Ethyl 8-fluoro-11,13a-dihydro-9-oxo-9H-imidazo[1,5-a]pyrrolo[2,1-c][1,4]benzodiazepine-1-carboxylate.

16. Ethyl 5,6-dihydro-5-methyl-6-oxo-4H-imidazo[1,5-a][1,4]benzodiazepine-3-carboxylate, methyl 5,6-dihydro-5-methyl-6-oxo-4H-imidazo[1,5-a][1,4]benzodiazepine-3-carboxylate, isopropyl 5,6-dihydro-5-methyl-6-oxo-4H-imidazo[1,5-a][1,4]benzodiazepine-3-carboxylate, methyl 8-fluoro-5,6-dihydro-5-methyl-6-oxo-4H-imidazo[1,5-a][1,4]benzodiazepine-3-carboxylate, isopropyl 8-fluoro-5,6-dihydro-5-methyl-6-oxo-4H-imidazo[1,5-a][1,4]benzodiazepine-3-carboxylate and ethyl 5,6-dihydro-5,7-methyl-6-oxo-4H-imidazo[1,5-a][1,4]benzodiazepine-3-carboxylate.

17. Ethyl (S)-11,12,13,13a-tetrahydro-9-oxo-9H-imidazo[1,5-a]pyrrolo[2,1-c][1,4]benzodiazepine-1-carboxylate, methyl (S)-11,12,13,13a-tetrahydro-9-oxo-9H-imidazo[1,5-a]pyrrolo[2,1-c][1,4]benzodiazepine-1-carboxylate, isopropyl (S)-11,12,13,13a-tetrahydro-9-oxo-9H-imidazo[1,5-a]pyrrolo[2,1-c][1,4]benzodiazepine-1-carboxylate, ethyl (S)-7-fluoro-11,12,13,13a-tetrahydro-9-oxo-9H-imidazo[1,5-a]pyrrolo[2,1-c][1,4]benzodiazepine-1-carboxylate and ethyl (S)-11,12,13,13a-tetrahydro-9-oxo-9H-imidazo[1,5-a]pyrrolo[2,1-c][1,4]benzodiazepine-1-carboxylate.

18. Ethyl 5,6-dihydro-5-methyl-6-thioxo-4H-imidazo[1,5-a][1,4]benzodiazepine-3-carboxylate and ethyl 8-fluoro-5,6-dihydro-5-methyl-6-thioxo-4H-imidazo[1,5-a][1,4]benzodiazepine-3-carboxylate.

19. 8-Fluoro-5,6-dihydro-5-methyl-6-oxo-4H-imidazo[1,5-a][1,4]benzodiazepine-3-carbonitrile, (S)-11,12,13,13a-tetrahydro-9-oxo-9H-imidazo[1,5-a]pyrrolo[2,1-c][1,4]benzodiazepine-1-carbonitrile and 5,6-dihydro-5-methyl-6-oxo-4H-imidazo[1,5-a][1,4]benzodiazepine-3-carbonitrile.

20. Methyl (S)-10,11,12,12a-tetrahydro-8-oxo-8H-imidazo[5,1-c]pyrrolo[1,2-a]thieno[3,2-e][1,4]diazepine-1-carboxylate, ethyl (S)-10,11,12,12a-tetrahydro-8-oxo-8H-imidazo[5,1-c]pyrrolo[1,2-a]thieno[3,2-e][1,4]diazepine-1-carboxylate, ethyl 5,6-dihydro-5-methyl-6-oxo-4H-imidazo[1,5-a]thieno[2,3-f][1,4]diazepine-3-carboxylate and methyl 5,6-dihydro-5-methyl-6-oxo-4H-imidazo[1,5-a]thieno[2,3-f][1,4]diazepine-3-carboxylate.

21. Compounds of the general formula

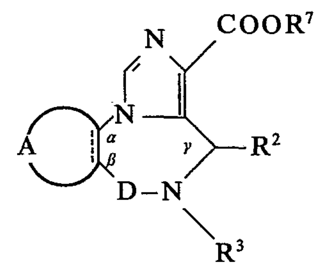

(XI)

wherein A, the dotted line, D, $R^2$ and $R^3$ have the significance given in claim 1 and $R^7$ signifies $(C_{1-7})$-alkyl, with the proviso that $R^7$ does not signify methyl, ethyl or isopropyl.

22. Compounds according to claim 21, characterized in that D signifies $>C=O$, $R^2$ signifies hydrogen and $R^3$ signifies methyl, A together with the two carbon atoms denoted as $\alpha$ and $\beta$ signifies the group (a) defined in claim 1 and the dotted line signifies the double bond present in this case, whereby $R^5$ signifies fluorine and $R^6$ signifies hydrogen.

23. Compounds according to any one of claims 1—20 and pharmaceutically acceptable acid addition salts thereof as pharmaceutically active substances.

24. Compounds according to any one of claims 1—20 and pharmaceutically acceptable acid addition salts thereof as active substances antagonizing the central-depressant, muscle relaxant, ataxic, blood pressure-lowering and respiratory-depressant properties of 1,4-benzodiazepines which have tranquillizing activity.

25. Ethyl 8-fluoro-5,6-dihydro-5-methyl-6-oxo-4H-imidazo[1,5-a][1,4]benzodiazepine-3-carboxylate and pharmaceutically acceptable acid addition salts thereof as pharmaceutically active substances.

26. Ethyl 8-fluoro-5,6-dihydro-5-methyl-6-oxo-4H-imidazo[1,5-a][1,4]benzodiazepine-3-carboxylate and pharmaceutically acceptable acid addition salts thereof as active substances antagonizing the central-depressant, muscle relaxant, ataxic, blood pressure-lowering and respiratory-depressant properties of 1,4-benzodiazepines which have tranquillizing activity.

27. Ethyl 7-chloro-5,6-dihydro-5-methyl-6-oxo-4H-imidazo[1,5-a][1,4]benzodiazepine-3-carboxylate and pharmaceutically acceptable acid addition salts thereof as pharmaceutically active substances.

28. Ethyl 7-chloro-5,6-dihydro-5-methyl-6-oxo-4H-imidazo[1,5-a][1,4]benzodiazepine-3-carboxylate and pharmaceutically acceptable acid addition salts thereof as active substances antagonizing the central-depressant, muscle relaxant, ataxic, blood pressure-lowering and respiratory-depressant properties of 1,4-benzodiazepines which have tranquillizing activity.

29. A process for the manufacture of compounds according to any one of claims 1—20 and of pharmaceutically acceptable acid addition salts thereof, characterized by

a) reacting a compound of the general formula

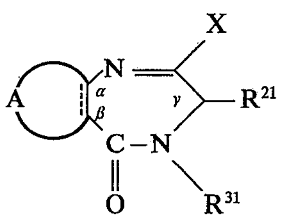

(II)

wherein A and the dotted line have the significance given in claim 1 and X signifies a leaving group, and either $R^{21}$ signifies hydrogen and $R^{31}$ signifies $(C_{1-7})$-alkyl, or $R^{21}$ and $R^{31}$ together signify trimethylene or propenylene, and the carbon atom denoted as $\gamma$ has the S- or R,S-configuration,

in the presence of a base with an isocyanoacetic ester of the general formula

$$CN-CH_2-COOR^{41} \qquad (III)$$

wherein $R^{41}$ signifies methyl, ethyl or isopropyl,

or

b) treating a compound of the general formula

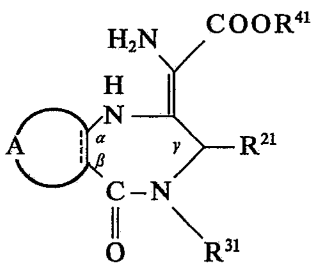

(IV)

wherein A and the dotted line have the significance given in claim 1 and $R^{21}$, $R^{31}$ and $R^{41}$ have the above significance,

with a formylating agent, or

c) dehydrogenating a compound of the general formula

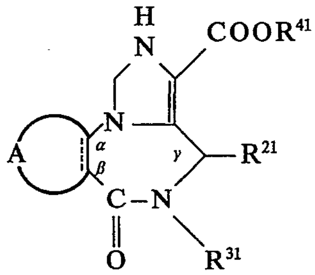

(V)

or

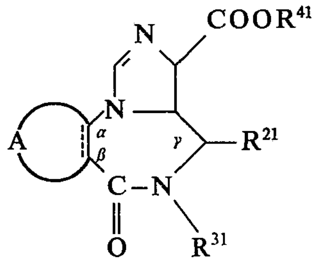

(VI)

wherein A and the dotted line have the significance given in claim 1 and $R^{21}$, $R^{31}$ and $R^{41}$ have the above significance,

or

d) converting the carboxamido group in a compound ot the general formula

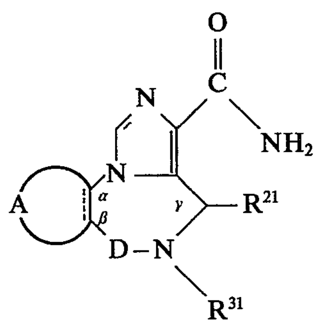

(VII)

wherein A, the dotted line and D have the significance given in claim 1 and $R^{21}$ and $R^{31}$ have the above significance,

into the nitrile group, or

e) appropriately substituting a compound of the general formula

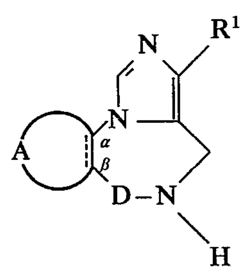

(Ia)

wherein A, the dotted line, D and $R^1$ have the above significance,

at the secondary amino group, or

f) cleaving off the protecting group in a compound of the general formula

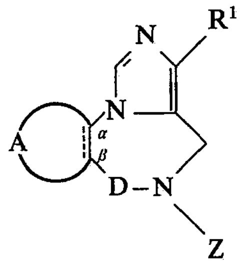

(VIII)

wherein A, the dotted line, D and $R^1$ have the significance given in claim 1 and Z signifies a protecting group,

or

g) converting the oxime groupe in a compound of the general formula

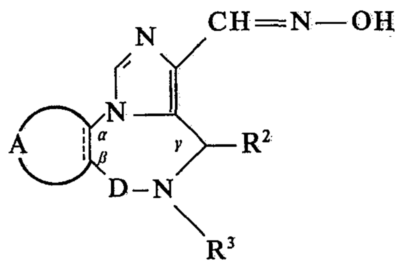

(IX)

wherein A, the dotted line, D, $R^2$ and $R^3$ have the significance given in claim 1,

into the nitrile group, or

h) converting the hydroxyl group in a compound of the general formula

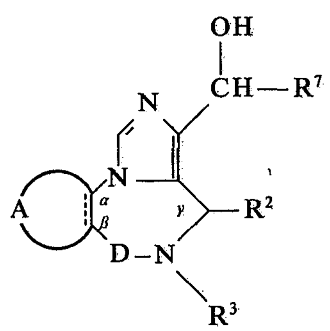

(X)

wherein A, the dotted line, D, $R^2$ and $R^3$ have the significance given in claim 1 and $R^7$ signifies $(C_{1-6})$-alkyl,

into the keto group, or

i) trans-esterifying a compound of the general formula

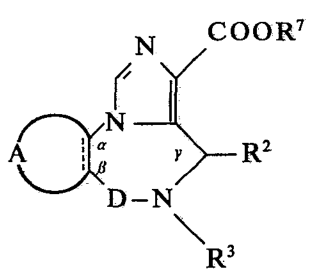

(XI)

wherein A, the dotted line, D, $R^2$ and $R^3$ have the significance given in claim 1 and $R^7$ signifies

$(C_{1-7})$-alkyl,

or

j) converting the carbonyl group in a compound of the general formula

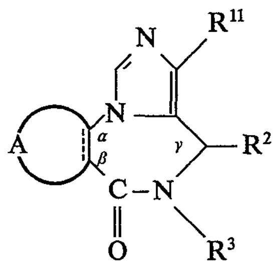

(Ib)

wherein $R^{11}$ signifies cyano or a residue of the formula $-COOR^4$ and A, the dotted line, $R^2$, $R^3$ and $R^4$ have the significance given in claim 1,

into the thiocarbonyl group, and

k) if desired, converting a compound of general formula I into a pharmaceutically acceptable acid addition salt.

30. A process according to claim 29, characterized in that ethyl 8-fluoro-5,6-dihydro-5-methyl-6-oxo-4H-imidazo[1,5-a][1,4]benzodiazepine-3-carboxylate is manufactured.

31. A process according to claim 29, characterized in that ethyl 7-chloro-5,6-dihydro-5-methyl-6-oxo-4H-imidazo[1,5-a][1,4]benzodiazepine-3-carboxylate is manufactured.

32. A medicament containing a compound according to any one of claims 1 to 20 or a pharmaceutically acceptable acid addition salt thereof.

33. A medicament which antagonizes the central-depressant, muscle relaxant, ataxic, blood pressure-lowering and respiratory-depressant properties of 1,4-benzodiazepines which have tranquillizing activity, said medicament containing a compound according to any one of claims 1 to 20 or a pharmaceutically acceptable acid addition salt thereof.

34. A medicament containing ethyl 8-fluoro-5,6-dihydro-5-methyl-6-oxo-4H-imidazo[1,5-a][1,4]benzodiazepine-3-carboxylate or a pharmaceutically acceptable acid addition salt thereof.

35. A medicament containing ethyl 7-chloro-5,6-dihydro-5-methyl-6-oxo-4H-imidazo[1,5-a][1,4]benzodiazepine-3-carboxylate or a pharmaceutically acceptable acid addition salt thereof.

36. A medicament which antagonizes the central-depressant, muscle relaxant, ataxic, blood pressure-lowering and respiratory-depressant properties of 1,4-benzodiazepines which have tranquillizing activity, said medicament containing ethyl 8-fluoro-5,6-dihydro-5-methyl-6-oxo-4H-imidazo[1,5-a][1,4]benzodiazepine-3-carboxylate or a pharmaceutically acceptable acid addition salt thereof.

37. A medicament which antagonizes the central-depressant, muscle relaxant, ataxic, blood pressure-lowering and respiratory-depressant properties of 1,4-benzodiazepines which have tranquillizing activity, said medicament containing ethyl 7-chloro-5,6-dihydro-5-methyl-6-oxo-4H-imidazo[1,5-a][1,4]benzodiazepine-3-carboxylate or a pharmaceutically acceptable acid addition salt thereof.

38. A schistosomicidally-active medicament containing a compound according to any one of claims 1 to 20 or a pharmaceutically acceptable acid addition salt thereof and a compound of the general formula

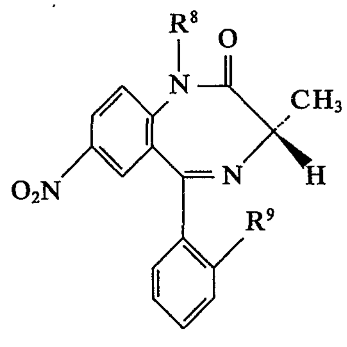

(XXII)

wherein $R^8$ signifies hydrogen or $(C_{1-7})$-alkyl and $R^9$ signifies halogen,

and/or a compound of the general formula

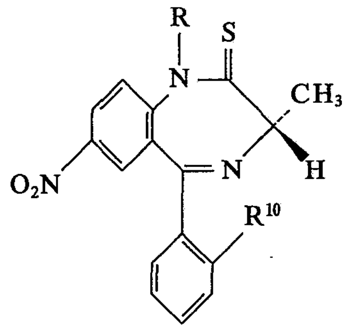

(XXIII)

wherein R signifies hydrogen or $(C_{1-7})$-alkyl and $R^{10}$ signifies hydrogen, halogen or trifluoromethyl.

39. A schistosomicidally-active medicament according to claim 38, characterized in that it contains ethyl 8-fluoro-5,6-dihydro-5-methyl-6-oxo-4H-imidazo[1,5-a][1,4]benzodiazepine-3-carboxylate or a pharmaceutically acceptable acid addition salt thereof.

40. A schistosomicidally-active medicament according to claim 38, characterized in that it contains ethyl 7-chloro-5,6-dihydro-5-methyl-6-oxo-4H-imidazo[1,5-a][1,4]benzodiazepine-3-carboxylate or a pharmaceutically acceptable acid addition salt thereof.

41. A schistosomicidally-active medicament according to claim 38, characterized in that it contains (+)-5-(o-chlorophenyl)-1,3-dihydro-3-methyl-7-nitro-2H-1,4-benzodiazepin-2-one.

42. A schistosomicidally-active medicament containing ethyl 8-fluoro-5,6-dihydro-5-methyl-6-oxo-4H-imidazo[1,5-a][1,4]benzodiazepine-3-carboxylate or a pharmaceutically acceptable acid addition salt thereof and (+)-5-(o-chlorophenyl)-1,3-dihydro-3-methyl-7-nitro-2H-1,4-benzodiazepin-2-one.

43. A schistosomicidally-active medicament containing ethyl 7-chloro-5,6-dihydro-5-methyl-6-oxo-4H-imidazo[1,5-a][1,4]benzodiazepine-3-carboxylate or a pharmaceutically acceptable acid addition thereof and (+)-5-(o-chlorophenyl)-1,3-dihydro-3-methyl-7-nitro-2H-1,4-benzodiazepin-2-one.

**Claims for the Contracting State: AT**

1. A process for the manufacture of imidazodiazepine derivatives of the general formula

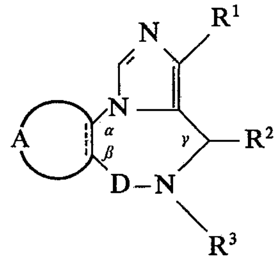

(I)

wherein A together with the two carbon atoms denoted as $\alpha$ and $\beta$ signifies one of the groups

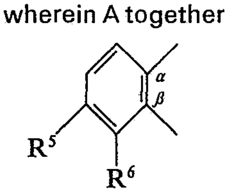

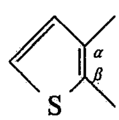

and

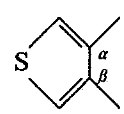

(a) (b) (c)

and the dotted line signifies the double bond present in cases (a) and (b), D signifies $>C=O$ or $>C=S$, $R^1$ signifies cyano, $(C_{2-7})$-alkanoyl or a residue of the formula $-COOR^4$, $R^4$ signifies methyl, ethyl, isopropyl or 2-hydroxyethyl, $R^5$ signifies hydrogen, trifluoromethyl or halogen and $R^6$ signifies hydrogen, trifluoromethyl, halogen or $(C_{1-7})$-alkyl and either $R^2$ signifies hydrogen and $R^3$ signifies hydrogen or $(C_{1-7})$-alkyl or $R^2$ and $R^3$ together signify trimethylene or propenylene and the carbon atom denoted as $\gamma$ has the S- or R,S-configuration,

and of pharmaceutically acceptable acid addition salts thereof, characterized by

a) reacting a compound of the general formula

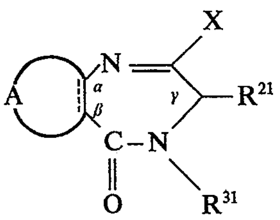

(II)

wherein A and the dotted line have the above significance and X signifies a leaving group, and either $R^{21}$ signifies hydrogen and $R^{31}$ signifies $(C_{1-7})$-alkyl, or $R^{21}$ and $R^{31}$ together signify trimethylene or propenylene and the carbon atom denoted as $\gamma$ has the S- or R,S-configuration,

in the presence of a base with an isocyanoacetic ester of the general formula

$CN{-}CH_2{-}COOR^{41}$ (III)

wherein $R^{41}$ signifies methyl, ethyl or isopropyl,

or

b) treating a compound of the general formula

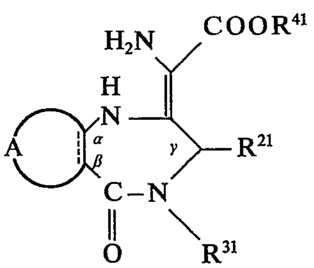

(IV)

wherein A, the dotted line, $R^{21}$, $R^{31}$ and $R^{41}$ have the above significance,

with a formylating agent, or

c) dehydrogenating a compound of the general formula

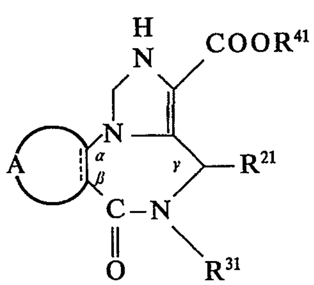

(V)

or

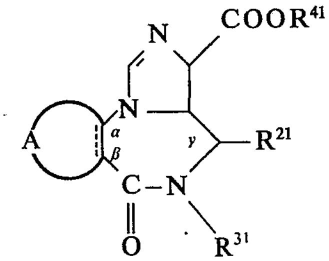

(VI)

wherein A, the dotted line, $R^{21}$, $R^{31}$ and $R^{41}$ have the above significance,

or

d) converting the carboxamido group in a compound of the general formula

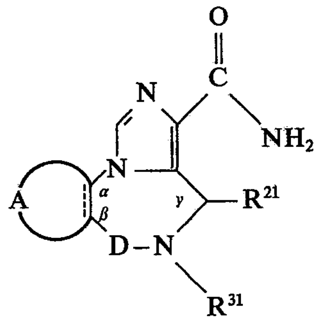

(VII)

wherein A, the dotted line, D, $R^{21}$ and $R^{31}$ have the above significance,

into the nitrile group, or

e) appropriately substituting a compound of the general formula

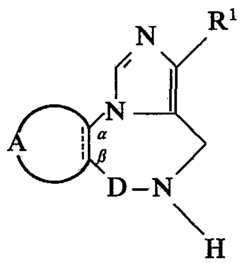

(Ia)

wherein A, the dotted line, D and $R^1$ have the above significance,

at the secondary amino group, or

f) cleaving off the protecting group in a compound of the general formula

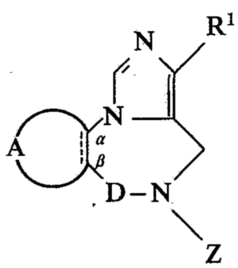

(VIII)

wherein A, the dotted line, D and $R^1$ have the above significance and Z signifies a protecting group,

or

g) converting the oxime group in a compound of the general formula

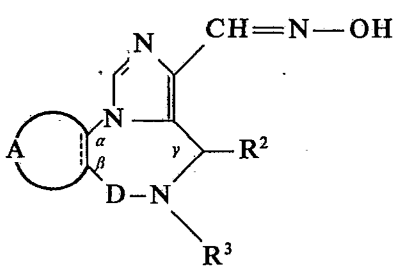

(IX)

wherein A, the dotted line, D, $R^2$ and $R^3$ have the above significance,

into the nitrile group, or

h) converting the hydroxyl group in a compound of the general formula

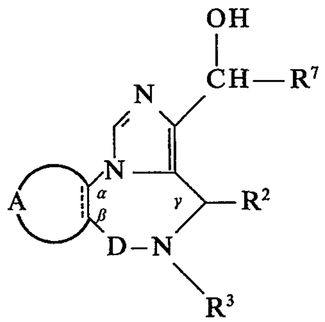

(X)

wherein A, the dotted line, D, $R^2$ and $R^3$ have the above significance and $R^7$ signifies $(C_{1-6})$-alkyl,

into the keto group, or

i) trans-esterifying a compound of the general formula

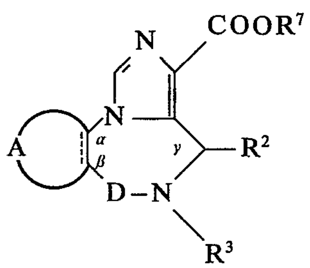

(XI)

wherein A, the dotted line, D, $R^2$ and $R^3$ have the above significance and $R^7$ signifies $(C_{1-7})$-alkyl,

or

j) converting the carbonyl group in a compound of the general formula

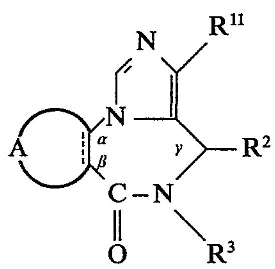

(Ib)

wherein $R^{11}$ signifies cyano or a residue of the formula $-COOR^4$ and A, the doted line, $R^2$, $R^3$ and $R^4$ have the above significance,

into the thiocarbonyl group, and

k) if desired, converting a compound of general formula I into a pharmaceutically acceptable acid addition salt.

2. A process according to claim 1, characterized in that compounds of general formula I defined in claim 1 wherein A together with the two carbon atoms denoted as $\alpha$ and $\beta$ signifies the group a) defined in claim 1 and the dotted line signifies the double bond present in this case, D signifies $>C=O$ or $>C=S$, $R^1$ signifies a residue of the formula $-COOR^4$, $R^2$ signifies hydrogen, $R^3$ signifies hydrogen or $(C_{1-7})$-alkyl, $R^4$ signifies methyl, ethyl or isopropyl and $R^5$ and $R^6$ both signify hydrogen are manufactured.

3. A process according to claim 1, characterized in that compounds of general formula I defined in claim 1 wherein A together with the two carbon atoms denoted as $\alpha$ and $\beta$ signifies the group a) defined in claim 1 and the dotted line signifies the double bond present in this case, D signifies $>C=O$

or $>C=S$, $R^1$ signifies a residue of the formula $-COOR^4$, $R^2$ and $R^3$ together signify trimethylene and the carbon atom denoted as $\gamma$ has the S-configuration, $R^4$ signifies methyl, ethyl or isopropyl and $R^5$ and $R^6$ both signify hydrogen are manufactured.

4. A process according to claim 1, characterized in that compounds of general formula I defined in claim 1 wherein A together with the two carbon atoms denoted as $\alpha$ and $\beta$ signifies the group a) defined in claim 1 and the dotted line signifies the double bond present in this case, D signifies $>C=O$ or $>C=R$, $R^1$ signifies a residue of the formula $-COOR^4$, $R^2$ signifies hydrogen, $R^3$ signifies hydrogen or $(C_{1-7})$-alkyl, $R^4$ signifies methyl, ethyl or isopropyl, $R^5$ signifies halogen and $R^6$ signifies hydrogen are manufactured.

5. A process according to claim 1, characterized in that compounds of general formula I defined in claim 1 wherein A together with the two carbon atoms denoted as $\alpha$ and $\beta$ signifies the group a) defined in claim 1 and the dotted line signifies the double bond present in this case, D signifies $>C=O$ or $>C=S$, $R^1$ signifies a residue of the formula $-COOR^4$, $R^2$ and $R^3$ together signify trimethylene and the carbon atom denoted as $\gamma$ has the S-configuration, $R^4$ signifies methyl, ethyl or isopropyl, $R^5$ signifies fluorine and $R^6$ signifies hydrogen are manufactured.

6. A process according to claim 1, characterized in that compounds of general formula I defined in claim 1 wherein $R^1$ signifies cyano or a residue of the formula $-COOR^4$ in which $R^4$ signifies methyl, ethyl or isopropyl are manufactured.

7. A process according to claim 1 or 6, characterized in that compounds of general formula I defined in claim 1 wherein $R^3$ signifies methyl when $R^2$ signifies hydrogen are manufactured.

8. A process according to claim 1 or 6, characterized in that compounds of general formula I defined in claim 1 wherein the carbon atom denoted as $\gamma$ has the S-configuration when $R^2$ and $R^3$ together signify trimethylene are manufactured.

9. A process according to any one of claim 1 and 6 to 8, characterized in that compounds of general formula I defined in claim 1 wherein A together with the two carbon atoms denoted as $\alpha$ and $\beta$ signifies the group a) defined in claim 1 and the dotted line signifies the double bond present in this case, $R^5$ signifies hydrogen or fluorine and $R^6$ signifies hydrogen, fluorine, chlorine or methyl, whereby at least one of $R^5$ and $R^6$ signifies hydrogen, are manufactured.

10. A process according to claim 1, characterized in that ethyl 8-fluoro-5,6-dihydro-5-methyl-6-oxo-4H-imidazo[1,5-a][1,4]benzodiazepine-3-carboxylate is manufactured.

11. A process according to claim 1, characterized in that ethyl 7-fluoro-5,6-dihydro-5-methyl-6-oxo-4H-imidazo[1,5-a][1,4]benzodiazepine-3-carboxylate is manufactured.

12. A process according to claim 1, characterized in that ethyl 7-chloro-5,6-dihydro-5-methyl-6-oxo-4H-imidazo[1,5-a][1,4]benzodiazepine-3-carboxylate is manufactured.

13. A process according to claim 1, characterized in that ethyl (S)-8-chloro-11,12,13,13a-tetrahydro-9-oxo-9H-imidazo[1,5-a]pyrrolo[2,1-c][1,4]benzodiazepine-1-carboxylate is manufactured.

14. A process according to claim 1, characterized in that ethyl (R,S)-11,13a-dihydro-9-oxo-9H-imidazo[1,5-a]pyrrolo[2,1-c][1,4]benzodiazepine-1-carboxylate is manufactured.

15. A process according to claim 1, characterized in that ethyl 8-fluoro-(S)-11,13a-dihydro-9-oxo-9H-imidazo[1,5-a]pyrrolo[2,1-c][1,4]benzodiazepine-1-carboxylate is manufactured.

**Revendications pour les Etats Contractans: BE, CH, DE, FR, IT, LI, LU, NL, SE.**

1. Dérivés de l'imidazodiazépine de formule générale

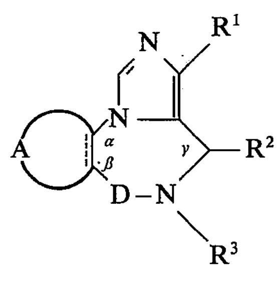

(I)

dans laquelle A forme, avec les deux atomes de carbone $\alpha$ et $\beta$, l'un des groupes

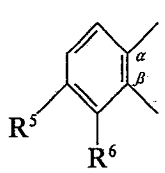

(a)

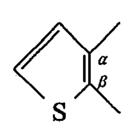

(b)

et

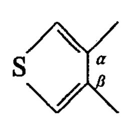

(c)

et le trait interrompu représente la double liaison existant dans les cas (a) et (b), D représente $>C=O$ ou $>C=S$, $R^1$ représente un groupe cyano, alcanoyle en C 2—C 7 ou un reste de formule $-COOR^4$, $R^4$ représente un groupe méthyle, éthyle, isopropyle ou 2-hydroxyéthyle, $R^5$ représente l'hydrogène, un groupe trifluorométhyle ou un halogène et $R^6$ représente l'hydrogène, un groupe trifluorométhyle, un halogène ou un groupe alkyle en C 1—C 7 et ou bien $R^2$ représente l'hydrogène et $R^3$ représente l'hydrogène ou un groupe alkyle en C 1—C 7, ou bien $R^2$ et $R^3$ forment ensemble un groupe triméthylène ou propénylène, et l'atome de carbone $\gamma$ est en configuration S ou en configuration R,S,

et leurs sels formés par addition avec des acides acceptables pour l'usage pharmaceutique.

2. Composés selon la revendication 1, caractérisés en ce que A forme avec les deux atomes de carbone $\alpha$ et $\beta$ le groupe (a) défini dans la revendication 1 et le trait interrompu représente la double liaison existant dans ce cas, D représente $>C=O$ ou $>C=S$, $R^1$ représente un reste de formule $-COOR^4$, $R^2$ représente l'hydrogène, $R^3$ représente l'hydrogène ou un groupe alkyle en C 1—C 7, $R^4$ représente un groupe méthyle, éthyle ou isopropyle et $R^5$ et $R^6$ représentent tous deux l'hydrogène.

3. Composés selon la revendication 1, caractérisés en ce que A forme avec les deux atomes de carbone $\alpha$ et $\beta$ le groupe (a) défini dans la revendication 1 et le trait interrompu représente la double liaison existant dans ce cas, D représente $>C=O$ ou $>C=S$, $R^1$ représente un reste de formule $-COOR^4$, $R^2$ et $R^3$ forment ensemble un groupe triméthylène et l'atome de carbone $\gamma$ est en configuration S, $R^4$ représente un groupe méthyle, éthyle ou isopropyle et $R^5$ et $R^6$ sont tous deux des atomes d'hydrogène.

4. Composés selon la revendication 1, caractérisés en ce que A forme avec les deux atomes de carbone $\alpha$ et $\beta$ le groupe (a) défini dans la revendication 1 et le trait interrompu représente la double liaison existant dans ce cas, D représente $>C=O$ ou $>C=S$, $R^1$ représente un reste de formule $-COOR^4$, $R^2$ représente l'hydrogène, $R^3$ représente l'hydrogène ou un groupe alkyle en C 1—C 7, $R^4$ représente un groupe méthyle, éthyle ou isopropyle, $R^5$ représente un halogène et $R^6$ l'hydrogène.

5. Composés selon la revendication 1, caractérisés en ce que A forme avec les deux atomes de carbonne $\alpha$ et $\beta$ le groupe (a) défini dans la revendication 1 et le trait interrompu représente la double liaison existant dans ce cas, D représente $>C=O$ ou $>C=S$, $R^1$ représente un reste de formule $-COOR^4$, $R^2$ et $R^3$ forment ensemble le groupe triméthylène et l'atome de carbone $\gamma$ est en configuration S, $R^4$ représente un groupe méthyle, éthyle ou isopropyle, $R^5$ représente le fluor et $R^6$ l'hydrogène.

6. Composés selon la revendication 1, caractérisés en ce que $R^1$ représente le groupe cyano ou un reste de formule $-COOR^4$, $R^4$ étant un groupe méthyle, éthyle ou isopropyle.

7. Composés selon la revendication 1 ou 6, caractérisés en ce que $R^3$ représente le groupe méthyle lorsque $R^2$ représente l'hydrogène.

8. Composés selon la revendication 1 ou 6, caractérisés en ce que l'atome de carbone de la formule I est en configuration S lorsque $R^2$ et $R^3$ forment ensemble le groupe triméthylène.

9. Composés selon l'une des revendications 1 et 6 à 8, caractérisés en ce que A forme avec les deux atomes de carbone $\alpha$ et $\beta$ le groupe (a) défini dans la revendication 1 et le trait interrompu représente la double liaison existant dans ce cas, $R^5$ représente l'hydrogène ou le fluor et $R^6$ l'hydrogène, le fluor, le chlore ou un groupe méthyle, l'un au moins des symboles $R^5$ et $R^6$ représentant l'hydrogène.

10. Le 8-fluoro-5,6-dihydro-5-méthyl-6-oxo-4H-imidazo[1,5-a][1,4]benzodiazépine-3-carboxylate d'éthyle.

11. Le 7-fluoro-5,6-dihydro-5-méthyl-6-oxo-4H-imidazo[1,5-a][1,4]benzodiazépine-3-carboxylate d'éthyle.

12. Le 7-chloro-5,6-dihydro-5-méthyl-6-oxo-4H-imidazo[1,5-a][1,4]benzodiazépine-3-carboxylate d'éthyle.

13. Le (S)-8-chloro-11,12,13,13a-tétrahydro-9-oxo-9H-imidazo[1,5-a]pyrrolo[2,1-c][1,4]benzodiazépine-1-carboxylate d'éthyle.

14. Le (R,S)-11,13a-dihydro-9-oxo-9H-imidazo[1,5-a]pyrrolo[2,1-c][1,4]benzodiazépine-1-carboxylate d'éthyle.

15. Le 8-fluoro-11,13a-dihydro-9-oxo-9H-imidazo[1,5-a]pyrrolo[2,1-c][1,4]benzodiazépine-1-carboxylate d'éthyle.

16. Le 5,6-dihydro-5-méthyl-6-oxo-4H-imidazo[1,5-a][1,4]benzodiazépine-3-carboxylate d'éthyle, le 5,6-dihydro-5-méthyl-6-oxo-4H-imidazo[1,5-a][1,4]benzodiazépine-3-carboxylate de méthyle, le 5,6-dihydro-5-méthyl-6-oxo-4H-imidazo[1,5-a][1,4]benzodiazépine-3-carboxylate d'isopropyle, le 8-fluoro-5,6-dihydro-5-méthyl-6-oxo-4H-imidazo[1,5-a][1,4]benzodiazépine-3-carboxylate de méthyle, le 8-fluoro-5,6-dihydro-5-méthyl-6-oxo-4H-imidazo[1,5-a][1,4]benzodiazépine-3-carboxylate d'isopropyle et le 5,6-dihydro-5,7-diméthyl-6-oxo-4H-imidazo[1,5-a][1,4]benzodiazépine-3-carboxylate d'éthyle.

17. Le (S)-11,12,13,13a-tétrahydro-9-oxo-9H-imidazo[1,5-a]pyrrolo[2,1-c][1,4]benzodiazépine-1-carboxylate d'éthyle, le (S)-11,12,13,13a-tétrahydro-9-oxo-9H-imidazo[1,5-a]pyrrolo[2,1-c][1,4]benzodiazépine-1-carboxylate de méthyle, le (S)-11,12,13,13a-tétrahydro-9-oxo-9H-imidazo[1,5-a]pyrrolo[2,1-c][1,4]benzodiazépine-1-carboxylate d'isopropyle, le (S)-7-fluoro-11,12,13,13a-tétrahydro-9-oxo-9H-imidazo[1,5-a]pyrrolo[2,1-c][1,4]benzodiazépine-1-carboxylate d'éthyle et le (S)-11,12,13,13a-tétrahydro-8-méthyl-9-oxo-9H-imidazo[1,5-a]pyrrolo[2,1-c][1,4]benzodiazépine-1-carboxylate d'éthyle.

18. Le 5,6-dihydro-5-méthyl-6-thioxo-4H-imidazo[1,5-a][1,4]benzodiazépine-3-carboxylate d'éthyle et le 8-fluoro-5,6-dihydro-5-méthyl-6-thioxo-4H-imidazo[1,5-a][1,4]benzodiazépine-3-carboxylate d'éthyle.

19. Le 8-fluoro-5,6-dihydro-5-méthyl-6-oxo-4H-imidazo[1,5-a][1,4]benzodiazépine-3-carbonitrile, le (S)-11,12,13,13a-tétrahydro-9-oxo-9H-imidazo[1,5-a]pyrrolo[2,1-c][1,4]benzodiazépine-1-carbonitrile et le 5,6-dihydro-5-méthyl-6-oxo-4H-imidazo[1,5-a][1,4]benzodiazépine-3-carbonitrile.

20. Le (S)-10,11,12,12a-tétrahydro-8-oxo-8H-imidazo[5,1-c]pyrrolo[1,2-a]thiéno[3,2-e][1,4]diazépine-1-carboxylate de méthyle, le (S)-10,11,12,12a-tétrahydro-8-oxo-8H-imidazo[5,1-c]pyrrolo[1,2-a]thiéno[3,2-e][1,4]diazépine-1-carboxylate d'éthyle, le 5,6-dihydro-5-méthyl-6-oxo-4H-imidazo[1,5-a]thiéno[2,3-f][1,4]diazépine-3-carboxylate d'éthyle et le 5,6-dihydro-5-méthyl-6-oxo-4H-imidazo[1,5-a]thiéno[2,3-f][1,4]diazépine-3-carboxylate de méthyle.

21. Composés de formule générale

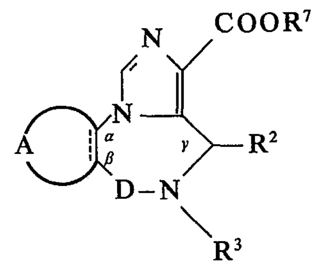

(XI)

dans laquelle A, le trait interrompu, D, $R^2$ et $R^3$ ont les significations indiquées dans la revendication 1, et $R^7$ représente un groupe alkyle en C 1 — C 7, à l'exception des groupes méthyle, éthyle et isopropyle.

22. Composés selon la revendication 21, caractérisés en ce que D représente $>C=O$, $R^2$ représente l'hydrogène et $R^3$ le groupe méthyle, A forme avec les deux atomes de carbone $\alpha$ et $\beta$ le groupe (a) défini dans le revendication 1 et le trait interrompu représente la double liaison existant dans ce cas, $R^5$ représente le fluor et $R^6$ l'hydrogène.

23. Composés selon l'une des revendications 1 à 20, et leurs sels formés par addition avec des acides acceptables pour l'usage pharmaceutique, en tant que substances actives pharmaceutiques.

24. Composés selon l'une des revendications 1 à 20, et leurs sels formés par addition avec des acides acceptables pour l'usage pharmaceutique en tant que substances actives antagonisant les propriétés sédatives centrales, myorelaxantes, atactiques, hypotensives et dépressives respiratoires des 1,4-benzodiazépines à activité tranquillisante.

25. Le 8-fluoro-5,6-dihydro-5-méthyl-6-oxo-4H-imidazo[1,5-a][1,4]benzodiazépine-3-carboxylate d'éthyle et ses sels formés par addition avec des acides acceptables pour l'usage pharmaceutique en tant que substances actives pharmaceutiques.

26. Le 8-fluoro-5,6-dihydro-5-méthyl-6-oxo-4H-imidazo[1,5-a][1,4]benzodiazépine-3-carboxylate d'éthyle et ses sels formés par addition avec des acides acceptables pour l'usage pharmaceutique en tant que substances actives antagonisant les propriétés sédatives centrales, myorelaxantes, atactiques, hypotensives et dépressives respiratoires des 1,4-benzodiazépines à activité tranquillisante.

27. Le 7-chloro-5,6-dihydro-5-méthyl-6-oxo-4H-imidazo[1,5-a][1,4]benzodiazépine-3-carboxylate d'éthyle et ses sels formés par addition avec des acides acceptables pour l'usage pharmaceutique, en tant que substances actives pharmaceutiques.

28. Le 7-chloro-5,6-dihydro-5-méthyl-6-oxo-4H-imidazo[1,5-a][1,4]benzodiazépine-3-carboxylate d'éthyle et ses sels formés par addition avec des acides acceptables pour l'usage pharmaceutique, en tant que substances actives antagonisant les propriétés sédatives centrales, myorelaxantes, atactiques, hypotensives et dépressives respiratoires des 1,4-benzodiazépines à activité tranquillisante.

29. Procédé de préparation des composés selon l'une des revendications 1 à 20 et de leurs sels formés par addition avec des acides acceptables pour l'usage pharmaceutique, caractérisé en ce que:

a) on fait réagir un composé de formule générale

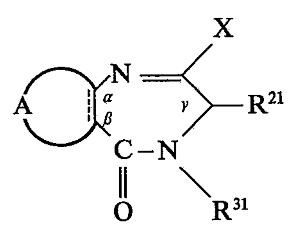

(II)

dans laquelle A et le trait interrompu ont les significations indiquées dans la revendication 1 et X représente un groupe éliminable, et ou bien $R^{21}$ représente l'hydrogène et $R^{31}$ un groupe alkyle en

C 1—C 7, ou bien $R^{21}$ et $R^{31}$ forment ensemble un groupe triméthylène ou propénylène, et l'atome de carbone $\gamma$ est en configuration S ou R,S,

en présence d'une base, avec un ester isocyanacétique de formule générale

$$CN-CH_2-COOR^{41} \qquad (III)$$

dans laquelle $R^{41}$ représente un groupe méthyle, éthyle ou isopropyle,

ou bien

b) on traite un composé de formule générale

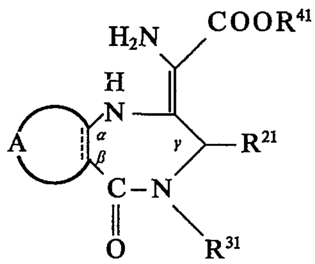

(IV)

dans laquelle A et le trait interrompu ont les significations indiquées dans la revendication 1 et $R^{21}$, $R^{31}$ et $R^{41}$ ont les significations indiquées ci-dessus,

par un agent formylant, ou bien

c) on soumet un composé de formule générale

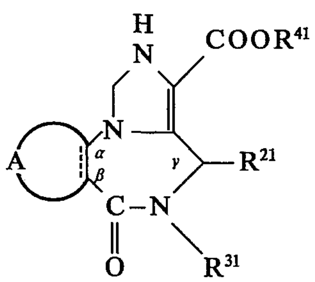

(V)

ou

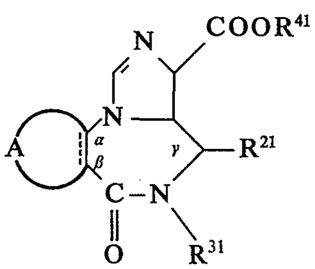

(VI)

dans lesquelles A et le trait interrompu ont les significations indiquées dans la revendication 1 et $R^{21}$, $R^{31}$ et $R^{41}$ ont les significations indiquées ci-dessus, à déshydrogénation,

ou bien

d) dans un composé de formule générale

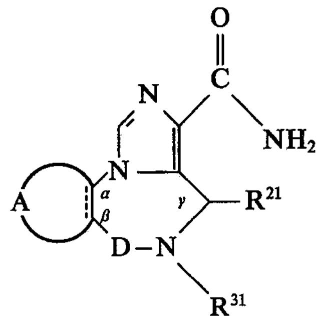

(VII)

dans laquelle A, le trait interrompu et D ont les significations indiquées dans la revendication 1, et $R^{21}$ et $R^{31}$ ont les significations indiquées ci-dessus,

on convertit le groupe carboxammido en le groupe nitrile, ou bien

e) dans un composé de formule générale

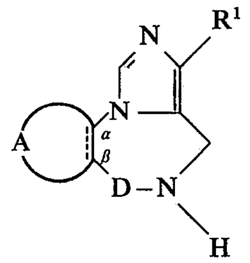

(Ia)

dans laquelle A, le trait interrompu, D et $R^1$ ont les significations indiquées dans la revendication 1,

on introduit le substituant voulu sur le groupe amino secondaire, ou bien

f) dans un composé de formule générale

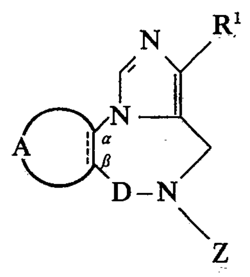

(VIII)

dans laquelle A, le trait interrompu, D et $R^1$ ont les significations indiquées dans la revendication 1, et Z représente un groupe protecteur,

on élimine le groupe protecteur, ou bien

g) dans un composé de formule générale

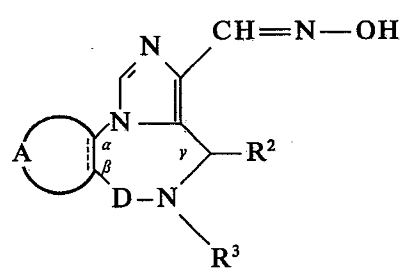

(IX)

dans laquelle A, le trait interrompu, D, $R^2$ et $R^3$ ont les significations indiquées dans la revendication 1,

on convertit le groupe oxime en le groupe nitrile, ou bien

h) dans un composé de formule générale

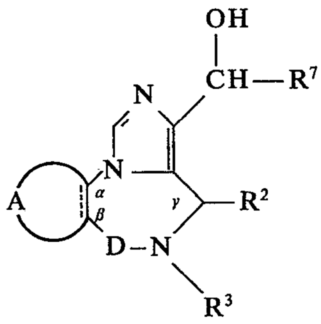

(X)

dans laquelle A, le trait interrompu, D, $R^2$ et $R^3$ ont les significations indiquées dans la revendication 1, et $R^7$ représente un groupe alkyle en C 1—C 6,

on convertit le groupe hydroxy en le groupe céto, ou bien

i) on transestérifie un composé de formule générale

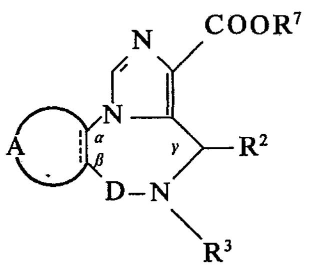

(XI)

dans laquelle A, le trait interrompu, D, $R^2$ et $R^3$ ont les significations indiquées dans la revendication 1, et $R^7$ représente un groupe alkyle en C 1—C 7,

ou bien

j) dans un composé de formule générale

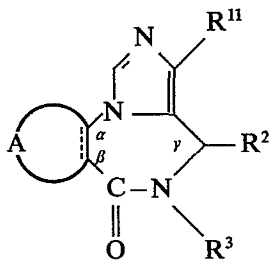

(Ib)

dans laquelle $R^{11}$ représente un groupe cyano ou un reste de formule —$COOR^4$, et A, le trait interrompu, $R^2$, $R^3$ et $R^4$ ont les significations indiquées dans la revendication 1,

on convertit le groupe carbonyle en le groupe thiocarbonyle, et

k) si on le désire, on convertit un composé de formule générale I en un sel formé par addition avec un acide acceptable pour l'usage pharmaceutique.

30. Procédé selon la revendication 29, caractérisé en ce que l'on prépare le 8-fluoro-5,6-dihydro-5-méthyl-6-oxo-4H-imidazo[1,5-a][1,4]benzodiazépine-3-carboxylate d'éthyle.

31. Procédé selon la revendication 29, caractérisé en ce que l'on prépare le 7-chloro-5,6-dihydro-5-méthyl-6-oxo-4H-imidazo[1,5-a][1,4]benzodiazépine-3-carboxylate d'éthyle.

32. Médicament contenant un composé selon l'une des revendications 1 à 20 ou un sel d'un tel

composé formé par addition avec un acide acceptable pour l'usage pharmaceutique.

33. Médicament antagonisant les propriétés sédatives centrales, myorelaxantes, atactiques, hypotensives et dépressives respiratoires des 1,4-benzodiazépines à activité tranquillisante, contenant un composé selon l'une des revendications 1 à 20 ou un sel d'un tel composé formé par addition avec un acide acceptable pour l'usage pharmaceutique.

34. Médicament contenant du 8-fluoro-5,6-dihydro-5-méthyl-6-oxo-4H-imidazo[1,5-a][1,4]benzodiazépine-3-carboxylate d'éthyle ou un sel de ce composé formé par addition avec un acide acceptable pour l'usage pharmaceutique.

35. Médicament contenant du 7-chloro-5,6-dihydro-5-méthyl-6-oxo-4H-imidazo[1,5-a][1,4]benzodiazépine-3-carboxylate d'éthyle ou un sel de ce composé formé par addition avec un acide acceptable pour l'usage pharmaceutique.

36. Médicaments antagonisant les propriétés sédatives centrales, myorelaxantes, atactiques, hypotensives et dépressives respiratoires des 1,4-benzodiazépines à activité tranquillisante, contenant du 8-fluoro-5,6-dihydro-5-méthyl-6-oxo-4H-imidazo[1,5-a][1,4]benzodiazépine-3-carboxylate d'éthyle ou ses sels formés par addition avec des acides acceptables pour l'usage pharmaceutique.

37. Médicaments antagonisant les propriétés sédatives centrales, myorelaxantes, atactiques, hypotensives et dépressives respiratoires des 1,4-benzodiazépines à activité tranquillisante, contenant du 7-chloro-5,6-dihydro-5-méthyl-6-oxo-4H-imidazo[1,5-a][1,4]benzodiazépine-3-carboxylate d'éthyle ou ses sels formés par addition avec des acides acceptables pour l'usage pharmaceutique.

38. Médicaments à activité schistosomicide, contenant un composé selon l'une des revendications 1 à 20 ou un sel d'un tel composé formé par addition avec un acide acceptable pour l'usage pharmaceutique, et un composé de formule générale

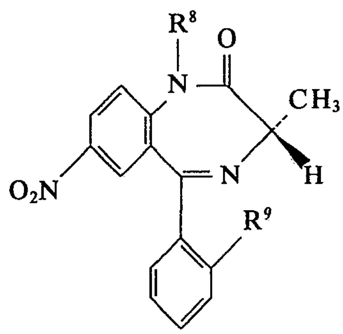

(XXII)

dans laquelle $R^8$ représente l'hydrogène ou un groupe alkyle en C 1—C 7 et $R^9$ représente un halogne,

et/ou un composé de formule générale

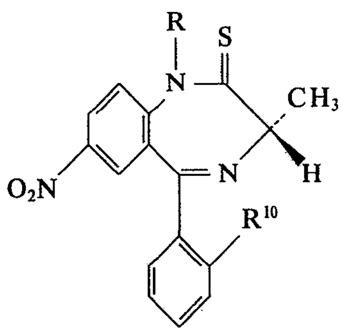

(XXIII)

dans laquelle R représente l'hydrogène ou un groupe alkyle en C 1—C 7 et $R^{10}$ représente l'hydrogène, un halogène ou un groupe trifluorométhyle.

39. Médicament à acitivté schistosomicide selon la revendiaction 38, caractérisé en ce qu'il contient du 8-fluoro-5,6-dihydro-5-méthyl-6-oxo-4H-imidazo[1,5-a][1,4]benzodiazépine-3-carboxylate d'éthyle ou un sel de ce composé formé par addition avec un acide acceptable pour l'usage pharmaceutique.

40. Médicament à activité schistosomicide selon la revendication 38, caractérisé en ce qu'il contient du 7-chloro-5,6-dihydro-5-méthyl-6-oxo-4H-imidazo[1,5-a][1,4]benzodiazépine-3-carboxylate d'éthyle ou un sel de ce composé formé par addition avec un acide acceptable pour l'usage pharmaceutique.

41. Médicament à activité schistosomicide selon la revendication 38, caractérisé en ce qu'il contient de la (+)-5-(o-chlorophényl)-1,3-dihydro-3-méthyl-7-nitro-2H-1,4-benzodiazépine-2-one.

42. Médicament à acitivté schistosomicide, contenant du 8-fluoro-5,6-dihydro-5-méthyl-6-oxo-4H-imidazo[1,5-a][1,4]benzodiazépine-3-carboxylate d'éthyle ou un sel de ce composé formé par addition avec un acide acceptable pour l'usage pharmaceutique, et de la (+)-5-(o-chlorophényl)-1,3-dihydro-3-méthyl-7-nitro-2H-1,4-benzodiazépine-2-one.

43. Médicament à activité schistosomicide, contenant du 7-chloro-5,6-dihydro-5-méthyl-6-oxo-4H-imidazo[1,5-a][1,4]benzodiazépine-3-carboxylate d'éthyle ou un sel de ce composé formé par addition avec un acide acceptable pour l'usage pharmaceutique, et de la (+)-5-(o-chlorophényl)-1,3-dihydro-3-méthyl-7-nitro-2H-1,4-benzodiazépine-2-one.

**Revendications pour l'Etat Contractant: AT**

1. Procédé de préparation de dérivés de l'imidazodiazépine de formule générale

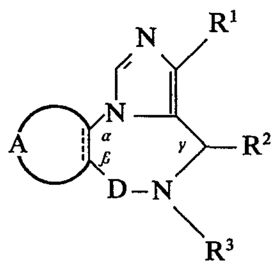

(I)

dans laquelle A forme avec les deux atomes de carbone $\alpha$ et $\beta$ l'un des groupes

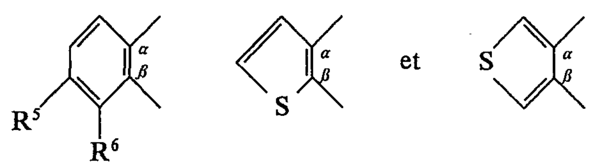

(a) (b) et (c)

et le trait interrompu représente la double liaison existant dans les cas (a) et (b), D représente $>C=O$ ou $>C=S$, $R^1$ représente un groupe cyano, alcanoyle en C 2—C 7 ou un rest de formule $-COOR^4$, $R^4$ représente un groupe méthyle, éthyle, isopropyle ou 2-hydroxyéthyle, $R^5$ représente l'hydrogène, un groupe trifluorométhyle ou un halogène et $R^6$ représente l'hydrogène, un groupe trifluorométhyle, un halogène ou un groupe alkyle en C 1—C 7, et ou bien $R^2$ représente l'hydrogène et $R^3$ l'hydrogène ou un groupe alkyle en C 1—C 7, ou bien $R^2$ et $R^3$ forment ensemble un groupe triméthylène ou propénylène, et l'atome de carbone $\gamma$ est en configuration S ou la configuration R, S,

et de leurs sels formés par addition avec des acides acceptables pour l'usage pharmaceutique, caractérisé en ce que:

a) on fait réagir un composé de formule générale

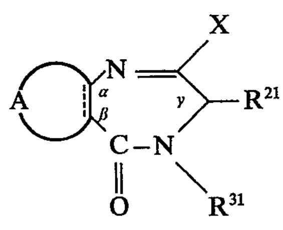

(II)

dans laquelle A et le trait interrompu ont les significations indiquées ci-dessus et X représente un groupe éliminable, et ou bien $R^{21}$ représente l'hydrogène et $R^{31}$ un groupe alkyle en C 1—C 7, ou bien $R^{21}$ et $R^{31}$ forment ensemble un groupe triméthylène ou propénylène, et l'atome de carbone $\gamma$ est en configuration S ou R, S,

en présence d'une base, avec un ester isocyanacétique de formule générale

$$CN-CH_2-COOR^{41} \qquad (III)$$

dans laquelle $R^{41}$ représente un groupe méthyle, éthyle ou isopropyle,

ou bien

b) on traite un composé de formule générale

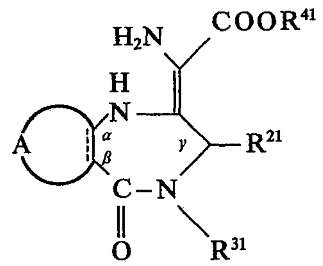

(IV)

dans laquelle A, le trait interrompu, $R^{21}$, $R^{31}$ et $R^{41}$ ont les significations indiquées ci-dessus, par un agent formylant, ou bien

c) on soumet à déshydrogénation un composé de formule générale

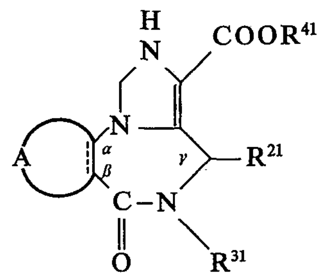

(V)

ou

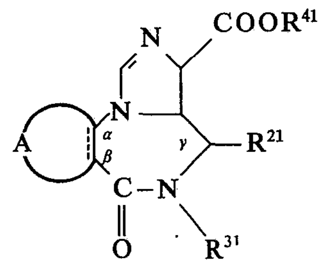

(VI)

dans lesquelles A, le trait interrompu, $R^{21}$, $R^{31}$ et $R^{41}$ ont les significations indiquées ci-dessus, ou bien

d) dans un composé de formule générale

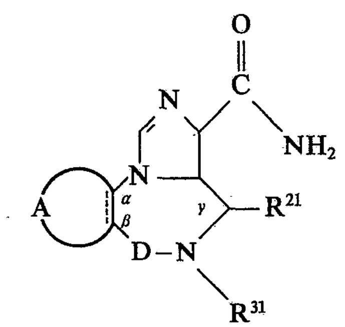

(VII)

dans laquelle A, le trait interrompu, D, $R^{21}$ et $R^{31}$ ont les significations indiquées ci-dessus, on convertit le groupe carboxamido en le groupe nitrile, ou bien

e) dans un composé de formule générale

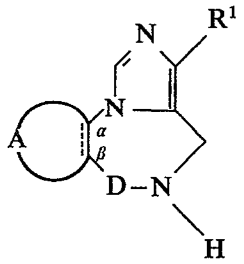

(Ia)

dans laquelle A, le trait interrompu, D et $R^1$ ont les significations indiquées ci-dessus,

on introduit le substituant voulu sur le groupe amino secondaire, ou bien

f) dans un composé de formule générale

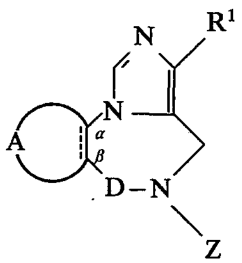

(VIII)

dans laquelle A, le trait interrompu, D et $R^1$ ont les significations indiquées ci-dessus, et Z représente un groupe protecteur,

on élimine le groupe protecteur, ou bien

g) dans un composé de formule générale

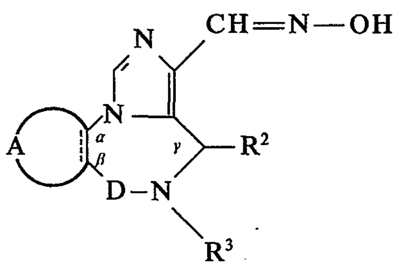

(IX)

dans laquelle A, le trait interrompu, D, $R^2$ et $R^3$ ont les significations indiquées ci-dessus,

on convertit le groupe oxime en le groupe nitrile, ou bien

h) dans un composé de formule générale

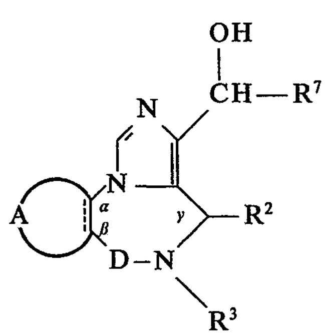

(X)

dans laquelle A, le trait interrompu, D, $R^2$ et $R^3$ ont les significations indiquées ci-dessus, et $R^7$ représente un groupe alkyle en C 1—C 6,

on convertit le groupe hydroxy en le groupe céto, ou bien

i) on soumet un composé de formule générale

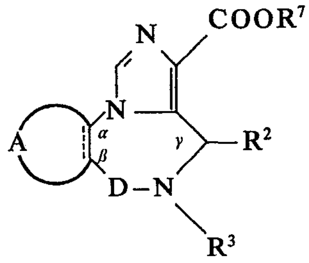

(XI)

dans laquelle A, le trait interrompu, D, $R^2$ et $R^3$ ont les significations indiquées ci-dessus, et $R^7$ représente un groupe alkyle en C 1—C 7, à transestérification,

ou bien

j) dans un composé de formule générale

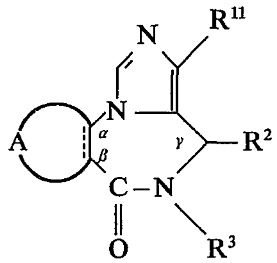

(Ib)

dans laquelle $R^{11}$ représente un groupe cyano ou un reste de formule $-COOR^4$, et A, le trait interrompu, $R^2$, $R^3$ et $R^4$ ont les significations indiquées ci-dessus,

on convertit le groupe carbonyle en le groupe thiocarbonyle, et

k) si on le désire, on convertit un composé de formule générale I en un sel formé par addition avec un acide acceptable pour l'usage pharmaceutique.

2. Procédé selon la revendication 1, caractérisé en ce que l'on prépare des composés de formule générale I définie dans la revendication 1, dans laquelle A forme avec les deux atomes de carbone $\alpha$ et $\beta$ le groupe (a) défini dans la revendication 1 et le trait interrompu représente la double liaison existant dans ce cas, D représente $>C=O$ ou $>C=S$, $R^1$ représente un reste de formule $-COOR^4$, $R^2$ représente l'hydrogène, $R^3$ l'hydrogène ou un groupe alkyle en C 1—C 7, $R^4$ représente un groupe méthyle, éthyle ou isopropyle, et $R^5$ et $R^6$ représentent tous deux l'hydrogène.

3. Procédé selon la revendication 1, caractérisé en ce que l'on prépare des composés de formule générale I définie dans la revendication 1, dans laquelle A forme avec les deux atomes de carbone $\alpha$ et $\beta$ le groupe (a) défini dans la revendication 1 et le trait interrompu représente la double liaison existant dans ce cas, D représente $>C=O$ ou $>C=S$, $R^1$ un reste de formule $-COOR^4$, $R^2$ et $R^3$ forment ensemble un groupe triméthylène et l'atome de carbone $\gamma$ est en configuration S, $R^4$ représente un groupe méthyle, éthyle ou isopropyle et $R^5$ et $R^6$ représentent tous deux des atomes d'hydrogène.

4. Procédé selon la revendication 1, caractérisé en ce que l'on prépare des composés de formule générale I définie dans la revendication 1, dans laquelle A forme avec les deux atomes de carbone $\alpha$ et $\beta$ le groupe (a) défini dans la revendication 1 et le trait interrompu représente la double liaison existant dans ce cas, D représente $>C=O$ ou $>C=S$, $R^1$ un reste de formule $-COOR^4$, $R^2$ l'hydrogène, $R^3$ l'hydrogène ou un groupe alkyle en C 1—C 7, $R^4$ un groupe méthyle, éthyle ou isopropyle, $R^5$ un halogène et $R^6$ l'hydrogène.

5. Procédé selon la revendication 1, caractérisé en ce que l'on prépare des composés de formule générale I définie dans la revendication 1, dans laquelle A forme avec les deux atomes de carbone $\alpha$ et $\beta$ le groupe (a) défini dans la revendication 1 et le trait interrompu représente la double liaison existant dans ce cas, D représente $>C=O$ ou $>C=S$, $R^1$ un reste de formule $-COOR^4$, $R^2$ et $R^3$ forment ensemble un groupe triméthylène et l'atome de carbone $\gamma$ est en configuration S, $R^4$ représente un groupe méthyle, éthyle ou isopropyle, $R^5$ le fluor et $R^6$ l'hydrogène.

6. Procédé selon la revendication 1, caractérisé en ce que l'on prépare des composés de formule générale I définie dans la revendication 1, dans laquelle $R^1$ représente un groupe cyano ou un reste de

formule $-COOR^4$, $R^4$ représentant un groupe méthyle, éthyle ou isopropyle.

7. Procédé selon la revendication 1 ou 6, caractérisé en ce que l'on prépare des composés de formule générale I définie dans la revendication 1, dans laquelle $R^3$ représente le groupe méthyle lorsque $R^2$ représente l'hydrogène.

8. Procédé selon la revendication 1 ou 6, caractérisé en ce que l'on prépare des composés de formule générale I définie dans la revendication 1, dans laquelle l'atome de carbone $\gamma$ est en configuration S lorsque $R^2$ et $R^3$ forment ensemble un groupe triméthylène.

9. Procédé selon l'une des revendications 1 et 6 à 8, caractérisé en ce que l'on prépare des composés de formule générale I définie dans la revendication 1, dans laquelle A forme avec les deux atomes de carbone $\alpha$ et $\beta$ le groupe (a) défini dans la revendication 1 et le trait interrompu représente la double liaison existant dans ce cas, $R^5$ représente l'hydrogène ou le fluor et $R^6$ l'hydrogène, le fluor, le chlore ou un groupe méthyle, l'un au moins des symboles $R^5$ et $R^6$ représentant l'hydrogène.

10. Procédé selon la revendication 4, caractérisé en ce que l'on prépare le 8-fluoro-5,6-dihydro-5-méthyl-6-oxo-4H-imidazo[1,5-a][1,4]benzodiazépine-3-carboxylate d'éthyle.

11. Procédé selon la revendication 1, caractérisé en ce que l'on prépare le 7-fluoro-5,6-dihydro-5-méthyl-6-oxo-4H-imidazo[1,5-a][1,4]benzodiazépine-3-carboxylate d'éthyle.

12. Procédé selon la revendication 1, caractérisé en ce que l'on prépare le 7-chloro-5,6-dihydro-5-méthyl-6-oxo-4H-imidazo[1,5-a][1,4]benzodiazépine-3-carboxylate d'éthyle.

13. Procédé selon la revendication 1, caractérisé en ce que l'on prépare le (S)-8-chloro-11,12,13,13a-tétrahydro-9-oxo-9H-imidazo[1,5-a]pyrrolo[2,1-c][1,4]benzodiazépine-1-carboxylate d'éthyle.

14. Procédé selon la revendication 1, caractérisé en ce que l'on prépare le (R,S)-11,13a-dihydro-9-oxo-9H-imidazo[1,5-a]parrolo[2,1-c][1,4]benzodiazépine-1-carboxylate d'éthyle.

15. Procédé selon la revendication 1, caractérisé en ce que l'on prépare le 8-fluoro-11,13a-dihydro-9-oxo-9H-imidazo[1,5-a]pyrrolo[2,1-c][1,4]benzodiazépine-1-carboxylate d'éthyle.